# EUROPEAN PATENT APPLICATION

(11) **EP 4 159 726 A1**
(43) Date of publication of application: **05.04.2023**
(21) Application number: 21813774.3
(22) Date of filing: 25.05.2021
(51) Int. Cl.: C07D 401/06, A61K 31/454, A61K 31/496, A61P 25/00, A61P 25/18, C07D 403/06, C07D 401/14, A23L 33/10

(54) **5-MEMBERED HETEROARYL DERIVATIVE CONTAINING AT LEAST ONE N, AND PHARMACEUTICAL COMPOSITION FOR PREVENTING OR TREATING MENTAL DISORDERS, CONTAINING SAME AS ACTIVE INGREDIENT**

(30) Priority: 29.05.2020 KR 20200065506
(71) Applicant: Daegu-Gyeongbuk Medical Innovation Foundation, Daegu 41061 (KR); Trineuro, Seoul 05029 (KR)
(72) Inventor: SONG, Minsoo, Daegu 41061 (KR); PARK, Ga Young, Daegu 41061 (KR); KO, Eun Bi, Daegu 41061 (KR); KANG, Jihee, Daegu 41061 (KR); IM, Chun Young, Daegu 41061 (KR); BAE, Seri, Daegu 41061 (KR); KIM, Soong-Hyun, Daegu 41061 (KR); PARK, Yoojin, Daegu 41061 (KR); LEE, Eunhye, Daegu 41061 (KR); CHOI, Yujeong, Daegu 41061 (KR); KIM, Sang Bum, Daegu 41061 (KR); KWON, Oh-Bin, Daegu 41061 (KR); SHIN, Chan Young, Seoul 05029 (KR); KWON, Kyoung Ja, Seoul 05029 (KR); PAUDEL, Suresh, Seoul 05029 (KR); JEON, Se Jin, Seoul 05029 (KR); CHO, Kyu Suk, Seoul 05029 (KR)
(74) Representative: EP&C
(86) International application number: PCT/KR2021/006496
(87) International publication number: WO 2021/241982

(57) **Abstract**

The present invention relates to a 5-membered heteroaryl derivative containing an N, and a pharmaceutical composition for use in preventing or treating mental disorders, containing same as an active ingredient, wherein the derivative is excellent in the triple reuptake inhibitory effect of serotonin, norepinephrine, and dopamine, and thus can be useful in the treatment of mental disorders.

## Description

### TECHNICAL FIELD

The present invention relates to a 5-membered heteroaryl derivative containing an N, and a pharmaceutical composition for use in preventing or treating mental disorders, containing same as an active ingredient.

### BACKGROUND ART

Mental disorders refer to pathological abnormalities of the brain characterized by identifiable symptoms resulting in abnormalities of cognition, emotion, mood, or emotion. Representative mental disorders include attention deficit hyperactivity disorder (ADHD), panic disorder, bipolar disorder, depression, schizophrenia, eating disorder, dissociative disorder, post-traumatic stress disorder, *etc*. and these disorders are characterized by various dysfunctions, severity and duration of symptoms, *etc*. Factors that cause mental disorders include: 1) brain secretion disorder caused by an excess or deficiency of neurotransmitters affecting cerebral nerve functions, 2) brain damage and degeneration of brain functions, 3) abnormal secretion of hormones, 4) stress, *etc.*

Numerous neurons constituting the brain regulate nerve functions by communicating with different neurons, and these neurons have two main transmission methods. One method is an electrical transmission, which is a method of transmission within neurons, and the other method is a chemical transmission, which is a method of extraneuronal transmission between neurons. The chemical transmission occurs at the synapse (the junction between the axon terminal of one neuron and the dendrite of the next neuron), and it is a method of secreting neurotransmitters (*i*.*e*., chemical substances) in the pre-synaptic process and transferring them to another synapse. As a postsynaptic process, the neuron which has received neurotransmitters through the synapse is activated and excited when the threshold is exceeded according to the amount of the neurotransmitters. The thus activated neuron generates an electrical signal in the dendrite and transmits the signal to the axon, and the axon secretes neurotransmitters through the synapse, and this whole process is repeated. Neurotransmitters secreted from neurons to regulate synaptic responses with neurons and eventually to control neural circuits are known to be the most important factors regulating human behavior.

When neurotransmitters are secreted from a neuron, specific receptors in a postsynaptic neuron forming a synapse with the neuron bind to the neurotransmitters to be activated, postsynaptic neurons and synaptic activation can be regulated depending on the signaling of receptors. Human behavior is presumed to be regulated by the regulation of activation of neurons and synapses by neurotransmitters, and mutations in genes of neurotransmitters (when the neurotransmitters are peptides), enzymes that produce neurotransmitters, or genes of neurotransmitter receptors have been reported to be associated with behavioral control disorders. Additionally, it is known that drugs that regulate the amount of neurotransmitters or signal transmission through receptors can regulate human behavior.

Neurotransmitters that regulate behavior as described above include monovalent amines (*e*.*g*., catecholamine and serotonin), acetylcholine, excitatory or inhibitory amino acids, and various neuropeptides and receptors thereof are largely classified into ionotropic receptors, G-protein-coupled receptors, and tyrosine kinase receptors.

Among various neurotransmitters in the brain, dopamine, which is a catecholamine-based neurotransmitter, may be an excitatory signal or inhibitory signal depending on the type of postsynaptic dopamine receptors. Dopamine is associated with behavior, attention, learning, motivation, reward system, reinforcing effect, *etc*. in our body. When dopamine activity is reduced, depressive symptoms (*e*.*g*., lethargy, decreased motivation, decreased energy, *etc*.) appear. In addition, dopamine excites neurons in the frontal lobe and affects mental activities such as short-term memory, planning, and strategy. In particular, the reward given by dopamine plays a very important role in the construction of neural pathways in the early brain because the reward makes humans repeat the behavior and learning in order to feel the pleasure of this reward.

Norepinephrine, which is a catecholamine-based neurotransmitter found in the autonomic nervous system, is known as a secondary neurotransmitter of dopamine, which is secreted by the release action of dopamine. Norepinephrine is associated with arousal, which makes the body tense, pay attention and alert to its surroundings, and involved in fear or stress response. In particular, norepinephrine is closely associated with emotions and is considered a cause of mood disorders (*e*.*g*., depression and bipolar disorder). That is, there is a hypothesis that depression is a state in which the function of norepinephrine or dopamine is reduced, and mania is a state in which the function of norepinephrine is excessively accelerated.

Serotonin, which is a neurotransmitter that usually acts in an inhibitory way, is associated with many basic human physiological phenomena (*e*.*g*., sleep, appetite, pain, regulation of body temperature, cardiovascular response, sexual desire, anxiety, depression, *etc*.). Clinically, serotonin is considered to be involved in the causes of various neuropsychiatric disorders (*e*.*g*., depression, schizophrenia, obsessive-compulsive disorder, anxiety disorder, eating disorder, sleep disorder, sexual disorder, impulse control disorder, developmental disorder, degenerative brain diseases, stress disorder, motor disorder, *etc.*), and in particular, the association with depression is very important.

Neurotransmitters are contained in synaptic vesicles of a neuron, and when stimulation is transferred to the neuron, calcium ion channels open before the synapse between neurons, leading to an influx of calcium into the neuron to move synaptic vesicles to the cell membrane of the neuron. The vesicles migrated to the cell membrane and is released into the synapse by exocytosis at the axon terminal. The released neurotransmitters interact with the receptors to excite or inhibit the neuron that has received the neurotransmitters, and the remaining neurotransmitters after acting on the receptors are reuptaken at the axon terminal of the presynaptic neuron, degraded by degradation enzymes present in the postsynaptic membrane, or diffused outward and removed by glial cells.

The reuptake process occurs by a neurotransmitter transporter. Depending on the type of neurotransmitters, specific neurotransmitter transporters exist, which are present in the synaptic membrane and are responsible for reuptake of neurotransmitters present in the synaptic cleft into the presynaptic neuron. The regulation of the concentration of neurotransmitters through this process would ultimately affect the intensity and duration of activation of various receptors present in the postsynaptic neuron.

As described above, the reuptake of neurotransmitters refers to the process in which neurotransmitters are inactivated by the reuptake into the presynaptic neuron by specific neurotransmitter transporters in the membrane of the presynaptic neuron from which the neurotransmitters have been released. The increase in reuptake of neurotransmitters causes deficiency and imbalance of neurotransmitters, resulting in diseases associated with reuptake of neurotransmitters. Treatments for these reuptake-related diseases improve symptoms by increasing the concentration of neurotransmitters in the synapse by blocking the reuptake of these substances.

Neurotransmitter reuptake inhibitors include selective serotonin reuptake inhibitors, serotonin-norepinephrine reuptake inhibitors, selective norepinephrine reuptake inhibitors, dopamine-norepinephrine reuptake inhibitors, serotonin receptor antagonists-serotonin reuptake inhibitors, *etc*., and these inhibitors are used to treat depression, mood disorder, ADHD, chronic neuropathic pain, obsessive-compulsive disorder, panic disorder, anxiety disorder, menopausal symptoms, *etc*.

Drugs for treating mental disorders are continuously under development, and among them, as related art associated with neurotransmitter reuptake inhibitors, Korean Patent Laid-open No. 10-2009-0089439 discloses chromen-2-one derivatives, a use thereof as monoamine neurotransmitter reuptake inhibitors, and a use for the treatment of mental disorders, and Korean Patent Laid-open No. 10-2009-0117736, which is directed to a composition for selective serotonin reuptake inhibition and a method for producing the same, discloses a composition containing asiaticoside and madecassoside.

However, currently-used treatments for mental disorders in the field of cranial nerves act merely on one nervous system among the various neurotransmitters suggested as related etiological mechanisms or have insufficient selectivity for neurotransmitters, and thus have many problems in therapeutic effects and compliance of patients, and cause serious adverse effects (*e*.*g*., growth retardation, sleep disorder, and dependence); therefore, there is a need to develop drugs that can flexibly regulate multiple targets with various differential selectivity for neurotransmitters.

In this regard, development on drugs that regulate multiple targets is in progress, in which Korean Patent Laid-open No. 10-2013-0026292, which is directed to novel azetidine derivatives and antidepressant composition containing the same, discloses an antidepressant composition containing an azetidine derivative capable of simultaneously inhibiting reuptake of dopamine, serotonin, and norepinephrine; Korean Patent Laid-open No. 2002-0079730 discloses a use of aryl- and heteroaryl-substituted tetrahydroisoquinolines to block reuptake of norepinephrine, dopamine, and serotonin; and Korean Patent Laid-open No. 10-2010019982, which is directed to phenyl substituted cycloalkylamines as monoamine reuptake inhibitors, discloses phenyl substituted cycloalkylamines which inhibit reuptake of endogenous monoamines, such as dopamine, serotonin and norepinephrine from the synaptic cleft and modulate one or more monoamine transporter.

The present inventors have made efforts to develop drugs that could flexibly regulate multiple targets by securing the differential selectivity of neuronal function control thereof, based on substances that simultaneously regulate dopamine, norepinephrine, and serotonin nervous systems, and confirmed that a 5-membered heteroaryl derivative containing at least one N has the triple reuptake inhibitory effect of serotonin, norepinephrine, and dopamine, and thus can be useful in the treatment of mental disorders, thereby completing the present invention.

### DISCLOSURE OF THE INVENTION

### TECHNICAL PROBLEM

One object of the present invention is to provide a 5-membered heteroaryl derivative containing an N.

Another object of the present invention is to provide a pharmaceutical composition for use in preventing or treating mental disorders, containing a 5-membered heteroaryl derivative containing an N as an active ingredient.

Still another object of the present invention is to provide a triple reuptake inhibitor of serotonin, norepinephrine, and dopamine for use in preventing or treating mental disorders, containing a 5-membered heteroaryl derivative containing an N as an active ingredient.

Still another object of the present invention is to provide a health functional food composition for use in preventing or improving mental disorders, containing a 5-membered heteroaryl derivative containing an N as an active ingredient.

### TECHNICAL SOLUTION

In order to accomplish the objects,
an aspect of the present invention provides a compound represented by Formula 1 below, a stereoisomer thereof, or a pharmaceutically acceptable salt thereof.

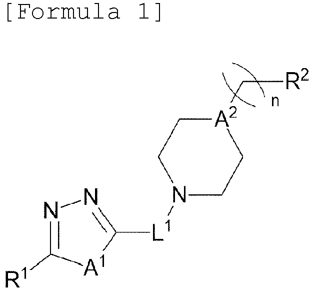

In Formula 1,
n is an integer from 0 to 5;
A¹ is O, NH, or S;
A² is N or CH;
L¹ is linear or branched C₁₋₅ alkylene or C (=O) ;
R¹ is 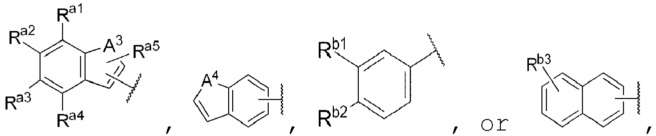
wherein A³ is NH, O, or S, A⁴ is NH, O, or S,
R^{a1}, R^{a2}, R^{a3}, R^{a4}, and R^{a5} are independently hydrogen, halogen, hydroxy, amino, CN, linear or branched C₁₋₁₀ alkyl which is unsubstituted or substituted with one or more halogens, linear or branched C₁₋₁₀ alkoxy which is unsubstituted or substituted with one or more halogens, - C(=O)OR^{c}, -C(=O)N(R^{c})₂, or -NR^{c}C(=O)R^{c}, or R^{a2} and R^{a3} together with the carbon to which they are attached form a 5- or 6-membered heterocycloalkenyl containing one or more O,
R^{b1} and R^{b2} are independently hydrogen, halogen, CN, linear or branched C₁₋₁₀ alkyl which is unsubstituted or substituted with one or more halogens, linear or branched C₁₋₁₀ alkoxy which is unsubstituted or substituted with one or more halogens, -C(=O)OR^{c}, -C(=O)N(R^{c})₂, -NR^{c}C(=O)R^{c} or phenoxy, or R^{b1} and R^{b2} together with the carbon to which they are attached form a 5- or 6-membered heterocycloalkenyl containing one or more O, R^{b3} is hydrogen, hydroxy, C₁₋₃ alkyl, or C₁₋₃ alkoxy,
R^{c} is independently hydrogen, substituted or unsubstituted linear or branched C₁₋₁₀ alkyl, in which the substituted alkyl is substituted with one or more substituents selected from the group consisting of halogen, amino, methylamino, and dimethylamino; and
R² is 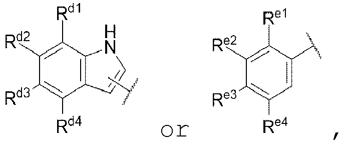 wherein R^{d1}, R^{d2}, R^{d3}, R^{d4}, R^{e1}, R^{e2}, R^{e3}, and R^{e4} are independently hydrogen, halogen, linear or branched C₁₋₁₀ alkyl which is unsubstituted or substituted with one or more halogens, or linear or branched C₁₋₁₀ alkoxy which is unsubstituted or substituted with one or more halogens.

Another aspect of the present invention provides a pharmaceutical composition for use in preventing or treating mental disorders containing the compound represented by Formula 1 above, a stereoisomer thereof, or a pharmaceutically acceptable salt thereof as an active ingredient.

Still another aspect of the present invention provides a triple reuptake inhibitor of serotonin, norepinephrine, and dopamine for use in preventing or treating mental disorders containing the compound represented by Formula 1 above, a stereoisomer thereof, or a pharmaceutically acceptable salt thereof as an active ingredient.

Still another aspect of the present invention provides a health functional food composition for use in preventing or improving mental disorders containing the compound represented by Formula 1 above, a stereoisomer thereof, or a pharmaceutically acceptable salt thereof as an active ingredient.

Still another aspect of the present invention provides a method for use in preventing or treating mental disorders, which includes administering a pharmaceutical composition or health functional food composition containing the compound represented by Formula 1 above, a stereoisomer thereof, or a pharmaceutically acceptable salt thereof as an active ingredient to a subject in need thereof.

Still another aspect of the present invention provides a use of a pharmaceutical composition or health functional food composition containing the compound represented by Formula 1 above, a stereoisomer thereof, or a pharmaceutically acceptable salt thereof as an active ingredient in the prevention or treatment of mental disorders.

### ADVANTAGEOUS EFFECTS

The compound represented by Formula 1 according to the present invention is excellent in the triple reuptake inhibitory effect of serotonin, norepinephrine, and dopamine, and thus can be useful in the treatment of mental disorders.

### MODE FOR CARRYING OUT THE INVENTION

Hereinafter, the present invention will be described in detail.

Meanwhile, the embodiments of the present invention may be modified in various forms, and the scope of the present invention is not limited to the embodiments described below. In addition, the embodiments of the present invention are provided to more completely explain the present invention to those skilled in the art. Furthermore, "including" a certain component throughout the specification means that other components may be further included instead of excluding other components unless otherwise stated.

An aspect of the present invention provides a compound represented by Formula 1 below, a stereoisomer thereof, or a pharmaceutically acceptable salt thereof.

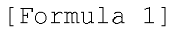

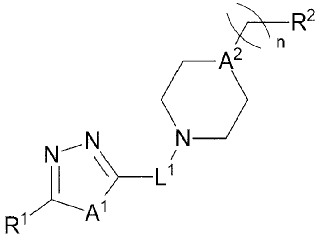

In Formula 1 above,
n is an integer from 0 to 5;
A¹ is O, NH, or S;
A² is N or CH;
L¹ is linear or branched C₁₋₅ alkylene or C(=O) ;
R¹ is 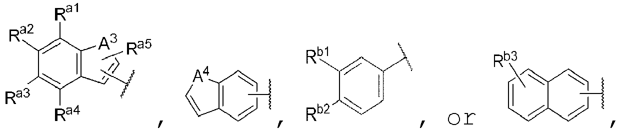
wherein A³ is NH, O, or S, A⁴ is NH, O, or S,
R^{a1}, R^{a2}, R^{a3}, R^{a4}, and R^{a5} are independently hydrogen, halogen, hydroxy, amino, CN, linear or branched C₁₋₁₀ alkyl which is unsubstituted or substituted with one or more halogens, linear or branched C₁₋₁₀ alkoxy which is unsubstituted or substituted with one or more halogens, - C(=O)OR^{c}, -C(=O)N(R^{c})₂, or -NR^{c}C(=O)R^{c}, or R^{a2} and R^{a3} together with the carbon to which they are attached form a 5- or 6-membered heterocycloalkenyl containing one or more O,
R^{b1} and R^{b2} are independently hydrogen, halogen, CN, linear or branched C₁₋₁₀ alkyl which is unsubstituted or substituted with one or more halogens, linear or branched C₁₋₁₀ alkoxy which is unsubstituted or substituted with one or more halogens, -C(=O)OR^{c}, -C(=O)N(R^{c})₂, -NR^{c}C(=O)R^{c} or phenoxy, or R^{b1} and R^{b2} together with the carbon to which they are attached form a 5- or 6-membered heterocycloalkenyl containing one or more O, and R^{b3} is hydrogen, hydroxy, C₁₋₃ alkyl, or C₁₋₃ alkoxy;
R^{c} is independently hydrogen, substituted or unsubstituted linear or branched C₁₋₁₀ alkyl, in which the substituted alkyl is substituted with one or more substituents selected from the group consisting of halogen, amino, methylamino, and dimethylamino; and
R² is 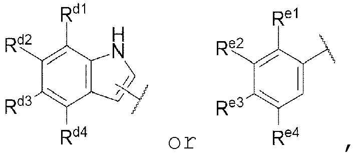 wherein R^{d1}, R^{d2}, R^{d3}, R^{d4}, R^{e1}, R^{e2}, R^{e3}, and R^{e4} are independently hydrogen, halogen, linear or branched C₁₋₁₀ alkyl which is unsubstituted or substituted with one or more halogens, or linear or branched C₁₋₁₀ alkoxy which is unsubstituted or substituted with one or more halogens.

In Formula 1 above,
R¹ is 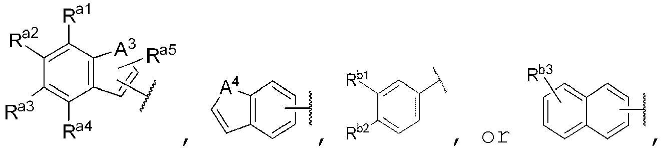
wherein A³ is NH, O, or S, A⁴ is NH, O, or S,
R^{a1}, R^{a2}, R^{a3}, R^{a4}, and R^{a5} are independently hydrogen, halogen, hydroxy, amino, CN, linear or branched C₁₋₅ alkyl which is unsubstituted or substituted with one or more halogens, linear or branched C₁₋₅ alkoxy which is unsubstituted or substituted with one or more halogens, - C(=O)OR^{c}, -C(=O)N(R^{c})₂, or -NR^{c}C(=O)R^{c}, or R^{a2} and R^{a3} together with the carbon to which they are attached form a 5- or 6-membered heterocycloalkenyl containing one or two O,
R^{b1} and R^{b2} are independently hydrogen, halogen, CN, linear or branched C₁₋₅ alkyl which is unsubstituted or substituted with one or more halogens, linear or branched C₁₋₅ alkoxy which is unsubstituted or substituted with one or more halogens, -C(=O)OR^{c}, -C(=O)N(R^{c})₂, -NR^{c}C(=O)R^{c}, or phenoxy, or R^{b1} and R^{b2} together with the carbon to which they are attached form a 5- or 6-membered heterocycloalkenyl containing one or two O,
R^{c} is independently hydrogen, substituted or unsubstituted linear or branched C₁₋₅ alkyl, in which the substituted alkyl is substituted with one or more substituents selected from the group consisting of amino, methylamino, and dimethylamino; and
R² is 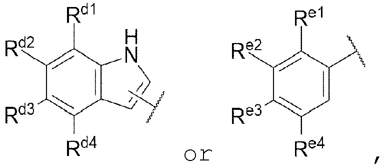 wherein R^{d1}, R^{d2}, R^{d3}, R^{d4}, R^{e1}, R^{e2}, R^{e3}, and R^{e4} are independently hydrogen, halogen, linear or branched C₁₋₅ alkyl which is unsubstituted or substituted with one or more halogens, or linear or branched C₁₋₅ alkoxy which is unsubstituted or substituted with one or more halogens.

In Formula 1 above,
n is an integer from 0 to 2;
A¹ is O, NH, or S;
A² is N or CH;
L¹ is CH₂ or C(=O);
R¹ is 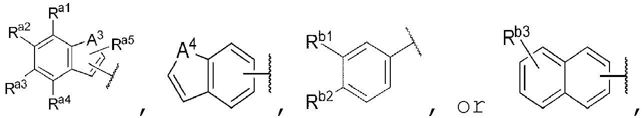
wherein A³ is NH, O, or S, A⁴ is NH, O, or S, R^{a1}, R^{a2}, R^{a3}, R^{a4}, and R^{a5} are independently hydrogen, F, Cl, OH, -NH₂, CN, methyl, -CF₃, methoxy, -C (=O) OCH₃, or -C (=O) OH, R^{b1} and R^{b2} are independently hydrogen or methoxy, or R^{b1} and R^{b2} together with the carbon to which they are attached form 1,3-dioxole; and
R² is 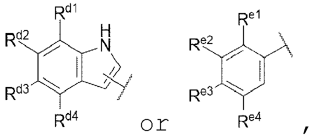 wherein R^{d1}, R^{d2}, R^{d3}, and R^{d4} may be hydrogen, and R^{e1}, R^{e2}, R^{e3} and R^{e4} may independently be hydrogen, Cl or methoxy.

In Formula 1 above,
n is an integer of 0 or 1;
A¹ is O, NH, or S;
A² is N or CH;
L¹ is CH₂ or C(=O) ;
R¹ is 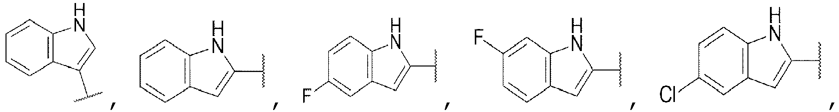 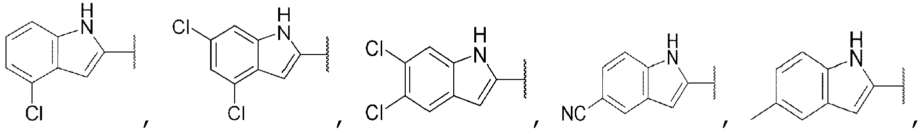 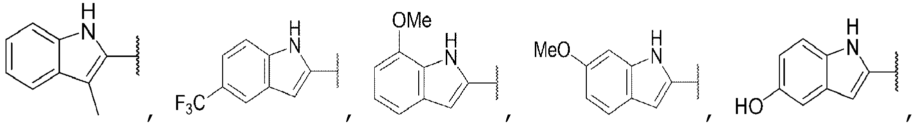 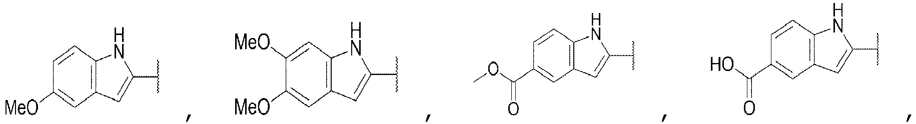 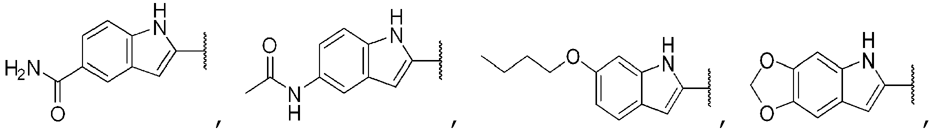 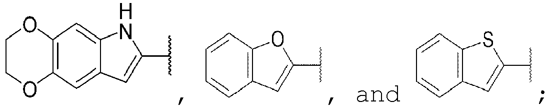 and 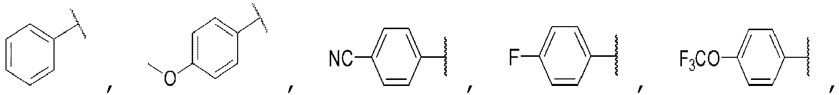 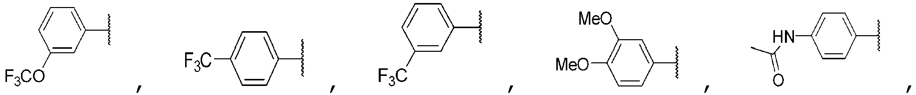 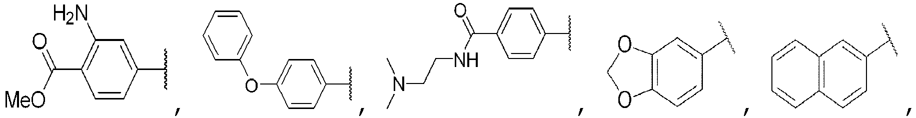 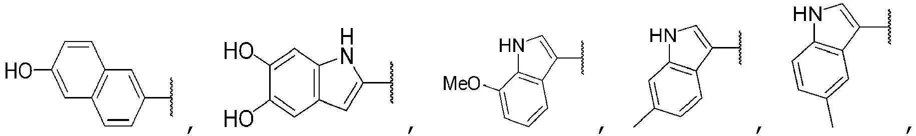 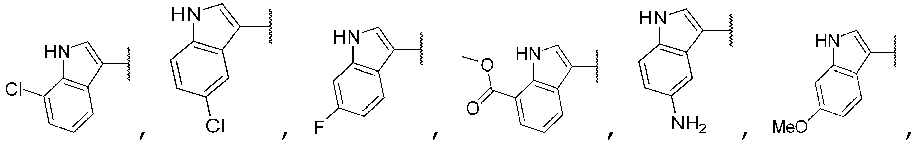 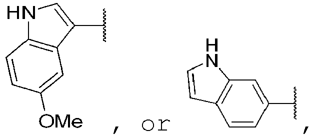
and
R² may be

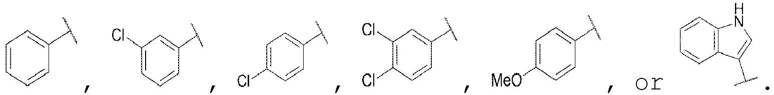

Examples of the compound represented by Formula 1 according to the present invention may include the following group of compounds:
<1> 2-(5-((4-benzylpiperidin-1-yl)methyl)-4H-1,2,4-triazol-3-yl)-1H-indole;
<2> 2-(5-((4-benzylpiperidin-1-yl)methyl)-4H-1,2,4-triazol-3-yl)-5-fluoro-1H-indole;
<3> 2-(5-((4-phenylpiperidin-1-yl)methyl)-4H-1,2,4-triazol-3-yl)-1H-indole;
<4> 2-(5-((4-benzylpiperazin-1-yl)methyl)-4H-1,2,4-triazol-3-yl)-1H-indole;
<5> 2-(5-((4-(3,4-dichlorobenzyl)piperidin-1-yl)methyl)-4H-1,2,4-triazol-3-yl)-1H-indole;
<6> 2-(5-((4-(4-methoxybenzyl)piperidin-1-yl)methyl)-4H-1,2,4-triazol-3-yl)-1H-indole;
<7> 2-(5-((4-(4-chlorobenzyl)piperidin-1-yl)methyl)-4H-1,2,4-triazol-3-yl)-1H-indole;
<8> 2-(5-((4-(3-chlorobenzyl)piperidin-1-yl)methyl)-4H-1,2,4-triazol-3-yl)-1H-indole;
<9> 4-benzyl-1-((5-phenyl-4H-1,2,4-triazol-3-yl)methyl)piperidine;
<10> 4-benzyl-1-((5-(4-methoxyphenyl)-4H-1,2,4-triazol-3-yl)methyl)piperidine;
<11> (4-benzylpiperidin-1-yl)(5-(4-methoxyphenyl)-4H-1,2,4-triazol-3-yl)methanone;
<12> 2-(5-((4-benzylpiperidin-1-yl)methyl)-4H-1,2,4-triazol-3-yl)-5,6-dimethoxy-1H-indole;
<13> 2-(5-((4-benzylpiperidin-1-yl)methyl)-4H-1,2,4-triazol-3-yl)-5-methoxy-1H-indole;
<14> 2-(5-((4-benzylpiperidin-1-yl)methyl)-4H-1,2,4-triazol-3-yl)-5-chloro-1H-indole;
<15> 2-(5-((4-benzylpiperidin-1-yl)methyl)-4H-1,2,4-triazol-3-yl)-5-methyl-1H-indole;
<16> 2-(5-((4-benzylpiperidin-1-yl)methyl)-4H-1,2,4-triazol-3-yl)-6-fluoro-1H-indole;
<17> 2-(5-((4-benzylpiperidin-1-yl)methyl)-4H-1,2,4-triazol-3-yl)-1H-indol-5-carbonitrile;
<18> 2-(5-((4-benzylpiperidin-1-yl)methyl)-4H-1,2,4-triazol-3-yl)-6-methoxy-1H-indole;
<19> 2-(5-((4-benzylpiperidin-1-yl)methyl)-4H-1,2,4-triazol-3-yl)-7-methoxy-1H-indole;
<20> methyl 2-(5-((4-benzylpiperidin-1-yl)methyl)-4H-1,2,4-triazol-3-yl)-1H-indol-5-carboxylate;
<21> 4-benzyl-1-((5-(naphthalen-2-yl)-4H-1,2,4-triazol-3-yl)methyl)piperidine;
<22> 3-(5-((4-benzylpiperidin-1-yl)methyl)-4H-1,2,4-triazol-3-yl)-1H-indole;
<23> 2-(5-((4-benzylpiperidin-1-yl)methyl)-4H-1,2,4-triazol-3-yl)-5-(trifluoromethyl)-1H-indole;
<24> 2-(5-((4-benzylpiperidin-1-yl)methyl)-4H-1,2,4-triazol-3-yl)-4-chloro-1H-indole;
<25> 1-((5-(benzofuran-2-yl)-4H-1,2,4-triazol-3-yl)methyl)-4-benzylpiperidine;
<26> 1-((5-(benzo[b]thiophen-2-yl)-4H-1,2,4-triazol-3-yl)methyl)-4-benzylpiperidine;
<27> 1-((5-(benzo[d][1,3]dioxol-5-yl)-4H-1,2,4-triazol-3-yl)methyl)-4-benzylpiperidine;
<28> 2-(5-((4-benzylpiperidin-1-yl)methyl)-4H-1,2,4-triazol-3-yl)-1H-indol-5-carboxylic acid;
<29> 2-(5-((4-(3,4-dichlorobenzyl)piperidin-1-yl)methyl)-4H-1,2,4-triazol-3-yl)-5-fluoro-1H-indole;
<30> 2-(5-((4-(3,4-dichlorobenzyl)piperidin-1-yl)methyl)-4H-1,2,4-triazol-3-yl)-5-methyl-1H-indole;
<31> 2-(5-((4-(3,4-dichlorobenzyl)piperidin-1-yl)methyl)-4H-1,2,4-triazol-3-yl)-5,6-dimethoxy-1H-indole;
<32> 2-(5-((4-(1H-indol-3-yl)piperidin-1-yl)methyl)-4H-1,2,4-triazol-3-yl)-5-fluoro-1H-indole;
<33> 2-((4-benzylpiperidin-1-yl)methyl)-5-(1H-indol-2-yl)-1,3,4-oxadiazole;
<34> 2-((4-(3,4-dichlorobenzyl)piperidin-1-yl)methyl)-5-(1H-indol-2-yl)-1,3,4-oxadiazole;
<35> 2-(5-((4-benzylpiperidin-1-yl)methyl)-4H-1,2,4-triazol-3-yl)-1H-indol-5,6-diol;
<36> 3-(5-((4-benzylpiperidin-1-yl)methyl)-4H-1,2,4-triazol-3-yl)-5-methoxy-1H-indole;
<37> 3-(5-((4-benzylpiperidin-1-yl)methyl)-4H-1,2,4-triazol-3-yl)-7-methoxy-1H-indole;
<38> 3-(5-((4-benzylpiperidin-1-yl)methyl)-4H-1,2,4-triazol-3-yl)-6-methyl-1H-indole;
<39> 3-(5-((4-benzylpiperidin-1-yl)methyl)-4H-1,2,4-triazol-3-yl)-5-methyl-1H-indole;
<40> 3-(5-((4-benzylpiperidin-1-yl)methyl)-4H-1,2,4-triazol-3-yl)-7-chloro-1H-indole;
<41> 3-(5-((4-benzylpiperidin-1-yl)methyl)-4H-1,2,4-triazol-3-yl)-5-chloro-1H-indole;
<42> methyl 3-(5-((4-benzylpiperidin-1-yl)methyl)-4H-1,2,4-triazol-3-yl)-1H-indol-7-carboxylate;
<43> 3-(5-((4-benzylpiperidin-1-yl)methyl)-4H-1,2,4-triazol-3-yl)-1H-indol-5-amine;
<44> 3-(5-((4-(3,4-dichlorobenzyl)piperidin-1-yl)methyl)-4H-1,2,4-triazol-3-yl)-5-methoxy-1H-indole;
<45> 3-(5-((4-(3,4-dichlorobenzyl)piperidin-1-yl)methyl)-4H-1,2,4-triazol-3-yl)-7-methoxy-1H-indole;
<46> 4-(5-((4-benzylpiperidin-1-yl)methyl)-4H-1,2,4-triazol-3-yl)benzonitrile;
<47> 4-benzyl-1-((5-(4-fluorophenyl)-4H-1,2,4-triazol-3-yl)methyl)piperidine;
<48> 4-benzyl-1-((5-(3,4-dimethoxyphenyl)-4H-1,2,4-triazol-3-yl)methyl)piperidine;
<49> 4-benzyl-1-((5-(4-(trifluoromethoxy)phenyl)-4H-1,2,4-triazol-3-yl)methyl)piperidine;
<50> 4-benzyl-1-((5-(4-(trifluoromethyl)phenyl)-4H-1,2,4-triazol-3-yl)methyl)piperidine;
<51> N-(4-(5-((4-benzylpiperidin-1-yl)methyl)-4H-1,2,4-triazol-3-yl)phenyl) acetamide;
<52> 6-(5-((4-benzylpiperidin-1-yl)methyl)-4H-1,2,4-triazol-3-yl)naphthalen-2-ol;
<53> 4-benzyl-1-((5-(4-phenoxyphenyl)-4H-1,2,4-triazol-3-yl)methyl)piperidine;
<54> 4-benzyl-1-((5-(3-(trifluoromethoxy)phenyl)-4H-1,2,4-triazol-3-yl)methyl)piperidine;
<55> 4-benzyl-1-((5-(3-(trifluoromethyl)phenyl)-4H-1,2,4-triazol-3-yl)methyl)piperidine;
<56> 3-(5-((4-benzylpiperidin-1-yl)methyl)-4H-1,2,4-triazol-3-yl)-6-methoxy-1H-indole;
<57> 2-(5-((4-benzylpiperidin-1-yl)methyl)-4H-1,2,4-triazol-3-yl)-1H-indol-5-carboxamide;
<58> 4-(5-((4-benzylpiperidin-1-yl)methyl)-4H-1,2,4-triazol-3-yl)-N-(2-(dimethylamino)ethyl)benzoamide;
<59> 3-(5-((4-(3,4-dichlorobenzyl)piperidin-1-yl)methyl)-4H-1,2,4-triazol-3-yl)-6-methoxy-1H-indole;
<60> methyl 3-(5-((4-(3,4-dichlorobenzyl)piperidin-1-yl)methyl)-4H-1,2,4-triazol-3-yl)-1H-indol-7-carboxylate;
<61> 4-(3,4-dichlorobenzyl)-1-((5-(4-(trifluoromethoxy)phenyl)-4H-1,2,4-triazol-3-yl)methyl)piperidine;
<62> 4-(3,4-dichlorobenzyl)-1-((5-(4-(trifluoromethyl)phenyl)-4H-1,2,4-triazol-3-yl)methyl)piperidine;
<63> methyl 2-amino-4-(5-((4-benzylpiperidin-1-yl)methyl)-4H-1,2,4-triazol-3-yl)benzoate;
<64> 2-(5-((4-benzylpiperazin-1-yl)methyl)-4H-1,2,4-triazol-3-yl)-5-fluoro-1H-indole;
<65> 2-(5-((4-benzylpiperazin-1-yl)methyl)-4H-1,2,4-triazol-3-yl)-5-chloro-1H-indole;
<66> N-(2-(5-((4-benzylpiperidin-1-yl)methyl)-4H-1,2,4-triazol-3-yl)-1H-indol-5-yl) acetamide;
<67> 2-(5-((4-benzylpiperidin-1-yl)methyl)-4H-1,2,4-triazol-3-yl)-1H-indol-5-ol;
<68> (4-benzylpiperidin-1-yl)(5-(5-fluoro-1H-indol-2-yl)-4H-1,2,4-triazol-3-yl)methanone;
<69> 2-(5-((4-benzylpiperidin-1-yl)methyl)-4H-1,2,4-triazol-3-yl)-6-butoxy-1H-indole;
<70> 2-(5-((4-benzylpiperidin-1-yl)methyl)-4H-1,2,4-triazol-3yl)-3-methyl-1H-indole;
<71> 2-(5-((4-benzylpiperidin-1-yl)methyl)-4H-1,2,4-triazol-3-yl)-4,6-dichloro-1H-indole;
<72> 2-(5-((4-benzylpiperidin-1-yl)methyl)-4H-1,2,4-triazol-3-yl)-5,6-dichloro-1H-indole;
<73> 6-(5-((4-benzylpiperidin-1-yl)methyl)-4H-1,2,4-triazol-3-yl)-5H-[1,3]dioxolo[4,5-f]indole;
<74> 7-(5-((4-benzylpiperidin-1-yl)methyl)-4H-1,2,4-triazol-3-yl)-2,3-dihydro-6H-[1,4]dioxino[2,3-f]indole;
<75> 3-(5-((4-benzylpiperidin-1-yl)methyl)-4H-1,2,4-triazol-3-yl)-6-fluoro-1H-indole; and
<76> 6-(5-((4-benzylpiperidin-1-yl)methyl)-4H-1,2,4-triazol-3-yl)-1H-indole.

The compound represented by Formula 1 of the present invention can be used in the form of a pharmaceutically acceptable salt, and an acid addition salt formed by a pharmaceutically acceptable free acid is useful as the salt. The acid addition salt is obtained from inorganic acids (e.g., hydrochloric acid, nitric acid, phosphoric acid, sulfuric acid, hydrobromic acid, hydroiodic acid, nitrous acid, phosphorous acid, etc.); non-toxic organic acids (e.g., aliphatic mono- and di-carboxylates, phenyl-substituted alkanoates, hydroxy alkanoates and alkanedioates, aromatic acids, aliphatic and aromatic sulfonic acids, etc.; organic acids (e.g., trifluoroacetic acid, acetate, benzoic acid, citric acid, lactic acid, maleic acid, gluconic acid, methanesulfonic acid, 4-toluenesulfonic acid, tartaric acid, fumaric acid, etc.). These types of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, maleate, tartrate, methanesulfonate, propanesulfonate, naphthalen-1-sulfonate, naphthalen-2-sulfonate, mandelate, etc.

The acid addition salt according to the present invention may be prepared by a conventional method, for example, may be prepared by dissolving the derivative of Formula 1 in an organic solvent (e.g., methanol, ethanol, acetone, methylene chloride, acetonitrile, etc.), and adding an organic or inorganic acid to filter and dry the resultant precipitate; or may be prepared by distilling the solvent and excess acid under reduced pressure, drying, and crystallizing in an organic solvent.

In addition, a pharmaceutically acceptable metal salt may be prepared using a base. An alkali metal or alkaline earth metal salt is obtained, for example, by dissolving a compound in an excess alkali metal hydroxide or alkaline earth metal hydroxide solution, filtering the undissolved compound salt, and evaporating and drying the filtrate. In this case, it is pharmaceutically suitable to prepare a sodium, potassium, or calcium salt as the metal salt. In addition, the corresponding salt is obtained by reacting an alkali metal or alkaline earth metal salt with a suitable negative salt (*e*.*g*., silver nitrate).

Furthermore, the present invention includes not only the compound represented by Formula 1 and a pharmaceutically acceptable salt thereof, but also solvates, optical isomers, hydrates, etc., which can be prepared therefrom.

The term "hydrate" refers to a compound of the present invention, which contains a stoichiometric or non-stoichiometric amount of water bound by a non-covalent intermolecular force, or a salt thereof. The hydrate of the compound represented by Formula 1 of the present invention may contain a stoichiometric or non-stoichiometric amount of water bound by a non-covalent intermolecular force. The hydrate may contain 1 equivalent or more, preferably 1-5 equivalents of water. Such a hydrate may be prepared by crystallizing the compound represented by Formula 1 of the present invention, a stereoisomer thereof, or a pharmaceutically acceptable salt thereof from water or a water-containing solvent.

The term "solvate" refers to the compound of the present invention or a salt thereof that contains a stoichiometric or non-stoichiometric amount of a solvent bound by a non-covalent intermolecular force. Preferred solvents as such include solvents that are volatile, non-toxic, and/or suitable for administration to humans.

The term "isomer" refers to compounds of the present invention or salts thereof that have the same chemical formula or molecular formula but differ structurally or sterically. These isomers include structural isomers (*e*.*g*., tautomers, *etc*.), R or S isomers having an asymmetric carbon center, stereoisomers (*e*.*g*., geometric isomers (*trans, cis*)), and optical isomers (enantiomers). All of these isomers and mixtures thereof are also included within the scope of the present invention.

The compound represented by Formula 1 according to the present invention may be prepared, as shown in Reaction 1 below,
may be prepared according to a preparation method which includes preparing a compound represented by Formula 1 by reacting a compound represented by Formula 2 with a compound represented by Formula 3.

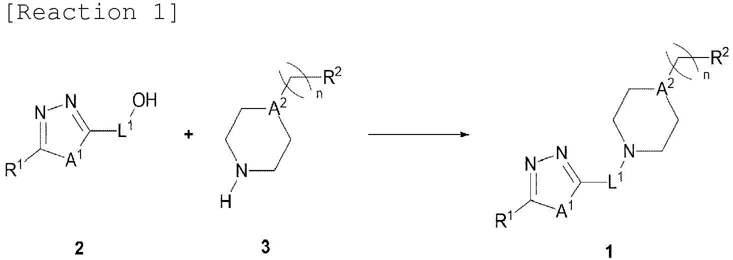

In Reaction 1 above,
R¹, R², A¹, A², L¹ and n are as defined in Formula 1 above.

Specifically, the preparation method of Reaction 1 is a method in which the compound represented by Formula 1 is prepared through a dehydration reaction between the hydroxy of the compound represented by Formula 2 and the amine of the compound represented by Formula 3 under Mitsunobu conditions. The preparation method may use reaction conditions widely known to those skilled in the art, and the preparation may be performed according to the embodiments of the present invention, but this is merely an example, and is not limited thereto.

Another aspect of the present invention provides a pharmaceutical composition for use in preventing or treating mental disorders containing, as an active ingredient, the compound represented by Formula 1, an optical isomer thereof or a pharmaceutically acceptable salt thereof.

The compound can inhibit the reuptake of serotonin, norepinephrine, and dopamine.

The mental disorders may be at least one selected from the group consisting of bulimia nervosa, mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substance-induced mood disorder, pseudo dementia, Ganger syndrome, obsessive-compulsive disorder, panic disorder, panic disorder without agoraphobia, panic disorder with agoraphobia, agoraphobia without history of panic disorder, panic attacks, memory deficit, memory loss, attention deficit hyperactivity disorder, obesity, anxiety, generalized anxiety disorder, eating disorder, Parkinson's disease, Parkinson's symptoms, dementia, aging dementia, senile dementia, Alzheimer's disease, Down syndrome, acquired immunodeficiency syndrome dementia complex, memory dysfunction in aging, specific phobia, social phobia, social anxiety disorder, post-traumatic stress disorder, acute stress disorder, chronic stress disorder, drug addiction, drug abuse, drug abuse tendency, cocaine abuse, nicotine abuse, tobacco abuse, alcohol addiction, alcoholism, pathological kleptomaniac, withdrawal syndrome due to withdrawal of intoxicating substances, pain, chronic pain, inflammatory pain, neuropathic pain, diabetic neuropathic pain, migraine, tension-type headache, chronic tension-type headache, depression-related pain, fibromyalgia, arthritis, osteoarthritis, rheumatoid arthritis, back pain, cancer pain, irritable bowel pain, irritable bowel syndrome, postoperative pain, postmastectomy pain syndrome (PMPS), poststroke pain, drug-induced neuropathy, diabetic neuropathy, pain sustained by the sympathetic nervous system, trigeminal neuralgia, toothache, facial muscle pain, phantom limb pain, anorexia, premenstrual syndrome, premenstrual dysphoric disorder, late luteal phase syndrome, posttraumatic syndrome, chronic fatigue syndrome, persistent vegetative state, urinary incontinence, stress incontinence, urge incontinence, nocturnal incontinence, sexual dysfunction, premature ejaculation, erectile difficulty, erectile dysfunction, female premature orgasm, restless legs syndrome, periodic limb movement disorder, eating disorder, anorexia nervosa, sleep disorder, pervasive developmental disorder, autism, Asperger's disorder, Rett disorder, childhood disintegrative disorder, learning disorder, motor ability disorder, mutism, trichotillomania, narcolepsy, post-stroke depression, stroke-induced brain injury, stroke-induced nerve injury, Tourette syndrome, tinnitus, tic disorder, body dysmorphic disorder, oppositional defiant disorder, and post-stroke disorder.

The compound represented by Formula 1 according to the present invention is excellent in the reuptake inhibitory effect of serotonin, norepinephrine, and dopamine, and thus can be useful in the treatment of mental disorders (see Experimental Example 1 and Table 13).

In the pharmaceutical composition according to the present invention, the compound represented by Formula 1 above or a pharmaceutically acceptable salt thereof may be administered in various oral and parenteral dosage forms during clinical administration, and more preferably, parenteral dosage forms. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, etc., and such solid formulations are prepared by mixing at least one excipient, for example, starch, calcium carbonate, sucrose or lactose, gelatin, etc. with one or more compounds. In addition to simple excipients, lubricants such as magnesium stearate and talc are also used. Liquid formulations for oral administration include suspensions, solutions for internal use, emulsions, syrups, etc., and in addition to water and liquid paraffin, which are commonly used simple diluents, various excipients such as wetting agents, sweeteners, fragrances, and preservatives may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (*e*.*g*., olive oil), injectable esters (*e*.*g*., ethyl oleate), *etc*. may be used.

The compound represented by Formula 1 or a pharmaceutically acceptable salt thereof may be administered in various oral and parenteral dosage forms during clinical administration. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, etc., and such solid formulations are prepared by mixing at least one excipient, for example, starch, calcium carbonate, sucrose or lactose, gelatin, etc. with one or more compounds. In addition to simple excipients, lubricants such as magnesium stearate and talc are also used. Liquid formulations for oral administration include suspensions, solutions for internal use, emulsions, syrups, etc., and in addition to water and liquid paraffin, which are commonly used simple diluents, various excipients such as wetting agents, sweeteners, fragrances, and preservatives may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (*e*.*g*., olive oil), injectable esters (*e*.*g*., ethyl oleate), *etc*. may be used.

The compound represented by Formula 1 or a pharmaceutically acceptable salt thereof may be administered in various oral and parenteral dosage forms during clinical administration. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, *etc.*, and such solid formulations are prepared by mixing at least one excipient, for example, starch, calcium carbonate, sucrose or lactose, gelatin, *etc*. with one or more compounds. In addition to simple excipients, lubricants such as magnesium stearate and talc are also used. Liquid formulations for oral administration include suspensions, solutions for internal use, emulsions, syrups, *etc.*, and in addition to water and liquid paraffin, which are commonly used simple diluents, various excipients such as wetting agents, sweeteners, fragrances, and preservatives may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (*e*.*g*., olive oil), injectable esters (*e*.*g*., ethyl oleate), etc. may be used.

The pharmaceutical composition containing, as an active ingredient, the compound represented by Formula 1 or a pharmaceutically acceptable salt thereof may be administered parenterally, and the parenteral administration is performed by subcutaneous injection, intravenous injection, intramuscular injection, or intrathoracic injection.

In this case, to be formulated into a dosage form for parenteral administration, the compound represented by Formula 1 above or a pharmaceutically acceptable salt thereof may be mixed in water along with a stabilizer or buffer to prepare a solution or suspension, which may be prepared in a unit dosage form in an ampoule or vial. The composition may be sterilized and/or contain preservatives, stabilizers, wetting agents or emulsification accelerators, salts and/or buffers for the control of osmotic pressure, and other therapeutically useful substances, and may be formulated according to a conventional method such as mixing, granulation, or coating method.

Dosage forms for oral administration include, for example, tablets, pills, hard/soft capsules, liquids, suspensions, emulsifiers, syrups, granules, elixirs, troches, *etc*., and these dosage forms contain diluents (*e*.*g*., lactose, dextrose, sucrose, mannitol, sorbitol, cellulose, and/or glycine), lubricants (*e*.*g*., silica, talc, stearic acid and a magnesium or calcium salt thereof, and/or polyethylene glycol) in addition to active ingredients. Tablets may contain binders (*e*.*g*., magnesium aluminum silicate, starch paste, gelatin, methylcellulose, sodium carboxymethylcellulose and/or polyvinylpyrrolidine); optionally contain disintegrants (*e*.*g*., starch, agar, alginic acid or a sodium salt thereof) or effervescent mixtures, and/or absorbents, colorants, flavors, and sweeteners.

The pharmaceutical composition for use in preventing or treating mental disorders containing, as an active ingredient, the compound represented by Formula 1 above, an optical isomer thereof or pharmaceutically acceptable salt thereof may be administered as an individual therapeutic agent or used in combination with other therapeutic agents in use.

Still another aspect of the present invention provides a triple reuptake inhibitor of serotonin, norepinephrine, and dopamine for use in preventing or treating mental disorders containing, as an active ingredient, the compound represented by Formula 1 above, a stereoisomer thereof, or a pharmaceutically acceptable salt thereof.

Yet another aspect of the present invention provides a health functional food composition for use in preventing or improving mental disorders containing, as an active ingredient, the compound represented by Formula 1 above, a stereoisomer thereof, or a pharmaceutically acceptable salt thereof.

Further another aspect of the present invention provides a method for use in preventing or treating mental disorders, the method including administering a pharmaceutical composition or health functional food composition containing, as an active ingredient, the compound represented by Formula 1 above, a stereoisomer thereof, or a pharmaceutically acceptable salt thereof to a subject in need thereof.

Yet still another aspect of the present invention provides a use of a pharmaceutical composition or health functional food composition containing, thereof as an active ingredient, the compound represented by Formula 1 above, a stereoisomer thereof, or a pharmaceutically acceptable salt in the prevention or treatment of mental disorders.

### MODES FOR CARRYING OUT THE INVENTION

Hereinafter, the present invention will be described in detail by Examples and Experimental Examples.

However, the following Examples and Experimental Examples are merely illustrative of the present invention, and the contents of the present invention are not limited to the following Examples and Experimental Examples.

### <Preparation Example 1> Preparation of Boronic Acid

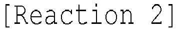

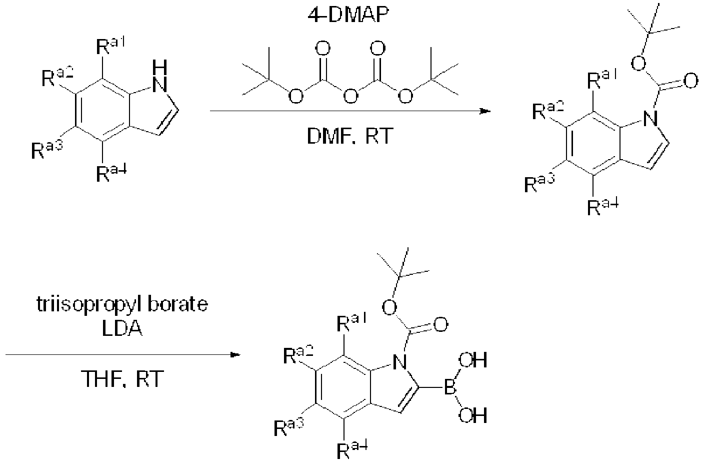

In Reaction 2 above,
R^{a1}, R^{a2}, R^{a3}, R^{a4}, and R^{a5} are as defined in Formula 1 above.

Step 1: Indole (1 eq) and N,N-dimethylpyridin-4-amine (DMAP) (2-4 mol%) were dissolved in DMF, and then di-tert-butyl dicarbonate (Boc anhydride)(1.5 eq) was added dropwise at 0°C. The reactants were stirred at room temperature for 12 hours. After completion of the reaction, water was added dropwise thereto and the resultant was extracted with ethyl acetate. The organic layer was washed sequentially with H₂O and brine and dried over anhydrous Na₂SO₄, and the solvent was removed under reduced pressure to obtain boc-indole.

Step 2: After dissolving the boc-indole (1 eq) synthesized in Step 1 in anhydrous tetrahydrofuran (THF), triisopropyl borate (1.5 eq) was added dropwise thereto under nitrogen atmosphere. After cooling the reactants to 0°C, a 2 M solution of lithium diisopropylamide (LDA 2.0 M solution in n-heptane/ethylbenzene/THF) (1.25 eq) was slowly added dropwise thereto over 15 minutes. The reactants were stirred at 0°C until the reaction was completed, and after completion of the reaction, 1 N HCl was added dropwise thereto and the resultant was extracted with ethyl acetate. The organic layer was washed sequentially with H₂O and brine and dried over anhydrous Na₂SO₄, and then the solvent was removed under reduced pressure. Then, boronic acid was obtained through solidification using ethyl acetate and hexane.

### <Example 1> Preparation of 2-(5-((4-benzylpiperidin-1-yl)methyl)-4H-1,2,4-triazol-3-yl)-1H-indole

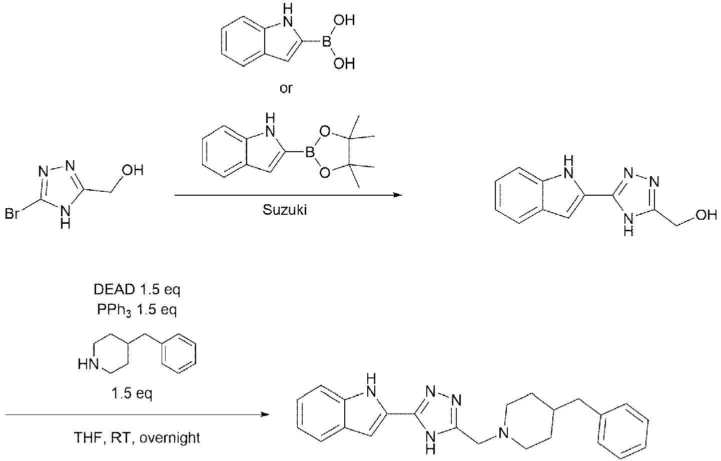

Step 1: 3-Bromo-1H-1,2,4-triazol-5-yl methanol (1 eq) and indol-2 boronic acid pinacol ester (or indol-2 boronic acid) (1.1 eq) were added into a microwave vial and dissolved in 1,4-dioxane:water (4:1), and then the catalyst Pd (dppf) Cl₂-CH₂Cl₂ (10 mol%) and K₂CO₃ (3 eq) were added dropwise thereto at room temperature and stirred for 5 minutes. The reaction mixture was reacted in the Biotage microwave at 100°C for 1.5 hours. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain (5-(1H-indol-2-yl)-4H-1,2,4-triazol-3-yl) methanol.

Step 2: The (5-(1H-indol-2-yl)-4H-1,2,4-triazol-3-yl) (1 eq) synthesized in Step 1, 4-benzylpiperidine (1.5 eq), and triphenylphosphine (1.5 eq) were dissolved in tetrahydrofuran (THF) at 0°C, and then a diethyl azodicarboxylate solution (DEAD) (1.5 eq) was added dropwise thereto. The reactants were stirred at room temperature for 12 hours under nitrogen atmosphere. After completion of the reaction, the solvent was removed under reduced pressure. After subjecting the resultant to extraction with dichloromethane, the organic layer was washed sequentially with H₂O and brine and dried over anhydrous Na₂SO₄, and the solvent was removed under reduced pressure. Then, the reaction mixture was separated and purified by MPLC to obtain the compound of Example 1.

The compound of Example 2 was obtained using R listed in Table 1 below instead of indol-2 boronic acid pinacol ester (or indol-2 boronic acid) in Step 1, while using the same method as in Example 1.

**[Table 1]**

| Example | R | Structure |
|---|---|---|
| 2 | 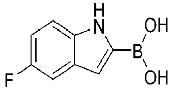 | 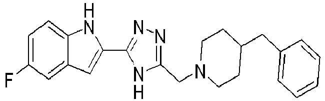 |

The compounds of Examples 3 to 8 were obtained using R listed in Table 2 below instead of 4-benzylpiperidine in Step 2, while using the same method as in Example 1.

**[Table 2]**

| Example | R | Structure |
|---|---|---|
| 3 | 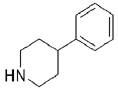 | 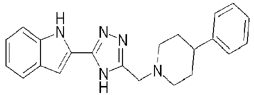 |
| 4 | 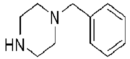 | 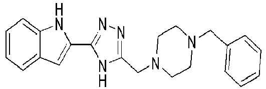 |
| 5 | 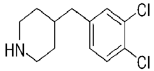 | 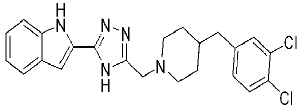 |
| 6 | 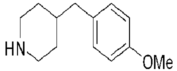 | 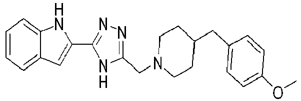 |
| 7 | 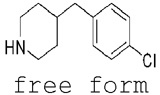 | 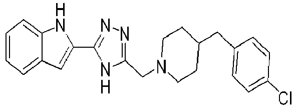 |
| 8 | 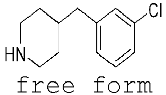 | 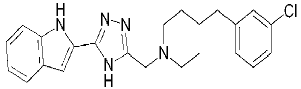 |

### <Example 9> Preparation of 4-benzyl-1-((5-phenyl-4H-1,2,4-triazol-3-yl)methyl)piperidine

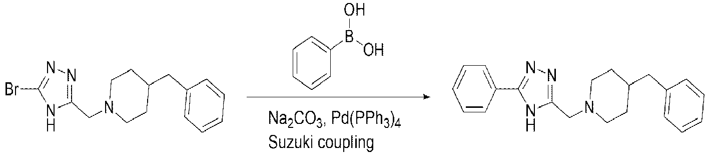

The synthesized 4-benzyl-1-((5-bromo-4H-1,2,4-triazol-3-yl)methyl) piperidine (1 eq), phenylboronic acid (1.3 eq), Na₂CO₃ (4 eq), and the catalyst Pd(Ph₃P)₄ (0.5 eq) were dissolved in DME in a microwave vial, and the reaction mixture was reacted in the Biotage microwave at 130°C for 3 hours. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the compound of Example 9.

### <Example 10> Preparation of 4-benzyl-1-((5-(4-methoxyphenyl)-4H-1,2,4-triazol-3-yl)methyl)piperidine

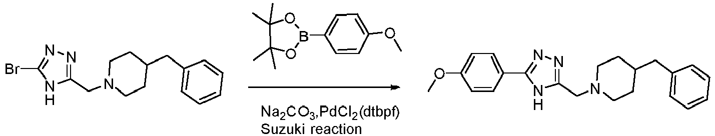

The synthesized 4-benzyl-1-((5-bromo-4H-1,2,4-triazol-3-yl)methyl) piperidine (1 eq), 2-(4-methoxyphenyl)-4,4,5,5-tetramethyl-1,3,2-dioxaboroleine (1.3 eq), Na₂CO₃ (5 eq), and the catalyst PdCl₂ (dtbpf) (0.1 eq) were dissolved in 4-dioxane:water (3:1) in a microwave vial, and the reaction mixture was reacted in the Biotage microwave at 130°C for 2 hours. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the compound of Example 10.

### <Example 11> Preparation of (4-benzylpiperidin-1-yl)(5-(4-methoxyphenyl)-4H-1,2,4-triazol-3-yl)methanone

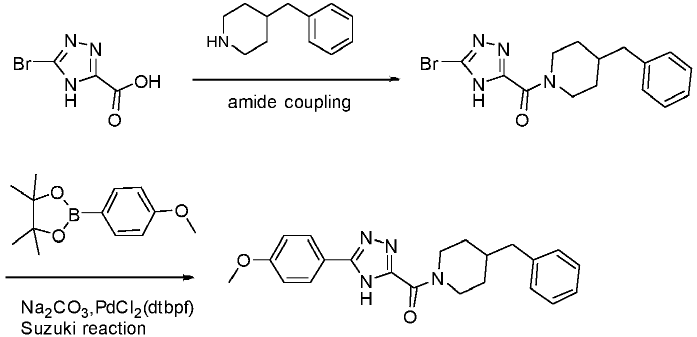

Step 1: After dissolving 5-bromo-4H-1,2,4-triazol-3-carboxylic acid (96 mg, 0.5 mmol, 1 eq) in DMF, DIPEA (175 µL, 1.000 mmol, 2 eq), HATU (285 mg, 0.750 mmol, 1.5 eq), and 4-benzylpiperidine (105 µL, 0.600 mmol, 1.2 eq) were added dropwise thereto. The reactants were stirred at room temperature for 12 hours. After completion of the reaction, the resultant was subjected to extraction with ethyl acetate, and the organic layer was sequentially washed with H₂O and brine and dried over anhydrous Na₂SO₄, and the solvent was removed under reduced pressure. Then, the reaction mixture was separated and purified by MPLC to obtain the (4-benzylpiperidin-1-yl) (5-bromo-4H-1,2,4-triazol-3-yl)methanone compound.

Step 2: After dissolving the (4-benzylpiperidin-1-yl)(5-bromo-4H-1,2,4-triazol-3-yl)methanone (1 eq) synthesized in Step 1, 2-(4-methoxyphenyl)-4,4,5,5-tetramethyl-1,3,2-dioxaboroleine (1.3 eq), Na₂CO₃ (5 eq), and the catalyst PdCl₂(dtbpf) (0.1 eq) in 4-dioxane in a microwave vial, the reaction mixture was reacted in the Biotage microwave at 130°C for 2 hours. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the compound of Example 11.

### <Example 12> Preparation of 2-(5-((4-benzylpiperidin-1-yl)methyl)-4H-1,2,4-triazol-3-yl)-5,6-dimethoxy-1H-indole

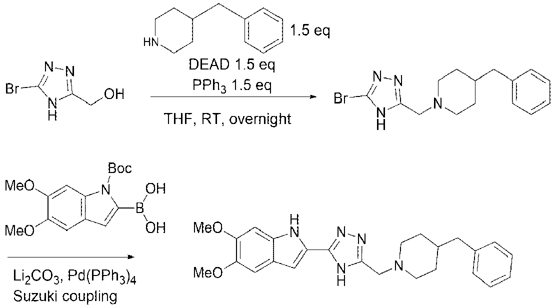

Step 1: After dissolving (5-bromo-4H-1,2,4-triazol-3-yl)methanol (500 mg, 2.81 mmol, 1 eq), 4-benzylpiperidine (0.741 mL, 4.21 mmol, 1.5 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. After subjecting the resultant to extraction with dichloromethane, the organic layer was washed sequentially with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 2: The 4-benzyl-1-((5-bromo-4H-1,2,4-triazol-3-yl)methyl)piperidine (1 eq) synthesized in Step 1, (1-(tert-butoxycarbonyl)-5,6-dimethoxy-1H-indol-2-yl)boronic acid (1.3 eq), Li₂CO₃ (2 eq), and the catalyst Pd(Ph₃P)₄ (0.1 eq) were dissolved in 4-dioxane:water (3:1) in a microwave vial, and the reaction mixture was reacted in the Biotage microwave at 130°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the compound of Example 12.

The compounds of Examples 13 to 27 were obtained using R listed in Table 3 below instead of (1-(tert-butoxycarbonyl)-5,6-dimethoxy-1H-indol-2-yl)boronic acid in Step 2, while using the same method as in Example 12.

**[Table 3]**

| Example | R | Structure |
|---|---|---|
| 13 | 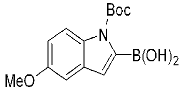 | 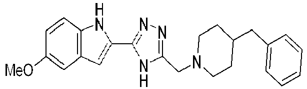 |
| 14 | 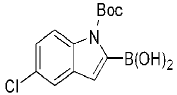 | 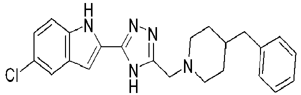 |
| 15 | 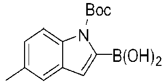 | 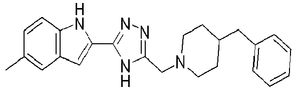 |
| 16 | 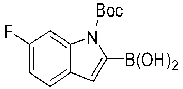 | 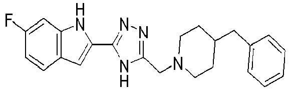 |
| 17 | 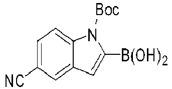 | 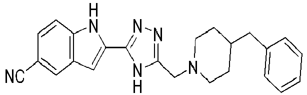 |
| 18 | 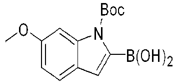 | 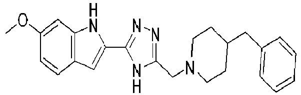 |
| 19 | 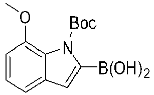 | 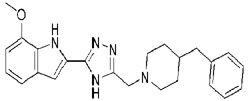 |
| 20 | 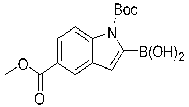 | 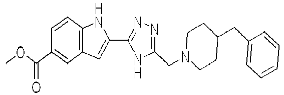 |
| 21 | 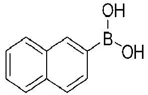 | 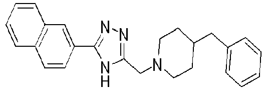 |
| 22 | 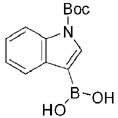 | 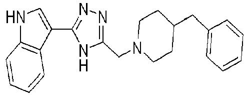 |
| 23 | 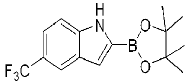 | 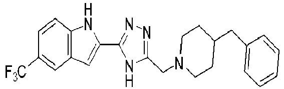 |
| 24 | 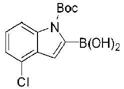 | 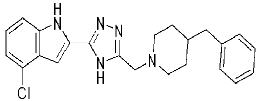 |
| 25 | 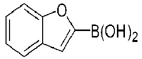 | 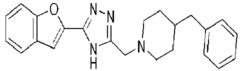 |
| 26 | 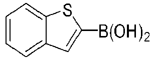 | 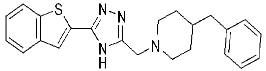 |
| 27 | 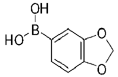 | 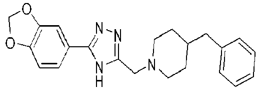 |

### <Example 28> Preparation of 2-(5-((4-benzylpiperidin-1-yl)methyl)-4H-1,2,4-triazol-3-yl)-1H-indol-5-carboxylic acid

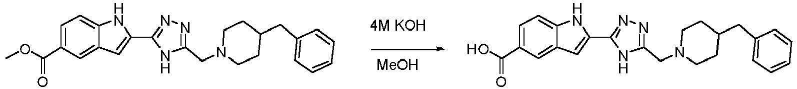

After dissolving the methyl 2-(5-((4-benzylpiperidine - 1-yl)methyl)-4H-1,2,4-triazol-3-yl)-1H-indol-5-carboxylate (1 eq) of Example 20 in methanol, an aqueous 4 M potassium hydroxide solution (150 eq, excess) was added dropwise thereto and allowed to react at room temperature for 12 hours. After completion of the reaction, the resultant was neutralized with 1 M aqueous hydrochloric acid, extracted with dichloromethane, and dried over anhydrous Na₂SO₄, and the solvent was removed under reduced pressure. Then, the reaction mixture was separated and purified by MPLC to obtain the compound of Example 28 in the form of a white solid.

### <Example 29> Preparation of 2-(5-((4-(3,4-dichlorobenzyl)piperidin-1-yl)methyl)-4H-1,2,4-triazol-3-yl)-5-fluoro-1H-indole

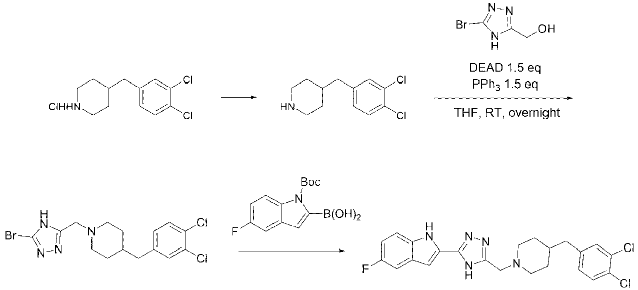

Step 1: After dissolving the 4-(3,4-dichlorobenzyl)piperidine HCl salt in dichloromethane, the resultant was washed with an aqueous 6 M NaOH solution to adjust its pH to 12 or higher. After drying the solvent of the organic layer with anhydrous Na₂SO₄, the solvent was removed under reduced pressure to obtain 4-(3,4-dichlorobenzyl)piperidine.

Step 2: After dissolving (5-bromo-4H-1,2,4-triazol-3-yl)methanol (500 mg, 2.81 mmol, 1 eq), the 4-(3,4-dichlorobenzyl)piperidine (892 mg, 3.65 mmol, 1.3 eq) obtained in Step 1, and triphenylphosphine (1.105 g, 4.21 mmol, 1.5 eq) in tetrahydrofuran (THF) at 0°C, diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was washed sequentially with H₂O and brine and dried over anhydrous Na₂SO₄, and then the solvent was removed under reduced pressure. Then, the reaction mixture was separated and purified by MPLC to obtain the 1-((5-bromo-4H-1,2,4-triazol-3-yl)methyl)-4-(3,4-dichlorobenzyl)piperidine compound.

Step 3: After dissolving the 1-((5-bromo-4H-1,2,4-triazol-3-yl)methyl)-4-(3,4-dichlorobenzyl)piperidine (1 eq) synthesized in Step 1, (1-(tert-butoxycarbonyl)-5-fluoro-1H-indol-2-yl)boronic acid (1.3 eq), Li₂CO₃ (2 eq), and the catalyst Pd(Ph₃P)₄ (0.1 eq) in 1,4-dioxane:water (3:1) in a microwave vial, the reaction mixture was reacted in the Biotage microwave at 130°C for 30 minutes. After completion of the reaction, the resultant was subjected to extraction with dichloromethane, the organic layer was washed sequentially with H₂O and brine and dried over anhydrous Na₂SO₄, and then the solvent was removed under reduced pressure. Then, the reaction mixture was separated and purified by MPLC to obtain the compound of Example 29.

The compounds of Examples 30 and 31 were obtained using R listed in Table 4 below instead of (1-(tert-butoxycarbonyl)-5-fluoro-1H-indol-2-yl)boronic acid in Step 2, while using the same method as in Example 29.

**[Table 4]**

| Example | R | Structure |
|---|---|---|
| 30 | 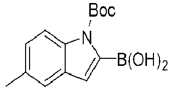 | 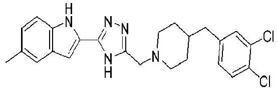 |
| 31 | 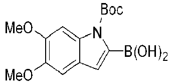 | 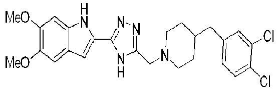 |

### <Example 32> Preparation of 2-(5-((4-(1H-indol-3-yl)piperidin-1-yl)methyl)-4H-1,2,4-triazol-3-yl)-5-fluoro-1H-indole

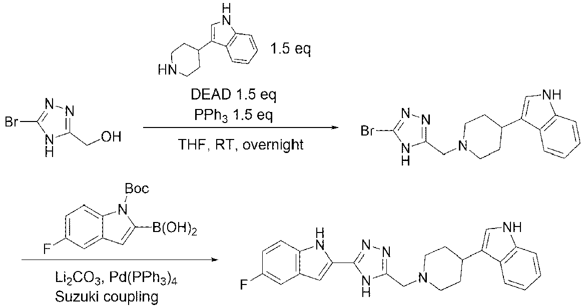

Step 1: After dissolving (5-bromo-4H-1,2,4-triazol-3-yl)methanol (100 mg, 0.562 mmol, 1 eq), 3-(piperidin-4-yl)-1H-indole (146 mg, 0.730 mmol, 1.3 eq), and triphenylphosphine (221 mg, 0.843 mmol, 1.5 eq) in tetrahydrofuran (THF) at 0°C, a diethyl azodicarboxylate (DEAD) solution (0.367 ml, 0.843 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was washed sequentially with H₂O and brine and dried over anhydrous Na₂SO₄, and then the solvent was removed under reduced pressure. Then, the reaction mixture was separated and purified by MPLC to obtain the 3-(1-((5-bromo-4H-1,2,4-triazol-3-yl)methyl)piperidin-4-yl)-1H-indole compound.

Step 2: After dissolving the 3-(1-((5-bromo-4H-1,2,4-triazol-3-yl)methyl)piperidin-4-yl)-1H-indole (1 eq) synthesized in Step 1, (1-(*tert*-butoxycarbonyl)-5-fluoro-1H-indol-2-yl)boronic acid (1.3 eq), Li₂CO₃ (2 eq), and the catalyst Pd(Ph₃P)₄ (0.1 eq) in 1,4-dioxane:water (3:1) in a microwave vial, the reaction mixture was reacted in the Biotage microwave at 130°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the compound of Example 32.

### <Example 33> Preparation of 2-((4-benzylpiperidin-1-yl)methyl)-5-(1H-indol-2-yl)-1,3,4-oxadiazole

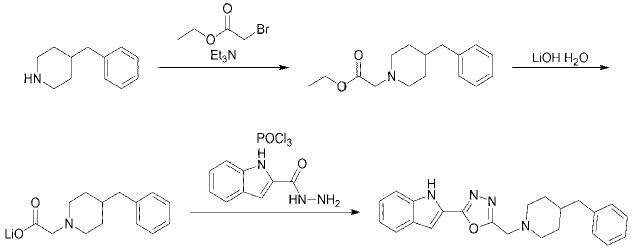

Step 1: After dissolving 4-benzylpiperidine (0.502 mL, 2.85 mmol), ethyl 2-bromoacetate (0.475 mL, 4.28 mmol), and triethylamine (0.390 mL, 2.85 mmol) in toluene, the reactants were stirred at 80°C for 12 hours. After completion of the reaction, water was added dropwise thereto. The resultant was subjected to extraction with ethyl acetate, the organic layer was sequentially washed with H₂O and brine and dried over anhydrous Na₂SO₄, and the solvent was removed under reduced pressure. Then, the reaction mixture was separated and purified by MPLC to obtain ethyl 2-(4-benzylpiperidin-1-yl)acetate.

Step 2: After dissolving the ethyl 2-(4-benzylpiperidin-1-yl)acetate (0.4 g, 1.530 mmol) synthesized in Step 1 in tetrahydrofuran (THF):water (1:1), the reactants were cooled to 0°C. Lithium hydroxide hydrate (0.071 g, 1.684 mmol) was added dropwise thereto at 0°C, and the mixture was stirred for 30 minutes. Then, the reactants were heated to room temperature and stirred at room temperature for 12 hours. After completion of the reaction, ethyl acetate was added dropwise thereto for extraction, and the solvent and a water layer were removed under reduced pressure. The obtained lithium 2-(4-benzylpiperidin-1-yl)acetate was used in the next reaction without purification.

Step 3: After dissolving 1H-indol-2-carbohydrazide (0.285 mmol) and lithium 2-(4-benzylpiperidin-1-yl)acetate (0.314 mmol) in POCl₃ (2 M), the resultant was stirred at 110°C for 3 hours. After completion of the reaction, ice water was added dropwise thereto and the mixture was stirred for 10 minutes. Ethyl acetate was added dropwise thereto for extraction, the organic layer was sequentially washed with H₂O and brine and dried over anhydrous Na₂SO₄, and the solvent was removed under reduced pressure. Then, the reaction mixture was separated and purified by MPLC to obtain the compound of Example 33.

### <Example 34> Preparation of 2-((4-(3,4-dichlorobenzyl)piperidin-1-yl)methyl)-5-(1H-indol-2-yl)-1,3,4-oxadiazole

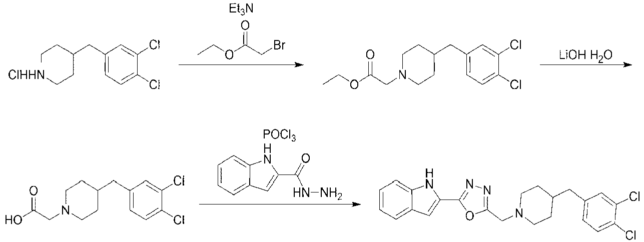

Step 1: After dissolving a 4-(3,4-dichlorobenzyl)piperidine HCl salt (0.3 g, 1.069 mmol), ethyl 2-bromoacetate (0.178 mL, 1.604 mmol), and triethylamine (0.146 mL, 1.069 mmol) in toluene, the reactants were stirred at 80°C for 12 hours. After completion of the reaction, water was added dropwise thereto, and the resultant was extracted with ethyl acetate. The organic layer was sequentially washed with H₂O and brine and dried over anhydrous Na₂SO₄, and the solvent was removed under reduced pressure. Then, the reaction mixture was separated and purified by MPLC to obtain ethyl 2-(4-(3,4-dichlorobenzyl)piperidin-1-yl) acetate.

Step 2: After dissolving the 2-(4-(3,4-dichlorobenzyl)piperidin-1-yl)acetate (0.17 g, 0.515 mmol) synthesized in Step 1 in tetrahydrofuran (THF):water (1:1), and the reactants were cooled to 0°C. Lithium hydroxide hydrate (0.024 g, 0.566 mmol) was added dropwise thereto at 0°C, and the mixture was stirred for 30 minutes. Then, the reactants were heated to room temperature and stirred at room temperature for 12 hours. After completion of the reaction, ethyl acetate was added dropwise thereto for extraction, and the solvent and a water layer were removed under reduced pressure. The obtained 2-(4-(-(3,4-dichlorobenzyl)piperidin-1-yl)acetic acid was used in the next reaction without purification.

Step 3: After dissolving 1H-indol-2-carbohydrazide (0.285 mmol) and 2-(4-(-(3,4-dichlorobenzyl)piperidin-1-yl)acetic acid (0.314 mmol) in POCl₃ (2 M), the resultant was stirred at 110°C for 3 hours. After completion of the reaction, ice water was added dropwise thereto and the mixture was stirred for 10 minutes. Ethyl acetate was added dropwise thereto for extraction, the organic layer was sequentially washed with H₂O and brine and dried over anhydrous Na₂SO₄, and the solvent was removed under reduced pressure. Then, the reaction mixture was separated and purified by MPLC to obtain the compound of Example 34.

### <Example 35> Preparation of 2-(5-((4-benzylpiperidin-1-yl)methyl)-4H-1,2,4-triazol-3-yl)-1H-indol-5,6-diol

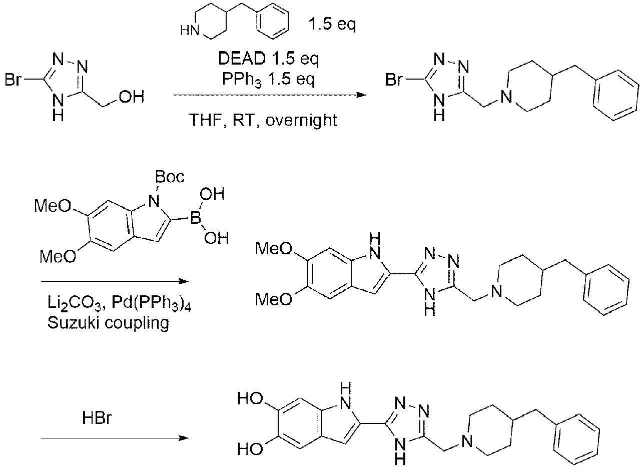

Step 1: After dissolving (5-bromo-4H-1,2,4-triazol-3-yl)methanol (500 mg, 2.81 mmol, 1 eq), 4-benzylpiperidine (0.741 mL, 4.21 mmol, 1.5 eq), and triphenylphosphine (1.105 g, 4.21 mmol, 1.5 eq) in tetrahydrofuran (THF) at 0°C, a diethyl azodicarboxylate (DEAD) solution (DEAD) (1.911 ml, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 2: After dissolving the 4-benzyl-1-((5-bromo-4H-1,2,4-triazol-3-yl)methyl)piperidine (1eq) synthesized in Step 1, (1-(tert-butoxycarbonyl)-5,6-dimethoxy-1H-indol-2-yl)boronic acid (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After once again dropwisely adding (1-(tert-butoxycarbonyl)-5,6-dimethoxy-1H-indol-2-yl)boronic acid (1.3 eq), Li₂CO₃ (2 eq), and the catalyst Pd(Ph₃P)₄ (0.2 eq) to the reactants, the reactants were allowed to react in the Biotage microwave at 160°C for 30 minutes. After completion of the reaction, the resultant was subjected to extraction with dichloromethane, the organic layer was sequentially washed with H₂O and brine and dried over anhydrous Na₃SO₄, and the solvent was removed under reduced pressure. Then, the reaction mixture was separated and purified by MPLC to obtain 2-(5-((4-benzylpiperidin-1-yl)methyl)-4H-1,2,4-triazol-3-yl)-5,6-dimethoxy-1H-indole.

Step 3: After dissolving the 2-(5-((4-benzylpiperidin-1-yl)methyl)-4H-1,2,4-triazol-3-yl)-5,6-dimethoxy-1H-indole (1 eq) synthesized in Step 2 in acetic acid, hydrogen bromate (an acetic acid solution) (30 eq) was added dropwise thereto at room temperature. The reaction mixture was refluxed and stirred for 12 hours. After completion of the reaction, the solvent was removed under reduced pressure, and the resultant was dissolved in a mixed solvent of ethyl acetate and tetrahydrofuran (THF). A saturated aqueous solution of NaHCO₃ was added dropwise, and the resultant was subjected to extraction with DCM, the organic layer was sequentially washed with H₂O and brine and dried over anhydrous Na₂SO₄, and the solvent was removed under reduced pressure. Then, the resultant was separated and purified by MPLC and Prep HPLC to obtain the compound of Example 35.

### <Example 36> Preparation of 3-(5-((4-benzylpiperidin-1-yl)methyl)-4H-1,2,4-triazol-3-yl)-5-methoxy-1H-indole

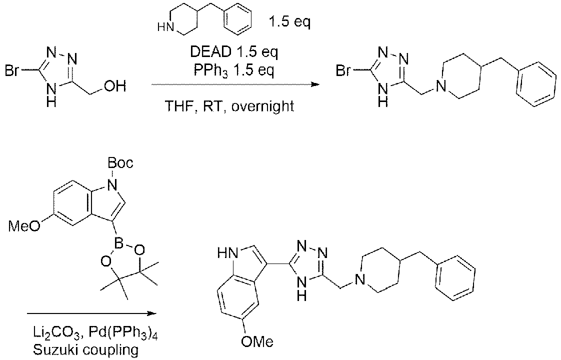

Step 1: After dissolving (5-bromo-4H-1,2,4-triazol-3-yl)methanol (500 mg, 2.81 mmol, 1 eq), 4-benzylpiperidine (0.741 ml, 4.21 mmol, 1.5 eq), and triphenylphosphine (1.105 g, 4.21 mmol, 1.5 eq) in tetrahydrofuran (THF) at 0°C, diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 2: After dissolving the 4-benzyl-1-((5-bromo-4H-1,2,4-triazol-3-yl)methyl)piperidine (1 eq) synthesized in Step 1, tert-butyl 5-methoxy-3-(4,4,5,5-tetramethyl-1,3,2-dioxaborolan-2-yl)-1H-indol-1-carboxylate (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain the compound of Example 36.

The compounds of Examples 37 to 42 were obtained using R listed in Table 5 below instead of *tert*-butyl 5-methoxy-3-(4,4,5,5-tetramethyl-1,3,2-dioxaborolan-2-yl)-1H-indol-1-carboxylate in Step 2, while using the same method as in Example 36.

**[Table 5]**

| Example | R | Chemical Structures of Examples |
|---|---|---|
| 37 | 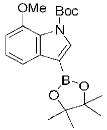 | 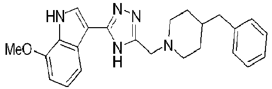 |
| 38 | 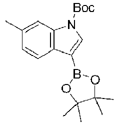 | 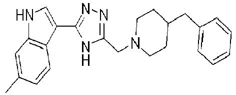 |
| 39 | 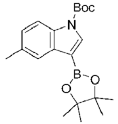 | 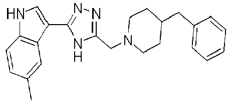 |
| 40 | 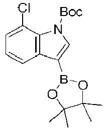 | 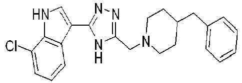 |
| 41 | 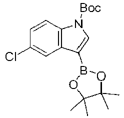 | 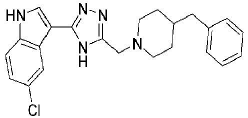 |
| 42 | 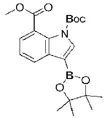 | 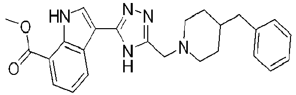 |

### <Example 43> Preparation of 3-(5-((4-benzylpiperidin-1-yl)methyl)-4H-1,2,4-triazol-3-yl)-1H-indol-5-amine

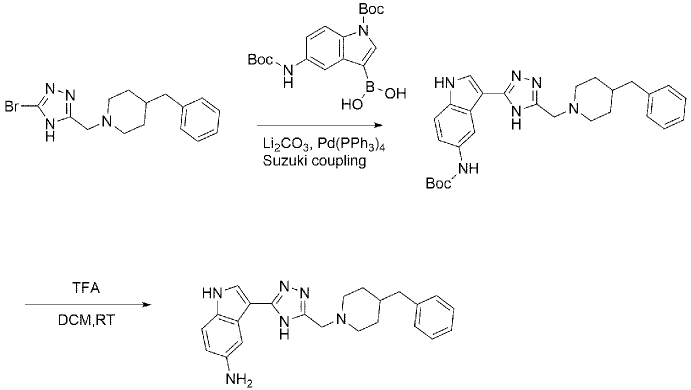

Step 1: After dissolving the synthesized 4-benzyl-1-((5-bromo-4H-1,2,4-triazol-3-yl)methyl)piperidine (1 eq), (1-(tert-butoxycarbonyl)-5-((*tert*-butoxycarbonyl)amino)-1H-indol-3-yl)boronic acid (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain *tert*-butyl (3-(5-((4-benzylpiperidin-1-yl)methyl)-4H-1,2,4-triazol-3-yl)-1H-indol-5-yl)carbamate.

Step 2: After dissolving the *tert*-butyl (3-(5-((4-benzylpiperidin-1-yl)methyl)-4H-1,2,4-triazol-3-yl)-1H-indol-5-yl)carbamate (1 eq) synthesized in Step 1 in DCM, an excess of TFA (50 eq) was added dropwise thereto and reacted at room temperature for 3 hours. After completion of the reaction, neutralization was performed using a supersaturated aqueous solution of sodium carbonate, followed by extraction with dichloromethane. The resultant was dried over anhydrous Na₂SO₄, and the solvent was removed under reduced pressure. Then, the reaction mixture was separated and purified by Prep HPLC to obtain the compound of Example 43 in the form of a white solid.

### <Example 44> Preparation of 3-(5-((4-(3,4-dichlorobenzyl)piperidin-1-yl)methyl)-4H-1,2,4-triazol-3-yl)-5-methoxy-1H-indole

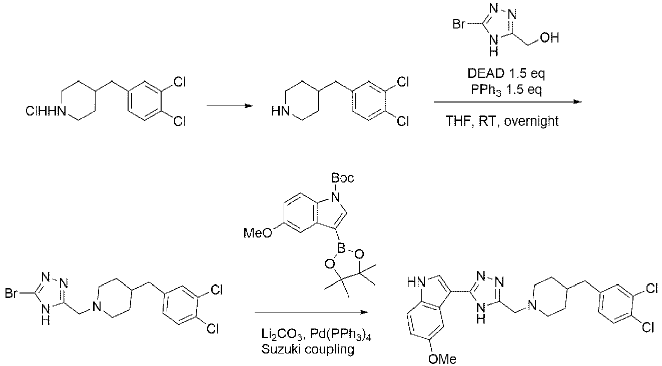

Step 1: After dissolving a 4-(3,4-dichlorobenzyl)piperidine HCl salt in dichloromethane, the pH of the resultant was adjusted to 12 or more by washing with an aqueous solution of 6 M NaOH. After drying the solvent of the organic layer over anhydrous Na₂SO₄, the solvent was removed under reduced pressure to obtain 4-(3,4-dichlorobenzyl)piperidine.

Step 2: After dissolving (5-bromo-4H-1,2,4-triazol-3-yl)methanol (500 mg, 2.81 mmol, 1 eq), the 4-(3,4-dichlorobenzyl)piperidine (892 mg, 3.65 mmol, 1.3 eq) obtained in Step 1, and triphenylphosphine (1.105 g, 4.21 mmol, 1.5 eq) in tetrahydrofuran (THF) at 0°C, a diethyl azodicarboxylate (DEAD) solution (1.911 ml, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 1-((5-bromo-4H-1,2,4-triazol-3-yl)methyl)-4-(3,4-dichlorobenzyl)piperidine compound.

Step 3: After dissolving the 1-((5-bromo-4H-1,2,4-triazol-3-yl)methyl)-4-(3,4-dichlorobenzyl)piperidine (1 eq) synthesized in Step 1, *tert*-butyl 5-methoxy-3-(4,4,5,5-tetramethyl-1,3,2-dioxaborolan-2-yl)-1H-indol-1-carboxylate (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain the compound of Example 44.

The compound of Example 45 was obtained using R listed in Table 6 below instead of *tert*-butyl 5-methoxy-3-(4,4,5,5-tetramethyl-1,3,2-dioxaborolan-2-yl)-1H-indol-1-carboxylate, while using the same method as in Example 44.

**[Table 6]**

| Example | R | Chemical Structure of Example |
|---|---|---|
| 45 | 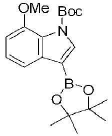 | 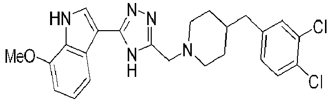 |

### <Example 46> Preparation of 4-(5-((4-benzylpiperidin-1-yl)methyl)-4H-1,2,4-triazol-3-yl)benzonitrile

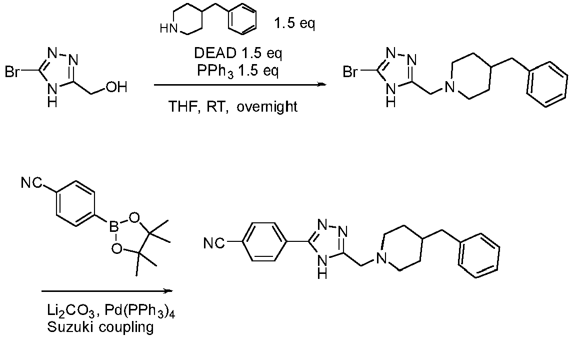

Step 1: After dissolving (5-bromo-4H-1,2,4-triazol-3-yl)methanol (500 mg, 2.81 mmol, 1 eq), 4-benzylpiperidine (0.741 mL, 4.21 mmol, 1.5 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 2: After dissolving the 4-benzyl-1-((5-bromo-4H-1,2,4-triazol-3-yl)methyl)piperidine (1 eq) synthesized in Step 1, 4-(4,4,5,5-tetramethyl-1,3,2-dioxaborolan-2-yl)benzonitrile (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After dropwisely adding once again 4-(4,4,5,5-tetramethyl-1,3,2-dioxaborolan-2-yl)benzonitrile (1.3 eq), Li₂CO₃ (2 eq), and the catalyst Pd(Ph₃P)₄ (0.2 eq) to the reactants, the reactants were reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain 4-(5-((4-benzylpiperidin-1-yl)methyl)-4H-1,2,4-triazol-3-yl)benzonitrile.

The compound of Example 55 was obtained using R listed in Table 7 below instead of 4-(4,4,5,5-tetramethyl-1,3,2-dioxaborolan-2-yl)benzonitrile in Step 2, while using the same method as in Example 46.

**[Table 7]**

| Example | R | Structure |
|---|---|---|
| 47 | 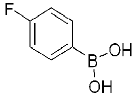 | 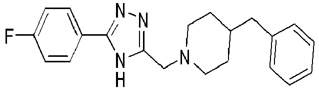 |
| 48 | 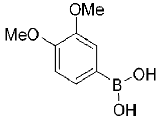 | 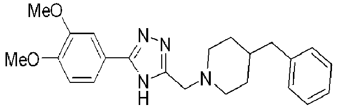 |
| 49 | 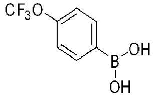 | 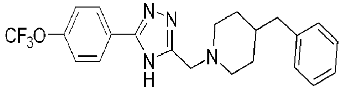 |
| 50 | 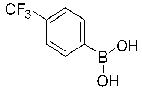 | 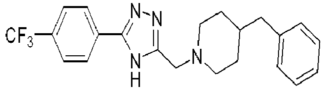 |
| 51 | 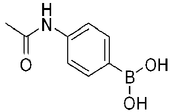 | 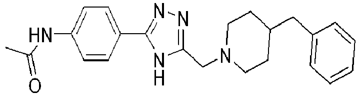 |
| 52 | 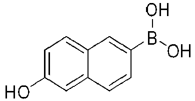 | 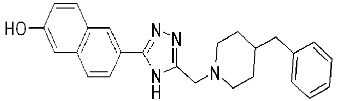 |
| 53 | 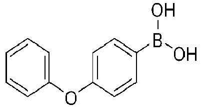 | 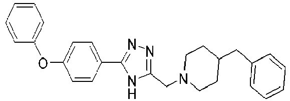 |
| 54 | 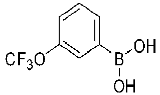 | 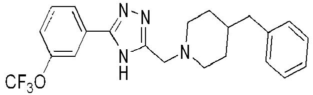 |
| 55 | 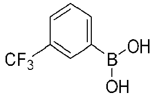 | 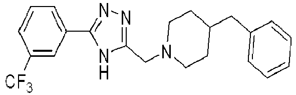 |

### <Example 56> Preparation of 3-(5-((4-benzylpiperidin-1-yl)methyl)-4H-1,2,4-triazol-3-yl)-6-methoxy-1H-indole

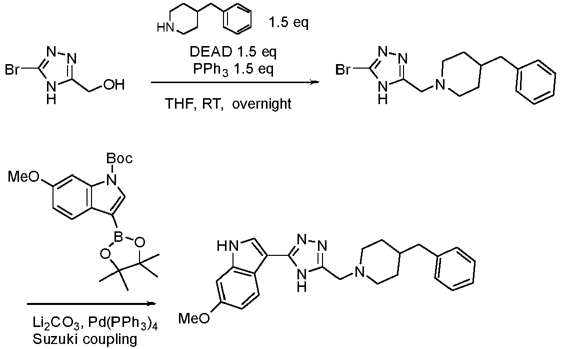

Step 1: After dissolving (5-bromo-4H-1,2,4-triazol-3-yl)methanol (500 mg, 2.81 mmol, 1 eq), 4-benzylpiperidine (0.741 mL, 4.21 mmol, 1.5 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 2: After dissolving the 4-benzyl-1-((5-bromo-4H-1,2,4-triazol-3-yl)methyl)piperidine (1 eq) synthesized in Step 1, *tert*-butyl 6-methoxy-3-(4,4,5,5-tetramethyl-1,3,2-dioxaborolan-2-yl)-1H-indol-1-carboxylate (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain the title compound 3-(5-((4-benzylpiperidin-1-yl)methyl)-4H-1,2,4-triazol-3-yl)-6-methoxy-1H-indole.

### <Example 57> Preparation of 2-(5-((4-benzylpiperidin-1-yl)methyl)-4H-1,2,4-triazol-3-yl)-1H-indol-5-carboxamide

After dissolving the 2-(5-((4-benzylpiperidin-1-yl)methyl)-4H-1,2,4-triazol-3-yl)-1H-indol-5-carboxylic acid (1 eq) of Example 28 in DMF, HBTU (3.5 eq), ammonium chloride (5 eq), and triethylamine (5 eq) were added dropwise thereto at 0°C. The reactants were stirred at room temperature for 12 hours.

After completion of the reaction, the resultant was subjected to extraction with ethyl acetate, the organic layer was sequentially washed with a saturated aqueous solution of NaHCO₃ and brine and dried over anhydrous Na₂SO₄, and the solvent was removed under reduced pressure. Then, the reaction mixture was separated and purified by MPLC to obtain the title compound 2-(5-((4-benzylpiperidin-1-yl)methyl)-4H-1,2,4-triazol-3-yl)-1H-indol-5-carboxamide.

### <Example 58> Preparation of 4-(5-((4-benzylpiperidin-1-yl)methyl)-4H-1,2,4-triazol-3-yl)-N-(2-(dimethylamino)ethyl)benzoamide

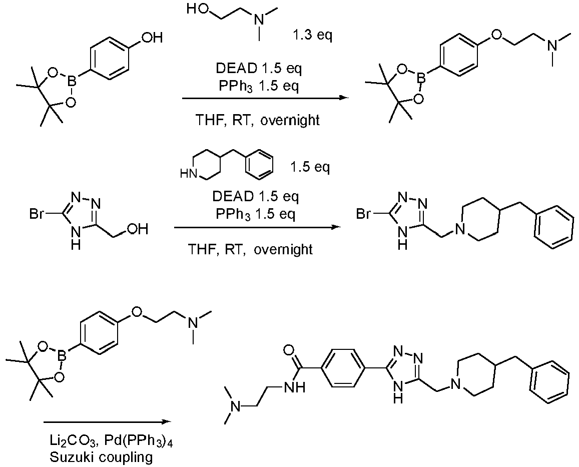

Step 1: After dissolving 4-(4,4,5,5-tetramethyl-1,3,2-dioxaborolan-2-yl)phenol (500 mg, 2.272 mmol), 2-(dimethylamino)ethan-1-ol (0.296 ml, 2.95 mmol), and triphenylphosphine (775 mg, 2.95 mmol, 1.3 eq) in tetrahydrofuran (THF) at 0°C, a diethyl azodicarboxylate (DEAD) solution (1.34 mL, 2.95 mmol, 1.3 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the *N*,*N*-dimethyl-2-(4-(4,4,5,5-tetramethyl-1,3,2-dioxaborolan-2-yl)phenoxy)ethan-1-amine compound.

Step 2: After dissolving (5-bromo-4H-1,2,4-triazol-3-yl)methanol (500 mg, 2.81 mmol, 1 eq), 4-benzylpiperidine (0.741 mL, 4.21 mmol,1.5 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 3: After dissolving the 4-benzyl-1-((5-bromo-4H-1,2,4-triazol-3-yl)methyl)piperidine (1 eq) synthesized in Step 2, the *N*,*N*-dimethyl-2-(4-(4,4,5,5-tetramethyl-1,3,2-dioxaborolan-2-yl)phenoxy)ethan-1-amine (1.3 eq) synthesized in Step 1, Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After dropwisely adding once again the *N*,*N-*dimethyl-2-(4-(4,4,5,5-tetramethyl-1,3,2-dioxaborolan-2-yl)phenoxy)ethan-1-amine (1.3 eq), Li₂CO₃ (2 eq), and the catalyst Pd(Ph₃P)₄ (0.2 eq) to the reactants, the reactants were reacted at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain the title compound 4-(5-((4-benzylpiperidin-1-yl)methyl)-4H-1,2,4-triazol-3-yl)-*N*-(2-(dimethylamino)ethyl)benzoamide.

### <Example 59> Preparation of 3-(5-((4-(3,4-dichlorobenzyl)piperidin-1-yl)methyl)-4H-1,2,4-triazol-3-yl)-6-methoxy-1H-indole

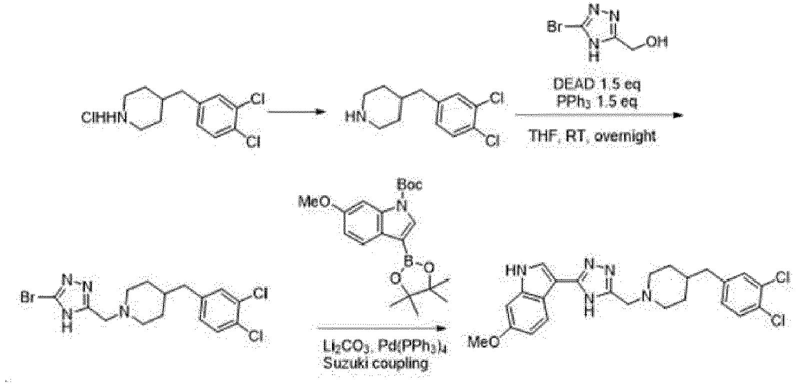

Step 1: After dissolving a 4-(3,4-dichlorobenzyl)piperidine HCl salt in dichloromethane, the pH of the resultant was adjust to 12 or more by washing with an aqueous solution of 6 M NaOH. The solvent in the organic layer was dried over anhydrous Na₂SO₄, and the solvent was removed under reduced pressure to obtain 4-(3,4-dichlorobenzyl)piperidine.

Step 2: After dissolving (5-bromo-4H-1,2,4-triazol-3-yl)methanol (500 mg, 2.81 mmol, 1 eq), the 4-(3,4-dichlorobenzyl)piperidine (892 mg, 3.65 mmol, 1.3 eq) obtained in Step 1, and triphenylphosphine (1.105 g, 4.21 mmol, 1.5 eq) in tetrahydrofuran (THF), a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 1-((5-bromo-4H-1,2,4-triazol-3-yl)methyl)-4-(3,4-dichlorobenzyl)piperidine compound.

Step 3: After dissolving the 1-((5-bromo-4H-1,2,4-triazol-3-yl)methyl)-4-(3,4-dichlorobenzyl)piperidine (1 eq) synthesized in Step 2, *tert*-butyl 6-methoxy-3-(4,4,5,5-tetramethyl-1,3,2-dioxaborolan-2-yl)-1H-indol-1-carboxylate (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the title compound 3-(5-((4-(3,4-dichlorobenzyl)piperidin-1-yl)methyl)-4H-1,2,4-triazol-3-yl)-6-methoxy-1H-indole.

The compounds of Examples 60 to 62 were obtained using R listed in Table 8 below instead of tert-butyl 6-methoxy-3-(4,4,5,5-tetramethyl-1,3,2-dioxaborolan-2-yl)-1H-indol-1-carboxylate in Step 3, while using the same method as in Example 59.

**[Table 8]**

| Example | R | Structure |
|---|---|---|
| 60 | 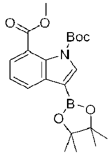 | 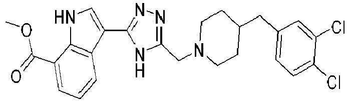 |
| 61 | 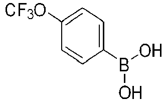 | 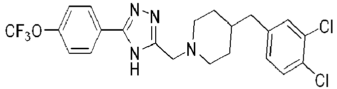 |
| 62 | 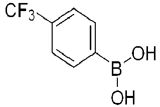 | 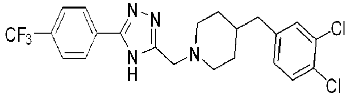 |

### <Example 63> Preparation of methyl 2-amino-4-(5-((4-benzylpiperidin-1-yl)methyl)-4H-1,2,4-triazol-3-yl)benzoate

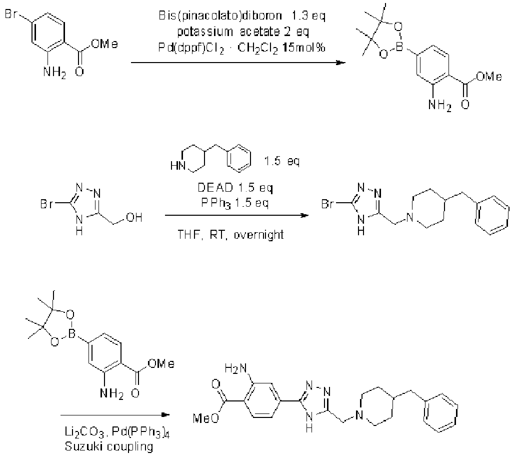

Step 1: After dissolving methyl 2-amino-4-bromobenzoate (500 mg, 2.173 mmol), potassium acetate (427 mg, 4.35 mmol), bis(pinacolato)diboron (717 mg, 2.83 mmol), and Pd(dppf)Cl₂.CH₂Cl₂ (266 mg, 0.326 mmol) in 1,4-dioxane (15 mL), the reaction mixture was degassed with nitrogen. The reactants were stirred at 100°C for 12 hours. After completion of the reaction, the reaction mixture was filtered through celite, and the organic layer was extracted with ethyl acetate. The organic layer was sequentially washed with an aqueous solution of 0.5 M HCl and brine and dried over anhydrous Na₂SO₄, and the solvent was removed under reduced pressure. Then, the resultant was separated and purified by MPLC to obtain the methyl 2-amino-4-(4,4,5,5-tetramethyl-1,3,2-dioxaborolan-2-yl)benzoate compound.

Step 2: After dissolving (5-bromo-4H-1,2,4-triazol-3-yl)methanol (500 mg, 2.81 mmol, 1 eq), 4-benzylpiperidine (0.741 mL, 4.21 mmol, 1.5 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 3: After dissolving the 4-benzyl-1-((5-bromo-4H-1,2,4-triazol-3-yl)methyl)piperidine (1 eq) synthesized in Step 2, the methyl 2-amino-4-(4,4,5,5-tetramethyl-1,3,2-dioxaborolan-2-yl)benzoate (1.3 eq) synthesized in Step 1, Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain the title compound methyl 2-amino-4-(5-((4-benzylpiperidin-1-yl)methyl)-4H-1,2,4-triazol-3-yl)benzoate.

### <Example 64> Preparation of 2-(5-((4-benzylpiperazin-1-yl)methyl)-4H-1,2,4-triazol-3-yl)-5-fluoro-1H-indole

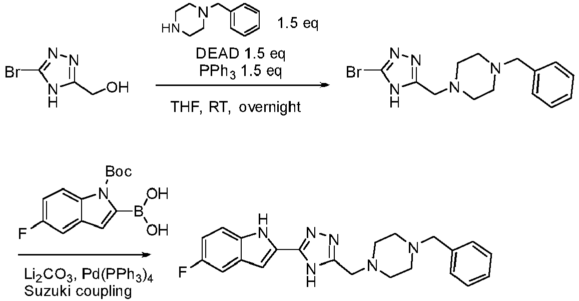

Step 1: After dissolving (5-bromo-4H-1,2,4-triazol-3-yl)methanol (500 mg, 2.81 mmol, 1 eq), 1-benzylpiperazine (0.594 mg, 3.37 mmol, 1.2 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 1-benzyl-4-((5-bromo-4H-1,2,4-triazol-3-yl)methyl)piperazine compound.

Step 2: After dissolving the 1-benzyl-4-((5-bromo-4H-1,2,4-triazol-3-yl)methyl)piperazine (1 eq) synthesized in Step 1, (1-(*tert*-butoxycarbonyl)-5-fluoro-1H-indol-2-yl)boronic acid (1.3 eq), Li₂CO₃ (2 eq), and the catalyst Pd(Ph₃P)₄ (0.2 eq) in 4-dioxane: water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain the title compound 2-(5-((4-benzylpiperazin-1-yl)methyl)-4H-1,2,4-triazol-3-yl)-5-fluoro-1H-indole.

The compound of Example 65 was obtained using R listed in Table 9 below instead of (1-(*tert*-butoxycarbonyl)-5-fluoro-1H-indol-2-yl)boronic acid in Step 2, while using the same method as in Example 64.

**[Table 9]**

| Example | R | Structure |
|---|---|---|
| 65 | 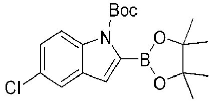 | 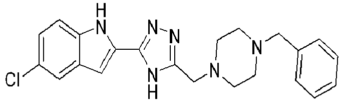 |

### <Example 66> Preparation of N-(2-(5-((4-benzylpiperidin-1-yl)methyl)-4H-1,2,4-triazol-3-yl)-1H-indol-5-yl)acetamide

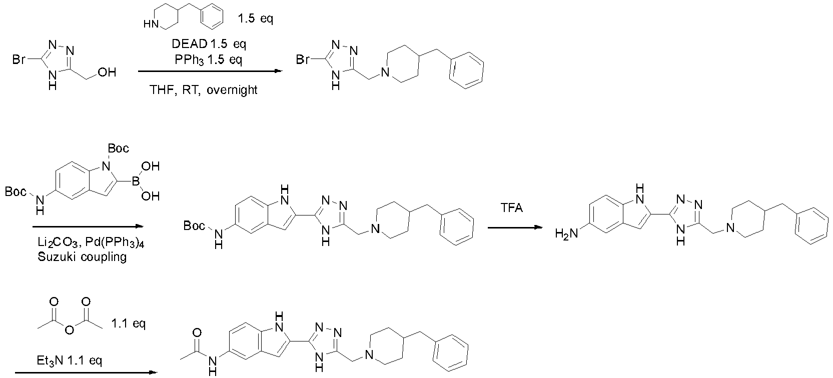

Step 1: After dissolving (5-bromo-4H-1,2,4-triazol-3-yl)methanol (500 mg, 2.81 mmol, 1 eq), 4-benzylpiperidine (0.741 mL, 4.21 mmol, 1.5 eq), and triphenylphosphine (1.105 g, 4.21 mmol, 1.5 eq) in tetrahydrofuran (THF) at 0°C, a diethyl azodicarboxylate (DEAD) solution (1.911 mL, 4.21 mmol, 1.5 eq) was added dropwise thereto. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain the 4-benzyl-1-((5-bromo-4H-1,2,4-triazol-3-yl)methyl)piperidine compound.

Step 2: After dissolving the 1-((5-bromo-4H-1,2,4-triazol-3-yl)methyl)-4-(3,4-dichlorobenzyl)piperidine (1 eq) synthesized in Step 1, (1-(*tert*-butoxycarbonyl)-5-((tert-butoxycarbonyl)amino)-1H-indol-2-yl)boronic acid (1.3 eq), Li₂CO₃ (2 eq), and the catalyst Pd(Ph₃P)₄ (0.2 eq) in 4-dioxane:water (3:1) in a microwave vial, the reaction mixture was reacted in the Biotage microwave at 160°C for 30 minutes. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC and Prep HPLC to obtain *tert*-butyl (2-(5-((4-benzylpiperidin-1-yl)methyl)-4H-1,2,4-triazol-3-yl)-1H-indol-5-yl)carbamate.

Step 3: After dissolving the *tert*-butyl (2-(5-((4-benzylpiperidin-1-yl)methyl)-4H-1,2,4-triazol-3-yl)-1H-indol-5-yl)carbamate (1 eq) synthesized in Step 2 in DCM, an excess of TFA (50 eq) was added dropwise thereto and reacted at room temperature for 3 hours. After completion of the reaction, neutralization was performed using an aqueous solution of saturated NaHCO₃, and the resultant was subjected to extraction with dichloromethane. The resultant was dried over anhydrous Na₂SO₄, and the solvent was removed under reduced pressure. 2-(5-((4-Benzylpiperidin-1-yl)methyl)-4H-1,2,4-triazol-3-yl)-1H-indol-5-amine was obtained without further purification.

Step 4: After dissolving 2-(5-((4-benzylpiperidin-1-yl)methyl)-4H-1,2,4-triazol-3-yl)-1H-indol-5-amine (1 eq) in DCM, triethylamine (1.1 eq) and acetic anhydride (1.1 eq) were added dropwise thereto, and the mixture was stirred at room temperature for 1.5 hours.

After completion of the reaction, the resultant was subjected to extraction with dichloromethane and methanol, the organic layer was sequentially washed with H₂O and brine and dried over anhydrous Na₂SO₄, and the solvent was removed under reduced pressure. Then, the reaction mixture was separated and purified by MPLC and Prep TLC to obtain the title compound N-(2-(5-((4-benzylpiperidin-1-yl)methyl)-4H-1,2,4-triazol-3-yl)-1H-indol-5-yl)acetamide.

### <Example 67> 2-(5-((4-benzylpiperidin-1-yl)methyl)-4H-1,2,4-triazol-3-yl)-1H-indol-5-ol

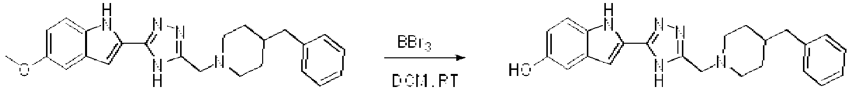

After dissolving the 2-(5-((4-benzylpiperidin-1-yl)methyl)-4H-1,2,4-triazol-3-yl)-5-methoxy-1H-indole (1 eq) of Example 13 in dichloromethane, boron tribromide (2.5 eq) was added dropwise at 0°C. The reactants were stirred at room temperature for 12 hours under nitrogen atmosphere. After completion of the reaction, the solvent was removed under reduced pressure. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain 2-(5-((4-benzylpiperidin-1-yl)methyl)-4H-1,2,4-triazol-3-yl)-1H-indol-5-ol.

### <Example 68> (4-benzylpiperidin-1-yl)(5-(5-fluoro-1H-indol-2-yl)-4H-1,2,4-triazol-3-yl)methanone

The compound of Example 68 was obtained using R listed in Table 10 below instead of (1-(*tert*-butoxycarbonyl)-5-yl-fluoro-1H-indol-2-yl)boronic acid in Step 1, while using the same method as in Example 11.

**[Table 10]**

| Example | R | Structure |
|---|---|---|
| 68 | 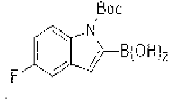 | 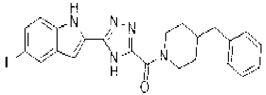 |

### <Example 69> 2-(5-((4-benzylpiperidin-1-yl)methyl)-4H-1,2,4-triazol-3-yl)-6-butoxy-1H-indole

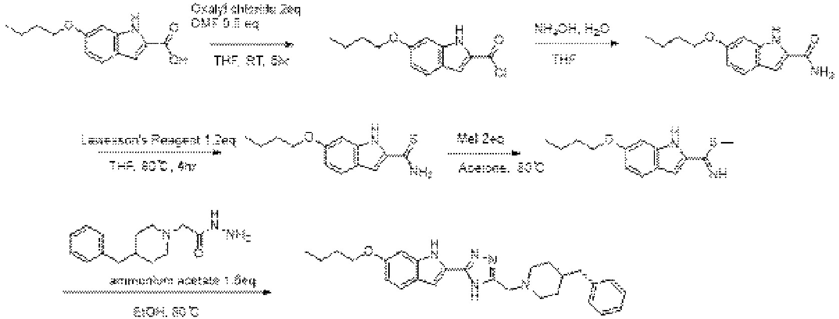

Step 1: After dissolving 6-butoxy-1H-indol-2=carboxylic acid (200 mg, 0.857 mmol, 1 eq) and dimethylformaldehyde (33 µL, 0.429 mmol, 0.5 eq) in tetrahydrofuran (THF), oxalyl chloride (150 µL, 1.1715 mmol, 2 eq) was added dropwise at 0°C. The reactants were stirred at room temperature for 5 hours under nitrogen atmosphere. After completion of the reaction, the solvent was removed under reduced pressure to obtain a 6-butoxy-1H-indol-2-carbonyl chloride compound.

Step 2: After dissolving the 6-butoxy-1H-indol-2-carbonyl chloride (1 eq) synthesized in Step 1 in tetrahydrofuran, ammonium hydroxide was added dropwise at 0°C to adjust the pH of the resultant to 9-10 and reacted at room temperature for 1 hour, and 5 mL of water was added dropwise thereto, followed by stirring for 30 minutes. The resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain tert-butyl 6-butoxy-1H-indol-2-carboxamide.

Step 3: After dissolving the 6-butoxy-1H-indol-2-carboxamide (1 eq) synthesized in Step 2 and Lawesson's Reagent (1.2 eq) in tetrahydrofuran, the reactants were stirred at 80°C for 4 hours. After completion of the reaction, the solvent was removed under reduced pressure, and the resultant was solidified with methanol to obtain 6-butoxy-1H-indol-2-carbothioamide.

Step 4: After dissolving the 6-butoxy-1H-indol-2-carbothioamide (1 eq) synthesized in Step 3 and methyl iodide (2 eq) in acetone, the reactants were stirred at 80°C for 4 hours. After completion of the reaction, the solvent was removed under reduced pressure, and the resultant was reacted at room temperature for 1 hour, 5 mL of water was added dropwise thereto, and the resultant was stirred for 30 minutes. The resultant was subjected to extraction with dichloromethane, the organic layer was sequentially washed with H₂O and brine and dried over anhydrous Na₂SO₄, and the solvent was removed under reduced pressure to obtain methyl-6-butoxy-1H-indol-2-carbimidothioate.

Step 5: The methyl-6-butoxy-1H-indol-2-carbimidothioate (1 eq) synthesized in Step 4, 2-(4-benzylpiperidin-1-yl)acetohydrazide (1 eq), and ammonium acetate (1.5 eq) were dissolved in ethanol and stirred at 80°C for 6 hours. After completion of the reaction, the resultant was subjected to extraction with dichloromethane, the organic layer was sequentially washed with H₂O and brine and dried over anhydrous Na₂SO₄, and the solvent was removed under reduced pressure. Then, the reaction mixture was separated and purified by MPLC to obtain 2-(5-((4-benzylpiperidin-1-yl)methyl)-4H-1,2,4-triazol-3-yl)-6-butoxy-1H-indole.

The compounds of Examples 70 to 76 were obtained using R listed in Table 11 below instead of 6-butoxy-1H-indol-2=carboxylic acid in Step 1, while using the same method as in Example 69.

**[Table 11]**

| Example | R | Structure |
|---|---|---|
| 70 | 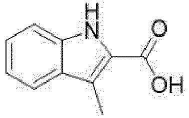 | 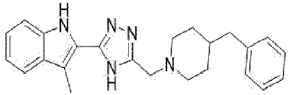 |
| 71 | 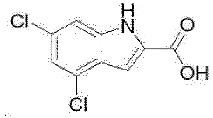 | 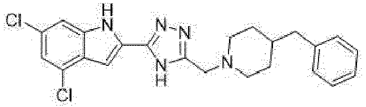 |
| 72 | 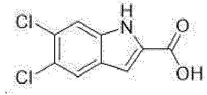 | 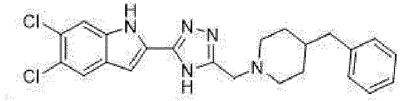 |
| 73 | 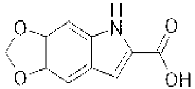 | 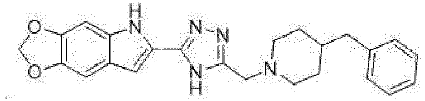 |
| 74 | 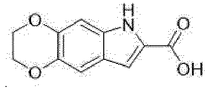 | 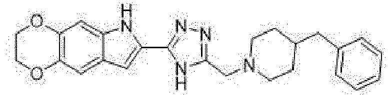 |
| 75 | 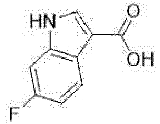 | 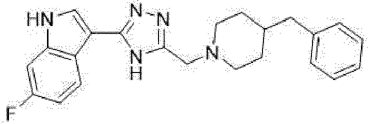 |
| 76 | 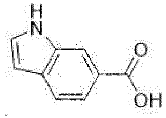 | 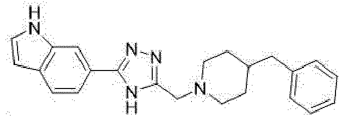 |

The compound names, the results of NMR analysis and the results of mass analysis of Examples 1 to 76 are summarized in Table 12 below.

**[Table 12]**

| Exa mpl e | Compound Name | NMR | Mass |
|---|---|---|---|
| 1 | 2-(5-((4-benzylpiperi din-1-yl)methyl)-4H-1,2,4-triazol-3-yl)-1H-indole | 1H NMR (400 MHz, MeOD) δ 7.57 (d, J = 8.0 Hz, 1H), 7.43 (d, J = 8.3 Hz, 1H), 7.27 - 7.20 (m, 2H), 7.15 (dd, J = 14.1, 7.7 Hz, 4H), 7.04 (t, J = 7.5 Hz, 1H), 7.00 (s, 1H), 3.73 (s, 2H), 2.98 (d, J = 11.7 Hz, 2H), 2.54 (d, J = 7.0 Hz, 2H), 2.15 (t, J = 10.8 Hz, 2H), 1.65 (d, J = 12.4 Hz, 2H), 1.61 - 1.54 (m, 1H), 1.40 - 1.32 (m, 2H). | 372 (M+1 ) |
| 2 | 2-(5-((4-benzylpiperi din-1-yl)methyl)-4H-1,2,4-triazol-3-yl)-5-fluoro-1H-indole | 1H NMR (400 MHz, MeOD) δ 7.41 (dd, J = 8.9, 4.6 Hz, 1H), 7.25 (d, J = 7.2 Hz, 3H), 7.17 (t, J = 6.8 Hz, 3H), 7.00 (s, 1H), 6.96 (t, J = 9.1 Hz, 1H), 3.75 (s, 2H), 3.00 (d, J = 11.5 Hz, 2H), 2.56 (d, J = 6.9 Hz, 2H), 2.16 (t, J = 11.4 Hz, 2H), 1.67 (d, J = 12.8 Hz, 2H), 1.63 - 1.54 (m, 1H), 1.44 - 1.32 (m, 2H). | 390. 08 (M+1 ) |
| 3 | 2-(5-((4-phenylpiperi din-1-yl)methyl)-4H-1,2,4-triazol-3-yl)-1H-indole | 1H NMR (400 MHz, MeOD) δ 7.61 (d, J = 8.0 Hz, 1H), 7.46 (d, J = 8.2 Hz, 1H), 7.33 - 7.23 (m, 4H), 7.19 (dd, J = 13.6, 6.7 Hz, 2H), 7.10 - 7.03 (m, 2H), 3.83 (s, 2H), 3.15 (d, J = 11.3 Hz, 2H), 2.56 (dd, J = 15.6, 7.7 Hz, 1H), 2.37 (dd, J = 15.2, 11.1 Hz, 2H), 1.91 - 1.81 (m, 4H). | 358. 01 (M+1 ) |
| 4 | 2-(5-((4-benzylpipera zin-1-yl)methyl)-4H-1,2,4-triazol-3-yl)-1H-indole | 1H NMR (400 MHz, CDCl₃) δ 8.97 (s, 1H), 7.66 (d, J = 7.9 Hz, 1H), 7.40 (d, J = 8.2 Hz, 1H), 7.32 - 7.31 (m, 5H), 7.26 - 7.22 (m, 1H), 7.16 - 7.10 (m, 1H), 7.09 (s, 1H), 3.79 (s, 2H), 3.54 (s, 2H), 2.63 (br, 4H), 2.54 (br, 4H). | 373. 06 (M+1 ) |
| 5 | 2-(5-((4-(3,4-dichlorobenz yl)piperidin -1-yl)methyl)-4H-1,2,4-triazol-3-yl)-1H-indole | 1H NMR (400 MHz, MeOD) δ 7.60 (d, J = 8.1 Hz, 1H), 7.45 (d, J = 8.2 Hz, 1H), 7.42 (d, J = 8.2 Hz, 1H), 7.36 (s, 1H), 7.18 (t, J = 7.6 Hz, 1H), 7.12 (d, J = 8.0 Hz, 1H), 7.06 (t, J = 7.5 Hz, 1H), 7.03 (s, 1H), 4.65 (s, 1H), 3.75 (s, 2H), 3.01 (d, J = 11.2 Hz, 2H), 2.57 (d, J = 6.8 Hz, 2H), 2.18 (t, J = 11.6 Hz, 2H), 1.66 (d, J = 14.0 Hz, 2H), 1.62 - 1.53 (m, 1H), 1.44 - 1.32 (m, 2H). | 440. 01 (M) |
| 6 | 2-(5-((4-(4-methoxybenzy l)piperidin-1-yl)methyl)-4H-1,2,4-triazol-3-yl)-1H-indole | 1H NMR (400 MHz, MeOD) δ 7.60 (d, J = 7.9 Hz, 1H), 7.45 (d, J = 8.2 Hz, 1H), 7.22 - 7.14 (m, 1H), 7.05 (dd, J = 14.7, 7.2 Hz, 4H), 6.83 (d, J = 8.4 Hz, 2H), 3.77 (s, 3H), 3.75 (s, 2H), 3.00 (d, J = 11.7 Hz, 2H), 2.50 (d, J = 7.0 Hz, 2H), 2.21 - 2.13 (m, 2H), 1.70 - 1.63 (m, 2H), 1.57 - 1.49 (m, 1H), 1.41 - 1.32 (m, 2H). | 402. 11 (M+1 ) |
| 7 | 2-(5-((4-(4-chlorobenzyl )piperidin-1-yl)methyl)-4H-1,2,4-triazol-3-yl)-1H-indole | 1H NMR (400 MHz, MeOD) δ 7.50 (d, J = 7.8 Hz, 1H), 7.35 (d, J = 8.3 Hz, 1H), 7.18 (d, J = 7.9 Hz, 2H), 7.09 (dd, J = 13.1, 7.5 Hz, 3H), 7.00 - 6.94 (m, 1H), 6.93 (s, 1H), 3.66 (s, 2H), 2.91 (d, J = 10.8 Hz, 2H), 2.47 (d, J = 6.9 Hz, 2H), 2.11 - 2.01 (m, 2H), 1.62 - 1.52 (m, 2H), 1.52 - 1.44 (m, 1H), 1.34 - 1.24 (m, 2H). | 406. 06 (M+1) |
| 8 | 2-(5-((4-(3-chlorobenzyl )piperidin-1-yl)methyl)-4H-1,2,4-triazol-3-yl)-1H-indole | 1H NMR (400 MHz, MeOD) δ 7.60 (d, J = 7.8 Hz, 1H), 7.45 (d, J = 8.2 Hz, 1H), 7.29 - 7.23 (m, 1H), 7.22 - 7.15 (m, 3H), 7.11 (d, J = 7.5 Hz, 1H), 7.09 - 7.03 (m, 1H), 7.02 (s, 1H), 3.75 (s, 2H), 3.01 (d, J = 11.0 Hz, 2H), 2.57 (d, J = 6.8 Hz, 2H), 2.17 (t, J = 12.1 Hz, 2H), 1.71 - 1.63 (m, 2H), 1.63 - 1.55 (m, 1H), 1.42 - 1.30 (m, 2H). | 406. 06 (M+1) |
| 9 | 4-benzyl-1-((5-phenyl-4H-1,2,4-triazol-3-yl)methyl)pi peridine | 1H NMR (400 MHz, CDCl₃) δ 8.07 (d, J = 6.8 Hz, 2H), 7.48 - 7.38 (m, 3H), 7.32 - 7.27 (m, 2H), 7.21 (d, J = 7.2 Hz, 1H), 7.14 (d, J = 7.3 Hz, 2H), 3.75 (s, 2H), 2.90 (d, J = 11.3 Hz, 2H), 2.56 (d, J = 7.0 Hz, 2H), 2.13 (t, J = 11.0 Hz, 2H), 1.67 (d, J = 12.8 Hz, 2H), 1.61 - 1.54 (m, 1H), 1.36 - 1.31 (m, 2H). | 333. 13 (M+1) |
| 10 | 4-benzyl-1-((5-(4-methoxypheny l)-4H-1,2,4-triazol-3-yl)methyl)pi peridine | 1H NMR (400 MHz, CDCl₃) δ 8.00 (d, J = 8.5 Hz, 2H), 7.32 - 7.26 (m, 2H), 7.22 - 7.17 (m, 1H), 7.13 (d, J = 7.3 Hz, 2H), 6.95 (d, J = 8.5 Hz, 2H), 3.85 (s, 3H), 3.73 (s, 2H), 2.91 (d, J = 11.1 Hz, 2H), 2.54 (d, J = 6.9 Hz, 2H), 2.12 (t, J = 11.3 Hz, 2H), 1.65 (d, J = 12.8 Hz, 2H), 1.60 - 1.50 (m, 1H), 1.37 - 1.29 (m, 2H). | 363. 07 (M+1) |
| 11 | (4-benzylpiperi din-1-yl) (5-(4-methoxypheny l)-4H-1,2,4-triazol-3-yl)methanone | 1H NMR (400 MHz, CDCl₃) δ 8.04 (d, J = 8.7 Hz, 2H), 7.31 (t, J = 7.4 Hz, 2H), 7.22 (t, J = 7.3 Hz, 1H), 7.16 (d, J = 7.3 Hz, 2H), 6.97 (d, J = 8.7 Hz, 2H), 5.71 (d, J = 12.9 Hz, 1H), 4.76 (d, J = 12.8 Hz, 1H), 3.86 (s, 3H), 3.17 (t, J = 12.4 Hz, 1H), 2.79 (t, J = 11.9 Hz, 1H), 2.59 (d, J = 6.8 Hz, 2H), 1.96 - 1.86 (m, 1H), 1.83 (d, J = 13.4 Hz, 2H), 1.41 - 1.32 (m, 2H). | 377. 14 (M+1) |
| 12 | 2-(5-((4-benzylpiperi din-1-yl)methyl)-4H-1,2,4-triazol-3-yl)-5,6-dimethoxy-1H-indole | 1H NMR (400 MHz, MeOD) δ 7.14 (t, J = 7.5 Hz, 2H), 7.04 (dd, J = 14.8, 7.1 Hz, 4H), 6.92 (s, 1H), 6.82 (s, 1H), 3.79 (s, 3H), 3.76 (s, 3H), 3.64 (s, 2H), 2.91 (d, J = 11.4 Hz, 2H), 2.45 (d, J = 7.0 Hz, 2H), 2.08 (t, J = 11.2 Hz, 2H), 1.56 (d, J = 13.5 Hz, 2H), 1.51 - 1.43 (m, 1H), 1.31 - 1.23 (m, 2H). | 432. 18 (M+1) |
| 13 | 2-(5-((4-benzylpiperi din-1-yl)methyl)-4H-1,2,4-triazol-3-yl)-5-methoxy-1H-indole | 1H NMR (400 MHz, MeOD) δ 7.20 (d, J = 8.8 Hz, 1H), 7.12 (t, J = 7.4 Hz, 2H), 7.03 (t, J = 7.5 Hz, 3H), 6.96 (d, J = 2.1 Hz, 1H), 6.83 (s, 1H), 6.72 (dd, J = 8.8, 2.2 Hz, 1H), 3.70 (s, 3H), 3.61 (s, 2H), 2.87 (d, J = 11.1 Hz, 2H), 2.42 (d, J = 6.9 Hz, 2H), 2.10 - 1.97 (m, 2H), 1.53 (d, J = 13.3 Hz, 2H), 1.49 - 1.37 (m, 1H), 1.26 (d, J = 16.4 Hz, 2H). | 402. 18 (M+1) |
| 14 | 2-(5-((4-benzylpiperi din-1-yl)methyl)-4H-1,2,4-triazol-3-yl)-5-chloro-1H-indole | 1H NMR (400 MHz, MeOD) δ 7.48 (d, J = 1.9 Hz, 1H), 7.31 (d, J = 8.7 Hz, 1H), 7.16 (t, J = 7.5 Hz, 2H), 7.08 - 7.02 (m, 4H), 6.88 (s, 1H), 3.67 (s, 2H), 2.90 (d, J = 11.6 Hz, 2H), 2.46 (d, J = 7.0 Hz, 2H), 2.15 - 2.04 (m, 2H), 1.57 (d, J = 13.8 Hz, 2H), 1.53 - 1.40 (m, 1H), 1.30 (dd, J = 12.5, 3.6 Hz, 2H). | 406. 15 (M+1) |
| 15 | 2-(5-((4-benzylpiperi din-1-yl)methyl)-4H-1,2,4-triazol-3-yl)-5-methyl-1H-indole | 1H NMR (400 MHz, CDCl₃) δ 8.93 (s, 1H), 7.44 (s, 1H), 7.29 (d, J = 6.9 Hz, 2H), 7.19 (t, J = 7.3 Hz, 1H), 7.14 (d, J = 7.0 Hz, 2H), 7.05 (dd, J = 8.3, 1.2 Hz, 1H), 7.02 (s, 1H), 3.73 (s, 2H), 2.90 (d, J = 11.5 Hz, 2H), 2.55 (d, J = 7.0 Hz, 2H), 2.44 (s, 3H), 2.13 (dd, J = 11.6, 9.7 Hz, 2H), 1.67 (d, J = 13.5 Hz, 2H), 1.62 - 1.52 (m, 1H), 1.38 - 1.30 (m, 2H). | 386. 20 (M+1) |
| 16 | 2-(5-((4-benzylpiperi din-1-yl)methyl)-4H-1,2,4-triazol-3-yl)-6-fluoro-1H-indole | 1H NMR (400 MHz, CDCl₃) δ 8.92 (s, 1H), 7.56 (dd, J = 8.6, 5.3 Hz, 1H), 7.29 (d, J = 7.2 Hz, 2H), 7.23 - 7.17 (m, 1H), 7.14 (d, J = 6.9 Hz, 2H), 7.07 (d, J = 6.8 Hz, 2H), 6.92 - 6.86 (m, 1H), 3.73 (s, 2H), 2.89 (d, J = 11.7 Hz, 2H), 2.56 (d, J = 7.0 Hz, 2H), 2.14 (t, J = 10.9 Hz, 2H), 1.68 (d, J = 13.7 Hz, 2H), 1.63 - 1.52 (m, 1H), 1.40 - 1.28 (m, 2H). | 390. 20 (M+1) |
| 17 | 2-(5-((4-benzylpiperi din-1-yl)methyl)-4H-1,2,4-triazol-3-yl)-1H-indol-5-carbonitrile | 1H NMR (400 MHz, MeOD) δ 7.93 (d, J = 0.8 Hz, 1H), 7.47 (d, J = 8.5 Hz, 1H), 7.33 (dd, J = 8.5, 1.5 Hz, 1H), 7.14 (t, J = 7.4 Hz, 2H), 7.05 (d, J = 5.8 Hz, 2H), 7.03 - 7.00 (m, 2H), 3.65 (s, 2H), 2.87 (d, J = 11.6 Hz, 2H), 2.44 (d, J = 6.9 Hz, 2H), 2.11 - 2.00 (m, 2H), 1.55 (d, J = 13.6 Hz, 2H), 1.51 - 1.44 (m, 1H), 1.30 - 1.24 (m, 2H). | 397. 18 (M+1) |
| 18 | 2-(5-((4-benzylpiperi din-1-yl)methyl)-4H-1,2,4-triazol-3-yl)-6-methoxy-1H-indole | 1H NMR (400 MHz, MeOD) δ 7.43 (d, J = 8.7 Hz, 1H), 7.28 - 7.19 (m, 2H), 7.14 (t, J = 6.7 Hz, 3H), 6.95 (d, J = 2.1 Hz, 1H), 6.93 (s, 1H), 6.71 (dd, J = 8.7, 2.3 Hz, 1H), 3.83 (s, 3H), 3.71 (s, 2H), 2.97 (d, J = 11.6 Hz, 2H), 2.54 (d, J = 7.0 Hz, 2H), 2.13 (dd, J = 11.7, 9.8 Hz, 2H), 1.65 (d, J = 13.0 Hz, 2H), 1.56 (ddd, J = 14.9, 7.3, 3.7 Hz, 1H), 1.41 - 1.29 (m, 2H). | 402. 17 (M+1) |
| 19 | 2-(5-((4-benzylpiperi din-1-yl)methyl)-4H-1,2,4-triazol-3-yl)-7-methoxy-1H-indole | 1H NMR (400 MHz, MeOD) δ 7.19 - 7.10 (m, 2H), 7.07 (d, J = 8.1 Hz, 1H), 7.04 (d, J = 7.8 Hz, 3H), 6.91 - 6.85 (m, 2H), 6.61 (d, J = 7.6 Hz, 1H), 3.88 (s, 3H), 3.62 (s, 2H), 2.88 (d, J = 11.7 Hz, 2H), 2.44 (d, J = 7.0 Hz, 2H), 2.10 - 1.98 (m, 2H), 1.55 (d, J = 13.5 Hz, 2H), 1.47 (tdd, J = 11.0, 7.2, 3.6 Hz, 1H), 1.27 (d, J = 3.4 Hz, 2H). | 402. 18 (M+1) |
| 20 | methyl 2-(5-((4-benzylpiperi din-1-yl)methyl)-4H-1,2,4-triazol-3-yl)-1H-indol-5-carboxylate | 1H NMR (400 MHz, CDCl₃) δ 9.13 (s, 1H), 8.14 (s, 1H), 7.82 (dd, J = 8.4, 1.4 Hz, 1H), 7.67 (d, J = 8.4 Hz, 1H), 7.32 - 7.28 (m, 2H), 7.20 (ddd, J = 8.6, 4.5, 1.2 Hz, 1H), 7.16 (dd, J = 9.1, 7.8 Hz, 3H), 3.94 (s, 3H), 3.75 (s, 2H), 2.89 (d, J = 11.5 Hz, 2H), 2.57 (d, J = 7.0 Hz, 2H), 2.15 (td, J = 11.7, 2.2 Hz, 2H), 1.69 (d, J = 13.5 Hz, 2H), 1.63 - 1.53 (m, 1H), 1.35 (ddd, J = 24.5, 12.1, 3.6 Hz, 2H). | |
| 21 | 4-benzyl-1-((5-(naphthalen-2-yl)-4H-1,2,4-triazol-3-yl)methyl)pi peridine | 1H NMR (400 MHz, MeOD) δ 8.52 (d, J = 0.9 Hz, 1H), 8.11 (dd, J = 8.6, 1.7 Hz, 1H), 8.02 - 7.95 (m, 2H), 7.95 - 7.88 (m, 1H), 7.59 - 7.53 (m, 2H), 7.29 - 7.23 (m, 2H), 7.16 (dd, J = 7.2, 5.2 Hz, 3H), 3.77 (s, 2H), 3.02 (d, J = 11.7 Hz, 2H), 2.57 (d, J = 7.0 Hz, 2H), 2.18 (td, J = 11.8, 2.3 Hz, 2H), 1.68 (d, J = 13.5 Hz, 2H), 1.63 - 1.53 (m, 1H), 1.38 (ddd, J = 15.3, 12.4, 3.8 Hz, 2H). | 383. 17 (M+1) |
| 22 | 3-(5-((4-benzylpiperi din-1-yl)methyl)-4H-1,2,4-triazol-3-yl)-1H-indole | 1H NMR (400 MHz, MeOD) δ 8.17 (d, J = 7.9 Hz, 1H), 7.87 (s, 1H), 7.47 (d, J = 7.8 Hz, 1H), 7.30 - 7.22 (m, 3H), 7.21 (dd, J = 3.8, 1.0 Hz, 1H), 7.16 (dd, J = 7.6, 5.7 Hz, 3H), 3.73 (s, 2H), 3.04 (d, J = 11.1 Hz, 2H), 2.56 (d, J = 7.0 Hz, 2H), 2.18 (t, J = 11.6 Hz, 2H), 1.71 - 1.63 (m, 2H), 1.63 - 1.52 (m, 1H), 1.38 (ddd, J = 15.3, 12.3, 3.8 Hz, 2H). | 372. 17 (M+1) |
| 23 | 2-(5-((4-benzylpiperi din-1-yl)methyl)-4H-1,2,4-triazol-3-yl)-5-(trifluorome thyl)-1H-indole | 1H NMR (400 MHz, MeOD) δ 7.79 - 7.73 (m, 2H), 7.31 (dd, J = 8.4, 1.3 Hz, 1H), 7.29 - 7.22 (m, 2H), 7.16 (dd, J = 7.2, 5.5 Hz, 3H), 7.11 (d, J = 0.7 Hz, 1H), 3.77 (s, 2H), 3.00 (d, J = 11.7 Hz, 2H), 2.57 (d, J = 7.0 Hz, 2H), 2.17 (td, J = 11.8, 2.3 Hz, 2H), 1.66 (t, J = 12.0 Hz, 2H), 1.59 (tdd, J = 10.8, 7.4, 3.8 Hz, 1H), 1.41 - 1.33 (m, 2H). | 440. 22 (M+1) |
| 24 | 2-(5-((4-benzylpiperi din-1-yl)methyl)-4H-1,2,4-triazol-3-yl)-4-chloro-1H-indole | 1H NMR (400 MHz, MeOD) δ 7.39 (d, J = 8.0 Hz, 1H), 7.25 (t, J = 7.3 Hz, 2H), 7.19 - 7.09 (m, 5H), 7.07 (dd, J = 7.6, 0.8 Hz, 1H), 3.75 (s, 2H), 2.98 (d, J = 11.6 Hz, 2H), 2.54 (d, J = 7.0 Hz, 2H), 2.16 (td, J = 11.8, 2.1 Hz, 2H), 1.65 (d, J = 13.3 Hz, 2H), 1.60 - 1.52 (m, 1H), 1.36 (ddd, J = 15.4, 12.6, 3.8 Hz, 2H). | 406. 17 (M+1) |
| 25 | 1-((5-(benzofuran-2-yl)-4H-1,2,4-triazol-3-yl)methyl)-4-benzylpiperi dine | 1H NMR (400 MHz, MeOD) δ 7.58 (d, J = 7.6 Hz, 1H), 7.47 (d, J = 7.7 Hz, 1H), 7.26 (dd, J = 13.0, 7.8 Hz, 2H), 7.21 - 7.11 (m, 3H), 7.04 (t, J = 6.8 Hz, 3H), 4.47 (s, 1H), 3.67 (s, 2H), 2.88 (d, J = 11.3 Hz, 2H), 2.44 (d, J = 6.9 Hz, 2H), 2.07 (t, J = 10.8 Hz, 2H), 1.55 (d, J = 13.7 Hz, 2H), 1.51 - 1.40 (m, 1H), 1.31 - 1.21 (m, 2H). | 373. 17 (M+1) |
| 26 | 1-((5-(benzo[b]thi ophen-2-yl)-4H-1,2,4-triazol-3-yl)methyl)-4-benzylpiperi dine | 1H NMR (400 MHz, MeOD) δ 7.91 (s, 1H), 7.90 - 7.84 (m, 2H), 7.42 - 7.36 (m, 2H), 7.25 (dd, J = 10.1, 4.5 Hz, 2H), 7.18 - 7.13 (m, 3H), 3.75 (s, 2H), 2.97 (d, J = 11.7 Hz, 2H), 2.55 (d, J = 7.0 Hz, 2H), 2.16 (td, J = 11.8, 2.3 Hz, 2H), 1.66 (d, J = 13.4 Hz, 5H), 1.57 - 1.53 (m, 1H), 1.36 (ddd, J = 15.3, 12.5, 3.8 Hz, 2H). | 389. 16 (M+1) |
| 27 | 1-((5-(benzo[d][1, 3]dioxol-5-yl)-4H-1,2,4-triazol-3-yl)methyl)-4-benzylpiperi dine | 1H NMR (400 MHz, CDCl₃) δ 7.60 (dd, J = 8.1, 1.6 Hz, 1H), 7.54 (d, J = 1.6 Hz, 1H), 7.29 (d, J = 7.1 Hz, 2H), 7.19 (dd, J = 8.4, 6.2 Hz, 1H), 7.15 - 7.11 (m, 2H), 6.86 (d, J = 8.1 Hz, 1H), 6.00 (s, 2H), 3.71 (s, 2H), 2.89 (d, J = 11.6 Hz, 2H), 2.55 (d, J = 7.0 Hz, 2H), 2.12 (td, J = 11.7, 2.2 Hz, 2H), 1.66 (d, J = 13.6 Hz, 2H), 1.57 (tdd, J = 11.1, 7.4, 3.7 Hz, 1H), 1.38 - 1.27 (m, 2H). | 377. 17 (M+1) |
| 28 | 2-(5-((4-benzylpiperi din-1-yl)methyl)-4H-1,2,4-triazol-3-yl)-1H-indol-5-carboxylic acid | 1H NMR (400 MHz, MeOD) δ 8.15 (s, 1H), 7.75 (dd, J = 8.4, 1.4 Hz, 1H), 7.60 (d, J = 8.4 Hz, 1H), 7.26 (dd, J = 9.5, 5.5 Hz, 2H), 7.17 (dd, J = 7.2, 5.4 Hz, 3H), 7.06 (d, J = 0.7 Hz, 1H), 3.82 (s, 2H), 3.05 (d, J = 11.4 Hz, 2H), 2.57 (d, J = 6.9 Hz, 2H), 2.24 (t, J = 12.0 Hz, 2H), 1.70 (d, J = 13.6 Hz, 2H), 1.65 - 1.57 (m, 1H), 1.39 (ddd, J = 14.7, 12.3, 3.1 Hz, 2H). | 416. 20 (M+1) |
| 29 | 2-(5-((4-(3,4-dichlorobenz | 1H NMR (400 MHz, CDCl₃) δ 9.07 (s, 1H), 7.34 (d, J = 8.2 Hz, 1H), 7.33 - 7.27 (m, 2H), 7.23 (d, J = 2.0 | 460. 09 (M+1 |
| | yl)piperidin -1-yl)methyl)-4H-1,2,4-triazol-3-yl)-5-fluoro-1H-indole | Hz, 1H), 7.06 (s, 1H), 7.00 - 6.94 (m, 2H), 3.75 (s, 2H), 2.91 (d, J = 11.6 Hz, 2H), 2.51 (d, J = 7.0 Hz, 2H), 2.20 - 2.08 (m, 2H), 1.65 (d, J = 11.6 Hz, 2H), 1.59 - 1.51 (m, 1H), 1.38 - 1.27 (m, 2H). | |
| 30 | 2-(5-((4-(3,4-dichlorobenz yl)piperidin -1-yl)methyl)-4H-1,2,4-triazol-3-yl)-5-methyl-1H-indole | 1H NMR (400 MHz, CDCl₃) δ 8.91 (s, 1H), 7.44 (s, 1H), 7.34 (d, J = 8.2 Hz, 1H), 7.29 (d, J = 8.3 Hz, 1H), 7.23 (d, J = 1.9 Hz, 1H), 7.06 (dd, J = 8.3, 1.2 Hz, 1H), 7.01 (s, 1H), 6.96 (dd, J = 8.2, 2.0 Hz, 1H), 3.74 (s, 2H), 2.91 (d, J = 11.6 Hz, 2H), 2.51 (d, J = 7.0 Hz, 2H), 2.44 (s, 3H), 2.18 - 2.08 (m, 2H), 1.65 (d, J = 12.6 Hz, 2H), 1.57 - 1.51 (m, 1H), 1.37 - 1.25 (m, 2H). | 454. 17 (M) |
| 31 | 2-(5-((4-(3,4-dichlorobenz yl)piperidin -1-yl)methyl)-4H-1,2,4-triazol-3-yl)-5,6-dimethoxy-1H-indole | 1H NMR (400 MHz, CDCl₃) δ 8.86 (s, 1H), 7.34 (d, J = 8.2 Hz, 1H), 7.23 (d, J = 1.8 Hz, 1H), 7.07 (s, 1H), 7.00 - 6.94 (m, 2H), 6.87 (s, 1H), 3.93 (s, 3H), 3.92 (s, 3H), 3.74 (s, 2H), 2.91 (d, J = 11.4 Hz, 2H), 2.51 (d, J = 7.0 Hz, 2H), 2.14 (dd, J = 11.6, 10.2 Hz, 2H), 1.65 (d, J = 12.4 Hz, 2H), 1.60 - 1.50 (m, 1H), 1.38 - 1.27 (m, 2H). | 500. 16 (M) |
| 32 | 2-(5-((4-(1H-indol-3-yl) piperidin -1-yl)methyl)-4H-1,2,4-triazol-3-yl)-5-fluoro-1H-indole | 1H NMR (400 MHz, CDCl₃) δ 8.97 (s, 1H), 7.96 (s, 1H), 7.65 (d, J = 7.9 Hz, 1H), 7.38 (d, J = 8.1 Hz, 1H), 7.35 - 7.27 (m, 2H), 7.23 - 7.17 (m, 1H), 7.15 - 7.09 (m, 1H), 7.07 (d, J = 0.7 Hz, 1H), 6.98 (ddd, J = 11.5, 6.7, 2.5 Hz, 2H), 3.85 (s, 2H), 3.06 (d, J = 11.7 Hz, 2H), 2.96 - 2.86 (m, 1H), 2.42 (td, J = 11.8, 2.2 Hz, 2H), 2.12 (d, J = 12.9 Hz, 2H), 1.88 (ddd, J = 16.0, 12.8, 3.8 Hz, 2H). | 415. 20 (M+1) |
| 33 | 2-((4-benzylpiperi din-1-yl)methyl)-5-(1H-indol-2-yl)-1,3,4-oxadiazole | 1H NMR (400 MHz, CDCl₃) δ 9.19 (s, 1H), 7.70 (d, J = 8.3 Hz, 1H), 7.48 (d, J = 8.7 Hz, 1H), 7.33 (t, J = 7.7 Hz, 1H), 7.29 (s, 1H), 7.25 (s, 1H), 7.22 (d, J = 1.2 Hz, 1H), 7.18 (t, J = 7.4 Hz, 2H), 7.13 (d, J = 7.0 Hz, 2H), 3.88 (s, 2H), 2.99 (d, J = 11.4 Hz, 2H), 2.54 (d, J = 7.0 Hz, 2H), 2.18 (t, J = 10.6 Hz, 2H), 1.68 (d, J = 13.5 Hz, 2H), 1.54 - 1.50 (m, 1H), 1.37 (ddd, J = 15.6, 12.5, 3.9 Hz, 2H). | 373. 04 (M+1) |
| 34 | 2-((4-(3,4-dichlorobenz yl)piperidin -1-yl)methyl)-5-(1H-indol-2-yl)-1,3,4-oxadiazole | 1H NMR (400 MHz, CDCl₃) δ 9.90 (s, 1H), 7.69 (d, J = 8.0 Hz, 1H), 7.60 (d, J = 8.3 Hz, 1H), 7.35 - 7.31 (m, 2H), 7.22 (s, 2H), 7.18 (t, J = 7.5 Hz, 1H), 6.96 (dd, J = 8.2, 1.8 Hz, 1H), 3.91 (s, 2H), 3.01 (d, J = 11.5 Hz, 2H), 2.50 (d, J = 7.0 Hz, 2H), 2.21 (t, J = 10.7 Hz, 2H), 1.66 (d, J = 13.1 Hz, 2H), 1.56 - 1.47 (m, 1H), 1.36 (ddd, J = 15.3, 12.3, 3.6 Hz, 2H). | 441. 03 (M) |
| 35 | 2-(5-((4-benzylpiperi din-1-yl)methyl)-4H-1,2,4-triazol-3-yl)-1H-indol-5,6-diol | 1H NMR (400 MHz, MeOD) δ 7.18 - 7.11 (m, 2H), 7.07 - 7.01 (m, 3H), 6.83 (s, 1H), 6.75 (s, 1H), 6.71 (s, 1H), 3.59 (s, 2H), 2.88 (d, J = 11.2 Hz, 2H), 2.44 (d, J = 7.0 Hz, 2H), 2.10 - 1.89 (m, 2H), 1.61 - 1.48 (m, 2H), 1.46 - 1.43 (m, 1H), 1.30 - 1.22 (m, 2H). | 404. 15 (M+1) |
| 36 | 3-(5-((4-benzylpiperi din-1-yl)methyl)-4H-1,2,4-triazol-3-yl)-5-methoxy-1H-indole | 1H NMR (400 MHz, MeOD) δ 7.83 (s, 1H), 7.70 (d, J = 2.0 Hz, 1H), 7.35 (d, J = 8.8 Hz, 1H), 7.29 - 7.23 (m, 2H), 7.17 (t, J = 6.8 Hz, 3H), 6.88 (dd, J = 8.8, 2.4 Hz, 1H), 3.88 (s, 3H), 3.75 (s, 2H), 3.06 (d, J = 10.4 Hz, 2H), 2.56 (d, J = 7.0 Hz, 2H), 2.20 (t, J = 11.1 Hz, 2H), 1.68 (d, J = 12.9 Hz, 2H), 1.63 - 1.54 (m, 1H), 1.43 - 1.34 (m, 2H). | 402. 05 (M+1 ) |
| 37 | 3-(5-((4-benzylpiperi din-1-yl)methyl)-4H-1,2,4-triazol-3-yl)-7-methoxy-1H-indole | 1H NMR (400 MHz, MeOD) δ 7.81 (s, 1H), 7.74 (d, J = 8.1 Hz, 1H), 7.26 (t, J = 7.6 Hz, 2H), 7.19 - 7.11 (m, 3H), 7.10 (d, J = 7.9 Hz, 1H), 6.76 (d, J = 7.7 Hz, 1H), 4.00 (s, 3H), 3.72 (s, 2H), 3.03 (d, J = 11.5 Hz, 2H), 2.55 (d, J = 7.0 Hz, 2H), 2.16 (t, J = 10.9 Hz, 2H), 1.66 (d, J = 13.2 Hz, 2H), 1.56 (dtd, J = 14.4, 7.3, 3.6 Hz, 1H), 1.42 - 1.33 (m, 2H). | 402. 07 (M+1 ) |
| 38 | 3-(5-((4-benzylpiperi din-1-yl)methyl)-4H-1,2,4-triazol-3-yl)-6-methyl-1H-indole | 1H NMR (400 MHz, MeOD) δ 7.90 (d, J = 8.2 Hz, 1H), 7.67 (s, 1H), 7.14 (t, J = 7.6 Hz, 3H), 7.04 (t, J = 6.7 Hz, 3H), 6.91 (d, J = 8.2 Hz, 1H), 3.61 (s, 2H), 2.93 (d, J = 11.3 Hz, 2H), 2.44 (d, J = 7.0 Hz, 2H), 2.35 (s, 3H), 2.07 (t, J = 10.9 Hz, 2H), 1.55 (d, J = 13.0 Hz, 2H), 1.50 - 1.38 (m, 1H), 1.25 (dt, J = 11.6, 8.5 Hz, 2H). | 386. 08 (M+1) |
| 39 | 3-(5-((4-benzylpiperi din-1-yl)methyl)-4H-1,2,4-triazol-3-yl)-5-methyl-1H-indole | 1H NMR (400 MHz, MeOD) δ 7.86 (s, 1H), 7.70 (s, 1H), 7.23 (d, J = 8.3 Hz, 1H), 7.14 (t, J = 7.6 Hz, 2H), 7.04 (t, J = 7.0 Hz, 3H), 6.95 (d, J = 7.6 Hz, 1H), 3.61 (s, 2H), 2.93 (d, J = 11.4 Hz, 2H), 2.43 (d, J = 6.9 Hz, 2H), 2.37 (s, 3H), 2.06 (t, J = 11.0 Hz, 2H), 1.55 (d, J = 13.3 Hz, 2H), 1.49 - 1.41 (m, 1H), 1.31 - 1.20 (m, 1H). | 386. 10 (M+1) |
| 40 | 3-(5-((4-benzylpiperi din-1-yl)methyl)-4H-1,2,4-triazol-3-yl)-7-chloro-1H-indole | 1H NMR (400 MHz, MeOD) δ 8.04 (d, J = 7.9 Hz, 1H), 7.82 (s, 1H), 7.14 (dd, J = 7.9, 6.4 Hz, 3H), 7.04 (dd, J = 7.6, 6.2 Hz, 4H), 3.65 (s, 2H), 2.93 (d, J = 11.3 Hz, 2H), 2.44 (d, J = 7.0 Hz, 2H), 2.08 (d, J = 14.2, 7.5 Hz, 3H), 1.55 (d, J = 13.5 Hz, 2H), 1.49 - 1.43 (m, J = 14.7, 7.2, 3.8 Hz, 2H), 1.29 (dd, J = 12.5, 3.3 Hz, 2H). | 406. 04 (M+1) |
| 41 | 3-(5-((4-benzylpiperi din-1-yl)methyl)-4H-1,2,4-triazol-3-yl)-5-chloro-1H-indole | 1H NMR (400 MHz, MeOD) δ 8.23 (d, J = 2.0 Hz, 1H), 7.91 (s, 1H), 7.43 (d, J = 8.7 Hz, 1H), 7.29 - 7.23 (m, 2H), 7.19 (dd, J = 8.8, 2.1 Hz, 1H), 7.15 (d, J = 7.7 Hz, 3H), 3.74 (s, 2H), 3.02 (d, J = 11.4 Hz, 2H), 2.56 (d, J = 7.0 Hz, 2H), 2.17 (t, J = 10.9 Hz, 2H), 1.67 (d, J = 13.3 Hz, 2H), 1.60 - 1.54 (m, J = 14.7, 7.2, 3.6 Hz, 1H), 1.38 (ddd, J = 15.4, 12.6, 3.8 Hz, 2H). | 406. 04 (M+1) |
| 42 | methyl 3-(5-((4-benzylpiperi din-1-yl)methyl)-4H-1,2,4-triazol-3-yl)-1H-indol-7-carboxylate | 1H NMR (400 MHz, MeOD) δ 8.41 (d, J = 12.1 Hz, 1H), 7.87 (s, 1H), 7.84 (d, J = 7.5 Hz, 1H), 7.20 - 7.09 (m, 1H), 7.04 (t, J = 7.1 Hz, 1H), 3.91 (s, 1H), 3.64 (s, 1H), 2.92 (d, J = 11.4 Hz, 2H), 2.44 (d, J = 7.0 Hz, 2H), 2.07 (t, J = 11.0 Hz, 2H), 1.55 (d, J = 13.1 Hz, 2H), 1.49 - 1.40 (m, J = 15.0, 11.2, 7.6, 4.0 Hz, 1H), 1.26 (ddd, J = 15.3, 12.5, 3.5 Hz, 2H). | 430. 09 (M+1) |
| 43 | 3-(5-((4-benzylpiperi din-1-yl)methyl)-4H-1,2,4-triazol-3-yl)-1H-indol-5-amine | 1H NMR (400 MHz, MeOD) δ 7.28 - 7.20 (m, 3H), 7.14 (t, J = 6.9 Hz, 3H), 6.98 (d, J = 2.0 Hz, 1H), 6.83 (s, 1H), 6.77 (dd, J = 8.6, 2.1 Hz, 1H), 3.72 (s, 2H), 2.98 (d, J = 11.6 Hz, 2H), 2.54 (d, J = 7.0 Hz, 2H), 2.19 - 2.09 (m, 2H), 1.65 (d, J = 13.5 Hz, 2H), 1.55 (dddd, J = 14.4, 10.7, 7.0, 3.5 Hz, 1H), 1.41 - 1.32 (m, 2H). | 387. 08 (M+1) |
| 44 | 3-(5-((4-(3,4-dichlorobenz yl)piperidin -1-yl)methyl)-4H-1,2,4-triazol-3-yl)-5-methoxy-1H-indole | 1H NMR (400 MHz, CDCl₃) δ 8.28 (s, 1H), 7.84 (d, J = 2.4 Hz, 1H), 7.79 (d, J = 2.0 Hz, 1H), 7.33 (dd, J = 10.2, 8.6 Hz, 2H), 7.24 (d, J = 1.9 Hz, 1H), 6.97 (dd, J = 8.2, 1.9 Hz, 1H), 6.93 (dd, J = 8.8, 2.5 Hz, 1H), 3.93 (s, 3H), 3.76 (s, 2H), 2.94 (d, J = 11.5 Hz, 2H), 2.51 (d, J = 7.0 Hz, 2H), 2.13 (t, J = 10.8 Hz, 2H), 1.65 (d, J = 11.9 Hz, 2H), 1.53 (ddd, J = 15.4, 7.6, 3.8 Hz, 1H), 1.32 (ddd, J = 15.5, 12.6, 3.9 Hz, 2H). | 471. 09 (M+1) |
| 45 | 3-(5-((4-(3,4-dichlorobenz yl)piperidin -1-yl)methyl)-4H-1,2,4-triazol-3-yl)-7-methoxy-1H-indole | 1H NMR (400 MHz, MeOD) δ 7.69 (s, 1H), 7.62 (d, J = 7.1 Hz, 1H), 7.30 (d, J = 8.2 Hz, 1H), 7.24 (d, J = 1.9 Hz, 1H), 7.00 (dd, J = 8.1, 2.0 Hz, 2H), 6.65 (d, J = 7.7 Hz, 1H), 3.88 (s, 3H), 3.63 (s, 2H), 2.94 (s, 2H), 2.44 (d, J = 6.9 Hz, 2H), 2.09 (t, J = 7.5 Hz, 2H), 1.58 - 1.50 (m, 2H), 1.50 - 1.43 (m, 1H), 1.31 - 1.20 (m, 2H). | 470. 02 (M) |
| 46 | 4-(5-((4-benzylpiperi din-1-yl)methyl)-4H-1,2,4-triazol-3-yl)benzonitr ile | 1H NMR (400 MHz, MeOD) δ 8.19 (d, J = 8.4 Hz, 2H), 7.83 (d, J = 8.4 Hz, 2H), 7.24 (t, J = 7.6 Hz, 2H), 7.15 (t, J = 7.0 Hz, 3H), 3.75 (s, 2H), 2.96 (d, J = 11.5 Hz, 2H), 2.54 (d, J = 7.0 Hz, 2H), 2.22 - 2.10 (m, 2H), 1.65 (d, J = 14.4 Hz, 2H), 1.60 - 1.51 (m, 1H), 1.37 (dd, J = 17.9, 8.8 Hz, 2H). | 358. 1 (M+1) |
| 47 | 4-benzyl-1-((5-(4-fluorophenyl )-4H-1,2,4-triazol-3-yl)methyl)pi peridine | 1H NMR (400 MHz, MeOD) δ 7.95 - 7.88 (m, 2H), 7.18 - 7.08 (m, 4H), 7.05 (t, J = 7.1 Hz, 3H), 3.62 (s, 2H), 2.87 (d, J = 11.6 Hz, 2H), 2.44 (d, J = 7.0 Hz, 2H), 2.03 (t, J = 10.8 Hz, 2H), 1.54 (d, J = 13.4 Hz, 2H), 1.50 - 1.40 (m, 1H), 1.31 - 1.22 (m, 2H). | 351. |
| 48 | 4-benzyl-1-((5-(3,4-dimethoxyphe nyl)-4H-1,2,4-triazol-3-yl)methyl)pi peridine | 1H NMR (400 MHz, MeOD) δ 7.59 (d, J = 1.9 Hz, 1H), 7.57 (dd, J = 8.3, 1.9 Hz, 1H), 7.27 - 7.21 (m, 2H), 7.14 (t, J = 6.8 Hz, 3H), 7.06 (d, J = 8.4 Hz, 1H), 3.91 (s, 3H), 3.89 (s, 3H), 3.68 (s, 2H), 2.97 (d, J = 11.7 Hz, 2H), 2.54 (d, J = 7.0 Hz, 2H), 2.12 (t, J = 10.7 Hz, 2H), 1.64 (d, J = 13.2 Hz, 2H), 1.60 - 1.49 (m, 1H), 1.41 - 1.31 (m, 2H). | 393. 2 (M+1) |
| 49 | 4-benzyl-1-((5-(4-(trifluorome thoxy) phenyl )-4H-1,2,4-triazol-3-yl)methyl)pi peridine | 1H NMR (400 MHz, MeOD) δ 8.00 (d, J = 8.8 Hz, 2H), 7.28 (d, J = 8.5 Hz, 2H), 7.14 (t, J = 7.5 Hz, 2H), 7.04 (t, J = 7.5 Hz, 3H), 3.62 (s, 2H), 2.86 (d, J = 11.6 Hz, 2H), 2.43 (d, J = 7.0 Hz, 2H), 2.03 (t, J = 10.8 Hz, 2H), 1.54 (d, J = 13.4 Hz, 2H), 1.45 (tdd, J = 11.0, 7.4, 3.7 Hz, 1H), 1.30 - 1.21 (m, 2H). | 417. 13 (M+1) |
| 50 | 4-benzyl-1-((5-(4-(trifluorome thyl)phenyl) -4H-1,2,4-triazol-3-yl)methyl)pi peridine | 1H NMR (400 MHz, MeOD) δ 8.09 (d, J = 8.2 Hz, 2H), 7.67 (d, J = 8.4 Hz, 2H), 7.18 - 7.09 (m, 2H), 7.05 (t, J = 6.8 Hz, 3H), 3.64 (s, 2H), 2.86 (d, J = 11.6 Hz, 2H), 2.44 (d, J = 7.0 Hz, 2H), 2.04 (dd, J = 11.7, 9.8 Hz, 2H), 1.55 (d, J = 13.8 Hz, 2H), 1.51 - 1.37 (m, 1H), 1.32 - 1.22 (m, 2H). | 401. 14 (M+1) |
| 51 | N-(4-(5-((4-benzylpiperi din-1-yl)methyl)-4H-1,2,4-triazol-3-yl)phenyl)ac etamide | 1H NMR (400 MHz, MeOD) δ 7.82 (d, J = 8.7 Hz, 2H), 7.58 (d, J = 8.7 Hz, 2H), 7.17 - 7.10 (m, 2H), 7.04 (t, J = 7.0 Hz, 3H), 3.59 (s, 2H), 2.86 (d, J = 11.6 Hz, 2H), 2.43 (d, J = 7.0 Hz, 2H), 2.05 (s, 3H), 2.00 (dd, J = 12.9, 2.9 Hz, 2H), 1.54 (d, J = 13.3 Hz, 2H), 1.49 - 1.42 (m, 1H), 1.29 - 1.22 (m, 2H). | 390. 15 (M+1) |
| 52 | 6-(5-((4-benzylpiperi din-1-yl)methyl)-4H-1,2,4-triazol-3-yl)naphthale n-2-ol | 1H NMR (400 MHz, MeOD) δ 8.28 (s, 1H), 7.87 (d, J = 8.7 Hz, 1H), 7.72 (d, J = 8.6 Hz, 1H), 7.64 (d, J = 8.7 Hz, 1H), 7.18 - 7.11 (m, 2H), 7.05 (t, J = 6.3 Hz, 5H), 3.66 (s, 2H), 2.92 (d, J = 11.2 Hz, 2H), 2.45 (d, J = 7.0 Hz, 2H), 2.13 - 2.06 (m, 2H), 1.57 (d, J = 14.3 Hz, 2H), 1.51 - 1.42 (m, 1H), 1.34 - 1.28 (m, 2H). | 399. 23 (M+1) |
| 53 | 4-benzyl-1-((5-(4-phenoxypheny l)-4H-1,2,4-triazol-3-yl)methyl)pi peridine | 1H NMR (400 MHz, MeOD) δ 7.89 - 7.82 (m, 2H), 7.29 (dd, J = 8.5, 7.5 Hz, 2H), 7.13 (d, J = 7.0 Hz, 2H), 7.09 - 7.05 (m, 1H), 7.03 (d, J = 8.1 Hz, 3H), 6.97 - 6.92 (m, 4H), 3.60 (s, 2H), 2.86 (d, J = 11.6 Hz, 2H), 2.43 (d, J = 7.0 Hz, 2H), 2.09 - 1.93 (m, 2H), 1.53 (d, J = 13.4 Hz, 2H), 1.49 - 1.39 (m, 1H), 1.28 - 1.19 (m, 2H). | 425. 27 (M+1) |
| 54 | 4-benzyl-1-((5-(3-(trifluorome thoxy) phenyl )-4H-1,2,4-triazol-3-yl)methyl)pi peridine | 1H NMR (400 MHz, MeOD) δ 8.06 - 8.00 (m, 1H), 7.94 (s, 1H), 7.58 (t, J = 8.0 Hz, 1H), 7.36 (d, J = 7.1 Hz, 1H), 7.25 (t, J = 7.6 Hz, 2H), 7.16 (t, J = 7.3 Hz, 3H), 3.77 (s, 2H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 417. 19 (M+1) |
| 55 | 4-benzyl-1-((5-(3-(trifluorome thyl)phenyl) -4H-1,2,4-triazol-3-yl)methyl)pi peridine | 1H NMR (400 MHz, MeOD) δ 8.34 (s, 1H), 8.28 (d, J = 7.7 Hz, 1H), 7.75 (d, J = 7.8 Hz, 1H), 7.68 (t, J = 7.7 Hz, 1H), 7.25 (t, J = 7.4 Hz, 2H), 7.16 (t, J = 7.8 Hz, 3H), 3.79 (s, 2H), 3.00 (d, J = 11.6 Hz, 2H), 2.55 (d, J = 7.0 Hz, 2H), 2.19 (t, J = 10.8 Hz, 2H), 1.67 (d, J = 13.6 Hz, 2H), 1.58 (dddd, J = 14.5, 10.7, 7.3, 3.6 Hz, 1H), 1.37 (ddd, J = 15.1, 12.5, 3.7 Hz, 2H). | 401. 18 (M+1) |
| 56 | 3-(5-((4-benzylpiperi din-1-yl)methyl)-4H-1,2,4-triazol-3-yl)-6-methoxy-1H-indole | 1H NMR (400 MHz, MeOD) δ 7.90 (d, J = 8.8 Hz, 1H), 7.63 (s, 1H), 7.19 - 7.10 (m, 2H), 7.05 (t, J = 6.6 Hz, 3H), 6.87 (d, J = 2.1 Hz, 1H), 6.73 (dd, J = 8.8, 2.1 Hz, 1H), 3.74 (s, 3H), 3.61 (s, 2H), 2.92 (d, J = 11.3 Hz, 2H), 2.44 (d, J = 7.0 Hz, 2H), 2.06 (t, J = 11.8 Hz, 2H), 1.53 (t, J = 13.9 Hz, 2H), 1.49 - 1.41 (m, 1H), 1.32 - 1.22 (m, 2H). | 402. 13 (M+1) |
| 57 | 2-(5-((4-benzylpiperi din-1-yl)methyl)-4H-1,2,4-triazol-3-yl)-1H-indol-5-carboxamide | 1H NMR (400 MHz, MeOD) δ 8.03 (s, 1H), 7.65 (d, J = 8.4 Hz, 1H), 7.57 (dd, J = 8.3, 1.3 Hz, 1H), 7.28 - 7.21 (m, 2H), 7.15 (t, J = 6.3 Hz, 2H), 7.06 (s, 1H), 3.75 (s, 2H), 2.98 (d, J = 11.6 Hz, 2H), 2.55 (d, J = 7.0 Hz, 2H), 2.15 (t, J = 10.7 Hz, 2H), 1.66 (d, J = 13.2 Hz, 2H), 1.61 - 1.51 (m, 1H), 1.43 - 1.31 (m, 2H). | 415. 22 (M+1) |
| 58 | 4-(5-((4-benzylpiperi din-1-yl)methyl)-4H-1,2,4-triazol-3-yl)-N-(2-(dimethylami no)ethyl)ben zoamide | 1H NMR (400 MHz, MeOD) δ 8.12 (d, J = 8.5 Hz, 2H), 7.95 (d, J = 8.4 Hz, 2H), 7.30 - 7.23 (m, 2H), 7.17 (t, J = 6.7 Hz, 3H), 3.75 (s, 2H), 3.57 (t, J = 6.8 Hz, 2H), 2.98 (d, J = 12.0 Hz, 2H), 2.62 (t, J = 6.8 Hz, 2H), 2.56 (d, J = 7.0 Hz, 2H), 2.35 (s, 6H), 2.15 (t, J = 11.8 Hz, 2H), 1.65 (t, J = 12.5 Hz, 2H), 1.62 - 1.52 (m, 1H), 1.44 - 1.34 (m, 2H). | 447. 3 (M+1) |
| 59 | 3-(5-((4-(3,4-dichlorobenz yl)piperidin -1-yl)methyl)-4H-1,2,4-triazol-3-yl)-6-methoxy-1H-indole | 1H NMR (400 MHz, MeOD) δ 8.02 (d, J = 8.7 Hz, 1H), 7.75 (s, 1H), 7.41 (d, J = 8.2 Hz, 1H), 7.35 (d, J = 1.9 Hz, 1H), 7.11 (dd, J = 8.2, 2.0 Hz, 1H), 6.98 (d, J = 2.1 Hz, 1H), 6.85 (dd, J = 8.8, 2.1 Hz, 1H), 3.86 (s, 3H), 3.72 (s, 2H), 3.04 (d, J = 10.8 Hz, 2H), 2.56 (d, J = 6.9 Hz, 2H), 2.18 (t, J = 11.1 Hz, 2H), 1.65 (d, J = 14.1 Hz, 2H), 1.61 - 1.53 (m, 1H), 1.43 - 1.33 (m, 2H). | 470. 16 (M+1) |
| 60 | methyl 3-(5-((4-(3,4-dichlorobenz yl)piperidin -1-yl)methyl)-4H-1,2,4-triazol-3-yl)-1H-indol-7-carboxylate | 1H NMR (400 MHz, MeOD) δ 8.50 (d, J = 7.9 Hz, 1H), 7.97 (s, 1H), 7.97 - 7.88 (m, 1H), 7.40 (d, J = 8.2 Hz, 1H), 7.34 (d, J = 1.8 Hz, 1H), 7.27 (t, J = 7.7 Hz, 1H), 7.09 (dd, J = 8.2, 1.8 Hz, 1H), 4.01 (s, 3H), 3.73 - 3.68 (m, 2H), 3.03 (d, J = 11.2 Hz, 2H), 2.54 (d, J = 6.9 Hz, 2H), 2.18 (dd, J = 13.8, 9.5 Hz, 2H), 1.63 (t, J = 9.7 Hz, 2H), 1.60 - 1.50 (m, 1H), 1.40 - 1.31 (m, 2H). | 498. 09 (M+1) |
| 61 | 4-(3,4-dichlorobenz yl)-1-((5-(4-(trifluorome | 1H NMR (400 MHz, MeOD) δ 8.15 - 8.07 (m, 2H), 7.40 (dd, J = 8.2, 3.6 Hz, 3H), 7.34 (d, J = 1.9 Hz, 1H), 7.09 (dd, J = 8.2, 2.0 Hz, 1H), 3.75 (s, 2H), 2.98 (d, J = | 485. 17 (M+1) |
| | thoxy) phenyl )-4H-1,2,4-triazol-3-yl) methyl) pi peridine | 11.6 Hz, 2H), 2.54 (d, J = 6.9 Hz, 2H), 2.15 (dt, J = 11.8, 5.9 Hz, 2H), 1.62 (t, J = 11.5 Hz, 2H), 1.55 (dtd, J = 10.7, 7.1, 3.5 Hz, 1H), 1.41 - 1.31 (m, 2H). | |
| 62 | 4-(3,4-dichlorobenz yl)-1-((5-(4-(trifluorome thyl)phenyl) -4H-1,2,4-triazol-3-yl)methyl)pi peridine | 1H NMR (400 MHz, MeOD) δ 8.09 (d, J = 8.2 Hz, 2H), 7.66 (d, J = 8.3 Hz, 2H), 7.28 (d, J = 8.2 Hz, 1H), 7.22 (d, J = 1.9 Hz, 1H), 6.98 (dd, J = 8.2, 1.9 Hz, 1H), 3.67 (s, 2H), 2.89 (d, J = 11.6 Hz, 2H), 2.43 (d, J = 6.9 Hz, 2H), 2.08 (t, J = 10.9 Hz, 2H), 1.53 (d, J = 13.5 Hz, 2H), 1.46 (dtd, J = 10.8, 7.1, 3.6 Hz, 1H), 1.24 (ddd, J = 15.0, 12.6, 3.7 Hz, 2H). | 469. 14 (M+1) |
| 63 | methyl 2-amino-4-(5-((4-benzylpiperi din-1-yl)methyl)-4H-1,2,4-triazol-3-yl) benzoate | 1H NMR (400 MHz, MeOD) δ 7.89 (d, J = 8.4 Hz, 1H), 7.39 (d, J = 1.5 Hz, 1H), 7.26 (t, J = 7.5 Hz, 2H), 7.22 - 7.12 (m, 4H), 3.88 (s, 3H), 3.73 (s, 2H), 2.98 (d, J = 11.6 Hz, 2H), 2.56 (d, J = 7.0 Hz, 2H), 2.14 (td, J = 11.9, 2.1 Hz, 2H), 1.66 (d, J = 14.0 Hz, 2H), 1.62 - 1.51 (m, 1H), 1.43 - 1.33 (m, 2H). | 406. 21 (M+1) |
| 64 | 2-(5-((4-benzylpipera zin-1-yl)methyl)-4H-1,2,4-triazol-3-yl)-5-fluoro-1H-indole | 1H NMR (400 MHz, MeOD) δ 7.39 (dd, J = 8.9, 4.5 Hz, 1H), 7.34 - 7.30 (m, 4H), 7.30 - 7.22 (m, 2H), 6.98 (d, J = 0.7 Hz, 1H), 6.97 - 6.90 (m, 1H), 3.76 (s, 2H), 3.54 (s, 2H), 2.60 (d, J = 32.9 Hz, 8H). | 391. 22 (M+1) |
| 65 | 2-(5-((4-benzylpipera zin-1-yl)methyl)-4H-1,2,4-triazol-3-yl)-5-chloro-1H-indole | 1H NMR (400 MHz, MeOD) δ 7.58 (d, J = 1.9 Hz, 1H), 7.42 (d, J = 8.7 Hz, 1H), 7.37 - 7.32 (m, 3H), 7.28 (dddd, J = 9.8, 8.3, 4.2, 2.4 Hz, 2H), 7.14 (dd, J = 8.7, 2.0 Hz, 1H), 6.99 (s, 1H), 3.79 (s, 2H), 3.56 (s, 2H), 2.62 (d, J = 31.9 Hz, 8H). | 407. 19 (M+1) |
| 66 | N-(2-(5-((4-benzylpiperi din-1-yl)methyl)-4H-1,2,4-triazol-3-yl)-1H-indol-5-yl) acetamide | 1H NMR (400 MHz, MeOD) δ 7.81 (s, 1H), 7.38 (d, J = 8.7 Hz, 1H), 7.29 - 7.21 (m, 3H), 7.15 (dd, J = 7.2, 5.4 Hz, 3H), 6.98 (s, 1H), 3.74 (s, 2H), 2.99 (d, J = 11.4 Hz, 2H), 2.55 (d, J = 7.0 Hz, 2H), 2.23 - 2.15 (m, 2H), 2.14 (s, 3H), 1.65 (t, J = 11.0 Hz, 2H), 1.62 - 1.52 (m, 1H), 1.41 - 1.32 (m, 2H). | 429. 17 (M+1) |
| 67 | 2-(5-((4-benzylpiperi din-1-yl)methyl)-4H-1,2,4-triazol-3-yl)-1H-indol-5-ol | 1H NMR (400 MHz, MeOD) δ 7.58 (d, J = 1.9 Hz, 1H), 7.42 (d, J = 8.7 Hz, 1H), 7.37 - 7.32 (m, 3H), 7.28 - 7.20 (m, 2H), 7.14 (dd, J = 8.7, 2.0 Hz, 1H), 6.99 (s, 1H), 3.77 (s, 2H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 388. 62 (M+1) |
| 68 | (4-benzylpiperi din-1-yl) (5-(5-fluoro-1H-indol-2-yl)-4H-1,2,4-triazol-3-yl)methanone | 1H NMR (400 MHz, MeOD) δ 7.56 (d, J = 1.9 Hz, 1H), 7.42 (d, J = 8.7 Hz, 1H), 7.37 - 7.32 (m, 3H), 7.28 - 7.20 (m, 2H), 7.14 (dd, J = 8.7, 2.0 Hz, 1H), 6.99 (s, 1H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.40 - 1.31 (m, 2H). | 404. 32 (M+1) |
| 69 | 2-(5-((4-benzylpiperi din-1-yl)methyl)-4H-1,2,4-triazol-3-yl)-6-butoxy-1H-indole | 1H NMR (400 MHz, MeOD) δ 7.38 (d, J = 8.7 Hz, 1H), 7.29 - 7.21 (m, 2H), 7.17 - 7.02 (m, 3H), 6.87 (d, J = 8.7 Hz, 1H), 6.85 (s, 1H), 6.54 (s, 1H), 3.89 (t, J = 11.0 Hz, 2H), 3.67(s, 2H), 2.99 (d, J = 11.4 Hz, 2H), 2.55 (d, J = 7.0 Hz, 2H), 2.17 - 2.05 (m, 2H), 1.71 - 1.61 (m, 2H), 1.56 (d, J = 7.0 Hz, 1.47 - 1.38 (m, 3H), 1.30 - 1.21 (m, 2H), 0.99 (t, J = 6.8 Hz, 3H) | 444. 32 (M+1) |
| 70 | 2-(5-((4-benzylpiperi din-1-yl)methyl)-4H-1,2,4-triazol-3yl)-3-methyl-1H-indole | 1H NMR (400 MHz, MeOD) δ 7.55 (d, J = 1.9 Hz, 1H), 7.42 (d, J = 8.7 Hz, 1H), 7.37 - 7.32 (m, 3H), 7.28 - 7.20 (m, 2H), 7.14 (dd, J = 8.7, 2.0 Hz, 1H), 3.77 (s, 2H), 2.99 (d, J = 11.4 Hz, 2H), 2.37 (s, 1H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 386. 71 (M+1) |
| 71 | 2-(5-((4-benzylpiperi din-1-yl)methyl)-4H-1,2,4-triazol-3-yl)-4,6-dichloro-1H-indole | 1H NMR (400 MHz, MeOD) δ 7.46 (s, 1H), 7.37 - 7.32 (m, 3H), 7.28 - 7.20 (m, 2H), 7.19 (s, 1H), 6.77 (s, 1H), 3.77 (s, 2H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 441. 42 (M+1) |
| 72 | 2-(5-((4-benzylpiperi din-1-yl) methyl)-4H-1,2,4-triazol-3-yl)-5,6-dichloro-1H-indole | 1H NMR (400 MHz, MeOD) δ 8.00 (s, 1H), 7.52 (s, 1H), 7.35 - 7.31 (m, 3H), 7.28 - 7.20 (m, 2H), 6.73 (s, 1H), 3.77 (s, 2H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 441. 37 (M+1) |
| 73 | 6-(5-((4-benzylpiperi din-1-yl) methyl)-4H-1,2,4-triazol-3-yl)-5H-[1,3]dioxolo [4,5-f]indole | 1H NMR (400 MHz, MeOD) δ 7.50 (s, 1H), 7.35 - 7.31 (m, 3H), 7.28 - 7.20 (m, 2H), 7.22 (s, 1H), 6.05 (s, 1H), 6.73 (s, 1H), 3.77 (s, 2H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 416. 7 (M+1) |
| 74 | 7-(5-((4-benzylpiperi din-1-yl) methyl)-4H-1,2,4-triazol-3-yl)-2,3-dihydro-6H-[1,4]dioxino [2,3-f]indole | 1H NMR (400 MHz, MeOD) δ 7.48 (s, 1H), 7.35 - 7.31 (m, 3H), 7.28 - 7.25 (m, 2H), 7.22 (s, 1H), 6.76 (s, 1H), 4.28 (s, 4H), 3.77 (s, 2H), 2.99 (d, J = 11.4 Hz, 2H), 2.55 (d, J = 6.8 Hz, 2H), 2.17 (t, J = 11.5 Hz, 2H), 1.66 (d, J = 13.1 Hz, 2H), 1.63 - 1.51 (m, 1H), 1.43 - 1.31 (m, 2H). | 430. 42 (M+1) |
| 75 | 3-(5-((4-benzylpiperi din-1-yl) methyl)-4H-1,2,4-triazol-3-yl)-6-fluoro-1H-indole | 1H NMR (400 MHz, MeOD) δ 7.91 (d, J = 8.8 Hz, 1H), 7.65 (s, 1H), 7.19 - 7.10 (m, 2H), 7.05 (t, J = 6.6 Hz, 3H), 6.87 (d, J = 2.1 Hz, 1H), 6.73 (dd, J = 8.8, 2.1 Hz, 1H), 3.61 (s, 2H), 2.92 (d, J = 11.3 Hz, 2H), 2.44 (d, J = 7.0 Hz, 2H), 2.06 (t, J = 11.8 Hz, 2H), 1.53 (t, J = 13.9 Hz, 2H), 1.49 - 1.41 (m, 1H), 1.32 - 1.22 (m, 2H). | 390. 52 (M+1) |
| 76 | 6-(5-((4-benzylpiperi din-1-yl) | 1H NMR (400 MHz, MeOD) δ 8.31 (d, J = 8.8 Hz, 1H), 7.91 (d, J = 8.8 Hz, 1H), 7.74 (s, 1H), 7.19 - 7.10 (m, | 372. 68 (M+1 |
| | methyl)-4H-1,2,4-triazol-3-yl)-1H-indole | 2H), 7.05 (t, J = 6.6 Hz, 3H), 7.07 (s, 1H), 6.56 (d, J = 2.1 Hz, 1H), 4.43 (s, 2H), 2.92 (d, J = 11.3 Hz, 2H), 2.44 (d, J = 7.0 Hz, 2H), 2.06 (t, J = 11.8 Hz, 2H), 1.53 (t, J = 13.9 Hz, 2H), 1.49 - 1.41 (m, 1H), 1.29 - 1.20 (m, 2H). | ) |

### <Experimental Example 1> Evaluation of Triple Reuptake Inhibitory Effect

In order to evaluate the reuptake inhibitory effect of the compounds of Examples according to the present invention on serotonin, norepinephrine and dopamine, the following experiments were performed, and the results are shown in Table 13.

To evaluate the reuptake inhibitory effect on dopamine, the dopamine transporter cDNA was transfected using the HEK-293 cell line, and then sub-seeded in a 24 well plate the next day. After removing the medium, each compound of Examples or Comparative Examples diluted to 1 µM in uptake buffer was added to a concentration of 200 µL/well and cultured at 37°C for 15 minutes. 100 µL of uptake buffer containing 20-30 nM [3H]-DA was added and cultured at 37°C for 5 minutes. The reaction solution was quickly removed and washed three times with 1 mL ice-cold uptake buffer. After dissolving the resultant in 1% SDS (0.5 mL), the degree of binding of radioisotope-labeled dopamine to the transporter was measured.

For norepinephrine(NE) and serotonin(5-HT), the degree of binding of radioisotope-labeled norepinephrine or serotonin to the transporter was measured in the same manner as above, except that cells transfected with NE or 5-HT transporter cDNA were used and [3H]-NE or [3H]-5-HT was used as a substrate.

In addition, when evaluating the reuptake inhibitory effect on dopamine, norepinephrine, and serotonin, the same method was performed without treating each compound of Examples or Comparative Examples as a control, the degree of binding of radioisotope-labeled dopamine, norepinephrine, and serotonin to the transporter was measured, and by using this as a reference (100%), the triple reuptake inhibitory effect was evaluated as a relative ratio (Ratio%). That is, in the control group, the binding of dopamine, norepinephrine, and serotonin to transporters is not inhibited, but when each compound of Examples is treated and thus the binding is inhibited, the Ratio% value decreases.

Meanwhile, the Ratio% valuse were compared, and in the case of compounds of Examples 1, 2, 10, 12, 13, 14, 15, 16, 17, 18, 19, 20, 21, 22, and 31 with excellent reuptake inhibitory effects on dopamine, norepinephrine and serotonin, the concentration at which the Ratio% is 50% was calculated using a total of 6 concentrations (1 nM, 5 nM, 10 nM, 50 nM, 100 nM, and 1 µM) and expressed as IC₅₀.

**[Table 13]**

| Example | Triple reuptake inhibition (Ratio%) | | | Triple reuptake inhibition (IC₅₀, µM) | | |
|---|---|---|---|---|---|---|
| | SERT | NET | DAT | SERT | NET | DAT |
| 1 | 91.56 | 26.06 | 14.90 | 3.73 | 0.343 | 0.170 |
| 2 | 67.47 | 2.58 | 3.94 | 5.20 | 0.014 | 0.083 |
| 3 | 95.94 | 124.39 | 81.14 | | | |
| 4 | 90.26 | 64.76 | 22.39 | | | |
| 5 | 91.11 | 78.01 | 88.53 | | | |
| 6 | 70.39 | 88.83 | 88.63 | | | |
| 7 | 79.48 | 103.43 | 73.88 | | | |
| 8 | 88.65 | 66.48 | 32.29 | | | |
| 9 | 95.15 | 73.45 | 32.29 | | | |
| 10 | 64.75 | 37.16 | 14.88 | 3.458 | 1.336 | 0.063 |
| 11 | 98.07 | 19.08 | 94.91 | | | |
| 12 | 63.05 | 3.38 | 5.96 | 1.147 | 0.036 | 0.174 |
| 13 | 60.60 | 4.78 | 10.00 | 1.21 | 0.070 | 50.28 |
| 14 | 74.39 | 5.84 | 7.44 | 1.66 | 0.173 | 0.016 |
| 15 | 83.60 | 49.82 | 21.04 | 25.02 | 1.11 | 0.173 |
| 16 | 80.93 | 36.38 | 12.06 | 3.648 | 0.501 | 0.048 |
| 17 | 50.31 | 11.66 | 9.25 | 0.945 | 0.226 | 0.083 |
| 18 | 66.40 | 22.07 | 37.07 | 1.58 | 0.611 | 0.076 |
| 19 | 81.23 | 15.80 | 21.23 | 1.17 | 0.188 | 0.247 |
| 20 | 50.14 | 38.22 | 5.14 | 0.643 | 0.748 | 0.080 |
| 21 | 50.05 | 27.49 | 20.41 | 2.088 | 0.619 | 0.259 |
| 22 | 47.99 | 32.07 | 19.30 | 0.945 | 0.644 | 0.187 |
| 23 | 125.38 | 90.00 | 46.60 | | | |
| 24 | 109.21 | 26.69 | 6.56 | | | |
| 25 | 73.17 | 76.00 | 26.92 | | | |
| 26 | 88.78 | 53.01 | 26.92 | | | |
| 27 | 59.52 | 53.55 | 9.71 | 0.968 | 1.986 | 0.062 |
| 28 | 93.63 | 85.85 | 65.47 | | | |
| 29 | 72.63 | 45.28 | 36.13 | | | |
| 30 | 88.71 | 96.77 | 72.11 | | | |
| 31 | 32.68 | 32.54 | 19.20 | 0.276 | 0.459 | 0.204 |
| 32 | 51.31 | 93.09 | 44.81 | | | |
| 33 | 103.08 | 145.38 | 97.72 | | | |
| 34 | 94.18 | 124.25 | 90.85 | | | |
| 35 | 27.99 | 56.53 | 35.65 | 0.3 | 0.427 | 0.629 |
| 36 | 41.52 | 85.40 | 69.78 | | | |
| 37 | 27.71 | 79.91 | 40.29 | 0.322 | 1.57 | 0.349 |
| 38 | 51.55 | 64.82 | 31.70 | 0.518 | 0.361 | 0.670 |
| 39 | 58.07 | 99.14 | 79.20 | | | |
| 40 | 53.66 | 92.42 | 50.15 | 0.648 | 0.924 | 0.670 |
| 41 | 57.01 | 86.61 | 62.96 | | | |
| 42 | 42.17 | 96.81 | 42.13 | 0.417 | 0.969 | 0.453 |
| 43 | 35.57 | 88.30 | 54.93 | 0.506 | 4.04 | 0.435 |
| 44 | 28.87 | 92.59 | 80.58 | | | |
| 45 | 21.98 | 112.38 | 72.49 | | | |
| 46 | 60.13 | 53.00 | 13.85 | 2.98 | 0.816 | 0.203 |
| 47 | 51.93 | 68.26 | 20.47 | 10.67 | 1.168 | 0.372 |
| 48 | 53.41 | 23.52 | 20.20 | 2.559 | 0.348 | 0.134 |
| 49 | 63.99 | 44.81 | 11.41 | 1.77 | 0.475 | 0.223 |
| 50 | 66.02 | 27.46 | 20.84 | 5.7 | 0.176 | 0.236 |
| 51 | 38.36 | 56.36 | 75.88 | | | |
| 52 | 52.31 | 50.92 | 48.72 | | | |
| 53 | 77.61 | 28.99 | 52.90 | | | |
| 54 | 88.15 | 52.40 | 38.97 | | | |
| 55 | 86.78 | 66.71 | 40.14 | | | |
| 56 | 15.26 | 39.66 | 45.52 | 0.3 | 0.640 | 1.2 |
| 57 | 56.55 | 37.21 | 20.95 | 0.58 | 0.463 | 0.208 |
| 58 | 50.24 | 76.51 | 77.67 | | | |
| 59 | 31.88 | 77.82 | 75.06 | 0.226 | | |
| 60 | 61.70 | 95.63 | 94.44 | 1.87 | | |
| 61 | 82.77 | 48.25 | 59.39 | | | |
| 62 | 79.73 | 36.41 | 52.96 | | | |
| 63 | 61.10 | 27.25 | 26.54 | 1.837 | 0.327 | 0.221 |
| 64 | 91.98 | 64.96 | 32.65 | | | |
| 65 | 92.53 | 67.11 | 28.61 | | | |
| 66 | | 58.77 | 51.59 | | | |
| 69 | | 90.43 | 50.33 | | | |

In Table 13 above, SERT means a serotonin transporter, NET means a norepinephrine transporter, and DAT means a dopamine transporter.

As shown in Table 13 above,
it can be seen that the compounds of the Examples of the present invention can inhibit reuptake of serotonin, norepinephrine, and dopamine.

Therefore, the compound represented by Formula 1 according to the present invention is excellent in the triple reuptake inhibitory effect of serotonin, norepinephrine, and dopamine, and thus can be useful in the treatment of mental disorders.

### INDUSTRIAL APPLICABILITY

The compound represented by Formula 1 according to the present invention is excellent in the triple reuptake inhibitory effect of serotonin, norepinephrine, and dopamine, and thus can be useful in the treatment of mental disorders.

## Claims

1. A compound represented by Formula 1 below, a stereoisomer thereof, or a pharmaceutically acceptable salt thereof: 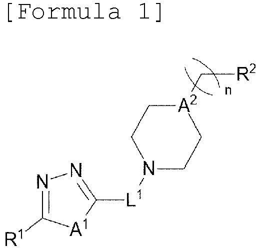
in Formula 1 above,
n is an integer from 0 to 5;
A¹ is O, NH, or S;
A² is N or CH;
L¹ is linear or branched C₁₋₅ alkylene or C(=O);
R¹ is 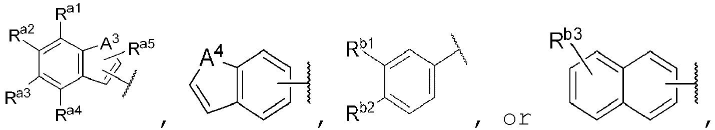
wherein A³ is NH, O, or S, A⁴ is NH, O, or S,
R^{a1}, R^{a2}, R^{a3}, R^{a4}, and R^{a5} are independently hydrogen, halogen, hydroxy, amino, CN, linear or branched C₁₋₁₀ alkyl which is unsubstituted or substituted with one or more halogens, linear or branched C₁₋₁₀ alkoxy which is unsubstituted or substituted with one or more halogens, - C(=O)OR^{c}. -C(=O)N(R^{c})₂, or -NR^{c}C(=O)R^{c}, or R^{a2} and R^{a3} together with the carbon to which they are attached form a 5- or 6-membered heterocycloalkenyl comprising one or more O,
R^{b1} and R^{b2} are independently hydrogen, halogen, CN, linear or branched C₁₋₁₀ alkyl which is unsubstituted or substituted with one or more halogens, linear or branched C₁₋₁₀ alkoxy which is unsubstituted or substituted with one or more halogens, -C(=O)OR^{c}, -C(=O)N(R^{c})₂, -NR^{c}C(=O)R^{c} or phenoxy, or R^{b1} and R^{b2} together with the carbon to which they are attached form a 5- or 6-membered heterocycloalkenyl comprising one or more O, and R^{b3} is hydrogen, hydroxy, C₁₋₃ alkyl, or C₁₋₃ alkoxy,
R^{c} is independently hydrogen, substituted or unsubstituted linear or branched C₁₋₁₀ alkyl, in which the substituted alkyl is substituted with one or more substituents selected from the group consisting of halogen, amino, methylamino, and dimethylamino; and
R² is 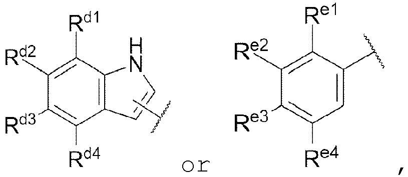 wherein R^{d1}, R^{d2}, R^{d3}, R^{d4}, R^{e1}, R^{e2}, R^{e3}, and R^{e4} are independently hydrogen, halogen, linear or branched C₁₋₁₀ alkyl which is unsubstituted or substituted with one or more halogens, or linear or branched C₁₋₁₀ alkoxy which is unsubstituted or substituted with one or more halogens.

2. The compound, a stereoisomer thereof, or a pharmaceutically acceptable salt thereof according to claim 1, wherein
n is an integer from 0 to 4;
A¹ is O, NH, or S;
A² is N or CH;
L¹ is linear or branched C₁₋₃ alkylene or C(=O);
R¹ is 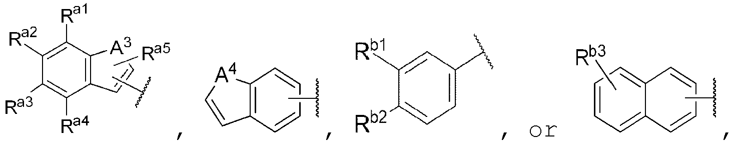
wherein A³ is NH, O, or S, A⁴ is NH, O, or S,
R^{a1}, R^{a2}, R^{a3}, R^{a4}, and R^{a5} are independently hydrogen, halogen, hydroxy, amino, CN, linear or branched C₁₋₅ alkyl which is unsubstituted or substituted with one or more halogens, linear or branched C₁₋₅ alkoxy which is unsubstituted or substituted with one or more halogens, - C(=O)OR^{c}, -C(=O)N(R^{c})₂, or -NR^{c}C(=O)R^{c}, or R^{a2} and R^{a3} together with the carbon to which they are attached form a 5- or 6-membered heterocycloalkenyl comprising one or two O;
R^{b1} and R^{b2} are independently hydrogen, halogen, CN, linear or branched C₁₋₅ alkyl which is unsubstituted or substituted with one or more halogens, linear or branched C₁₋₅ alkoxy which is unsubstituted or substituted with one or more halogens, -C(=O)OR^{c}, -C(=O)N(R^{c})₂, -NR^{c}C(=O)R^{c}, or phenoxy, or R^{b1} and R^{b2} together with the carbon to which they are attached form a 5- or 6-membered heterocycloalkenyl comprising one or two O;
R^{c} is independently hydrogen, substituted or unsubstituted linear or branched C₁₋₅ alkyl, in which the substituted alkyl is substituted with amino, methylamino, or dimethylamino; and
R² is 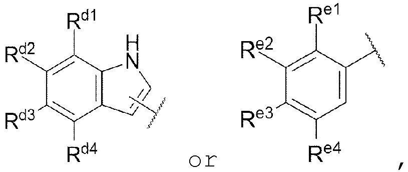 wherein R^{d1}, R^{d2}, R^{d3}, R^{d4}, R^{e1}, R^{e2}, R^{e3}, and R^{e4} are independently hydrogen, halogen, linear or branched C₁₋₅ alkyl which is unsubstituted or substituted with one or more halogens, or linear or branched C₁₋₅ alkoxy which is unsubstituted or substituted with one or more halogens.

3. The compound, a stereoisomer thereof, or a pharmaceutically acceptable salt thereof according to claim 1, wherein
n is an integer from 0 to 2;
A¹ is O, NH, or S;
A² is N or CH;
L¹ is CH₂ or C(=O);
R¹ is 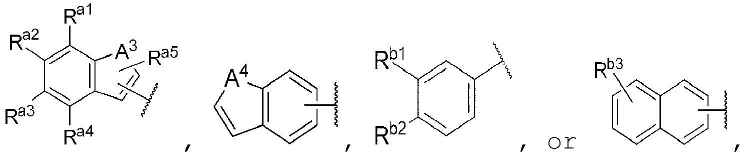
wherein A³ is NH, O, or S, A⁴ is NH, O, or S, and R^{a1}, R^{a2}, R^{a3}, R^{a4}, and R^{a5} are independently hydrogen, halogen, OH, -NH₂, CN, methyl, -CF₃, methoxy, butoxy, -OCF₃, -C(=O)OCH₃, - C(=O)OH, -C(=O)NH₂, or -NHC (=O) CH₃, or R^{a2} and R^{a3} together with the carbon to which they are attached form 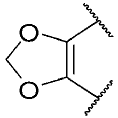 or 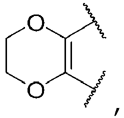 and
R^{b1} and R^{b2} are independently hydrogen, halogen, OH, -NH₂, CN, methyl, -CF₃, methoxy, butoxy, -OCF₃, -C(=O)OCH₃, -C(=O)OH, -C(=O)NH₂, -C(=O)NH(CH₂)₂N(CH₃)₂, -NHC(=O)CH₃, or phenoxy, or R^{b1} and R^{b2} together with the carbon to which they are attached form 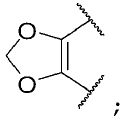 and
R² is 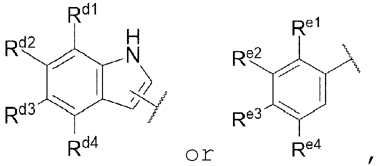
wherein R^{d1}, R^{d2}, R^{d3}, and R^{d4} are hydrogen, and R^{e1}, R^{e2}, R^{e3}, and R^{e4} are independently hydrogen, Cl, or methoxy.

4. The compound, a stereoisomer thereof, or a pharmaceutically acceptable salt thereof according to claim 1, wherein R¹ is:

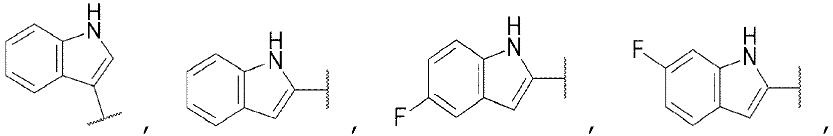

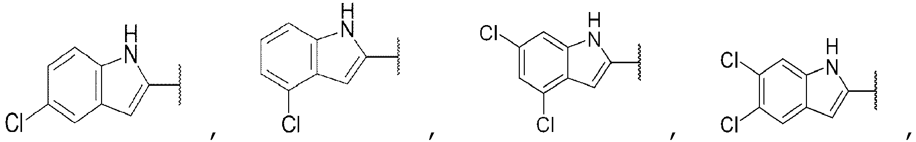

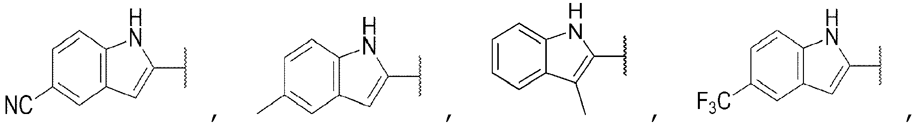

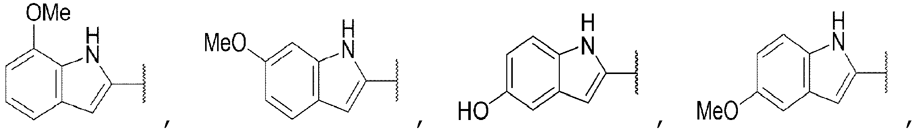

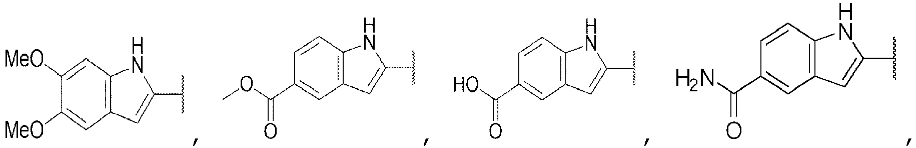

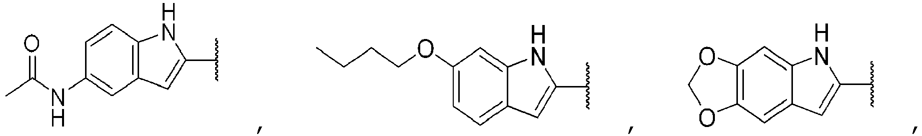

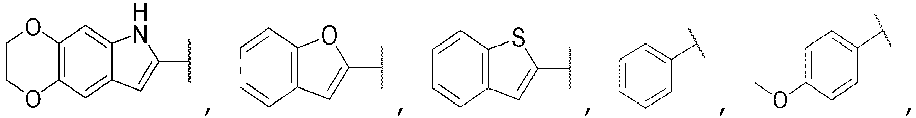

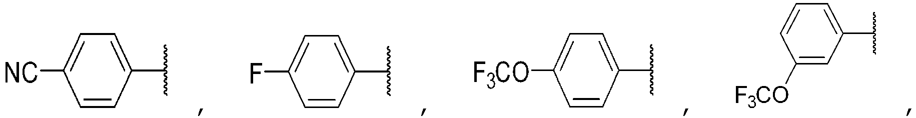

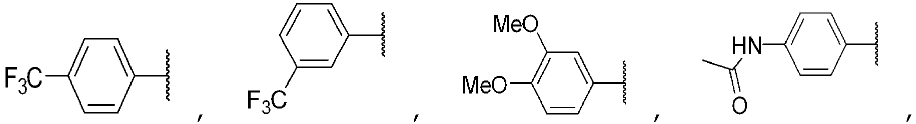

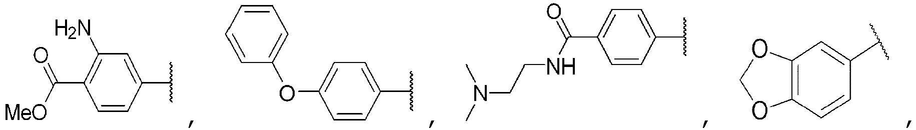

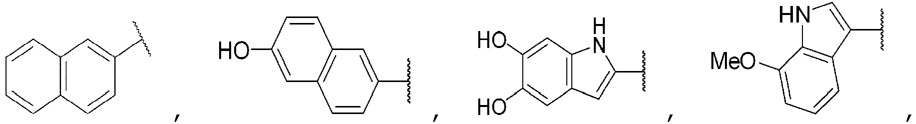

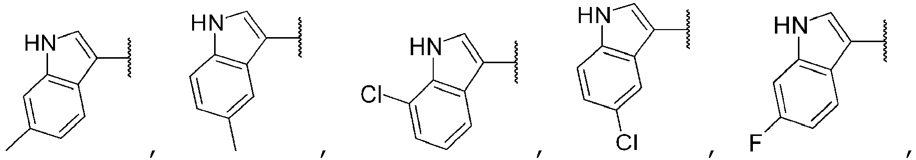

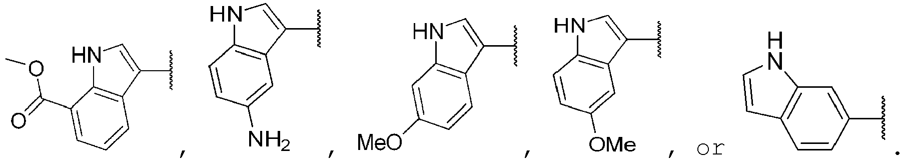

5. The compound, a stereoisomer thereof, or a pharmaceutically acceptable salt thereof according to claim 1, wherein:
R² is 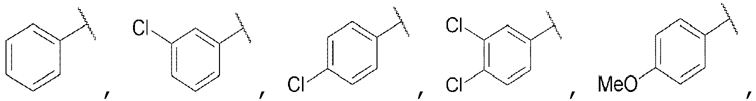 or

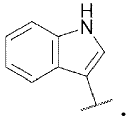

6. The compound, a stereoisomer thereof, or a pharmaceutically acceptable salt thereof according to claim 1, wherein the compound represented by Formula 1 is any one selected from the group of compounds below:
<1> 2-(5-((4-benzylpiperidin-1-yl)methyl)-4H-1,2,4-triazol-3-yl)-1H-indole;
<2> 2-(5-((4-benzylpiperidin-1-yl)methyl)-4H-1,2,4-triazol-3-yl)-5-fluoro-1H-indole;
<3> 2-(5-((4-phenylpiperidin-1-yl)methyl)-4H-1,2,4-triazol-3-yl)-1H-indole;
<4> 2-(5-((4-benzylpiperazin-1-yl)methyl)-4H-1,2,4-triazol-3-yl)-1H-indole;
<5> 2-(5-((4-(3,4-dichlorobenzyl)piperidin-1-yl)methyl)-4H-1,2,4-triazol-3-yl)-1H-indole;
<6> 2-(5-((4-(4-methoxybenzyl)piperidin-1-yl)methyl)-4H-1,2,4-triazol-3-yl)-1H-indole;
<7> 2-(5-((4-(4-chlorobenzyl)piperidin-1-yl)methyl)-4H-1,2,4-triazol-3-yl)-1H-indole;
<8> 2-(5-((4-(3-chlorobenzyl)piperidin-1-yl)methyl)-4H-1,2,4-triazol-3-yl)-1H-indole;
<9> 4-benzyl-1-((5-phenyl-4H-1,2,4-triazol-3-yl)methyl)piperidine;
<10> 4-benzyl-1-((5-(4-methoxyphenyl)-4H-1,2,4-triazol-3-yl)methyl)piperidine;
<11> (4-benzylpiperidin-1-yl)(5-(4-methoxyphenyl)-4H-1,2,4-triazol-3-yl)methanone;
<12> 2-(5-((4-benzylpiperidin-1-yl)methyl)-4H-1,2,4-triazol-3-yl)-5,6-dimethoxy-1H-indole;
<13> 2-(5-((4-benzylpiperidin-1-yl)methyl)-4H-1,2,4-triazol-3-yl)-5-methoxy-1H-indole;
<14> 2-(5-((4-benzylpiperidin-1-yl)methyl)-4H-1,2,4-triazol-3-yl)-5-chloro-1H-indole;
<15> 2-(5-((4-benzylpiperidin-1-yl)methyl)-4H-1,2,4-triazol-3-yl)-5-methyl-1H-indole;
<16> 2-(5-((4-benzylpiperidin-1-yl)methyl)-4H-1,2,4-triazol-3-yl)-6-fluoro-1H-indole;
<17> 2-(5-((4-benzylpiperidin-1-yl)methyl)-4H-1,2,4-triazol-3-yl)-1H-indol-5-carbonitrile;
<18> 2-(5-((4-benzylpiperidin-1-yl)methyl)-4H-1,2,4-triazol-3-yl)-6-methoxy-1H-indole;
<19> 2-(5-((4-benzylpiperidin-1-yl)methyl)-4H-1,2,4-triazol-3-yl)-7-methoxy-1H-indole;
<20> methyl 2-(5-((4-benzylpiperidin-1-yl)methyl)-4H-1,2,4-triazol-3-yl)-1H-indol-5-carboxylate;
<21> 4-benzyl-1-((5-(naphthalen-2-yl)-4H-1,2,4-triazol-3-yl)methyl)piperidine;
<22> 3-(5-((4-benzylpiperidin-1-yl)methyl)-4H-1,2,4-triazol-3-yl)-1H-indole;
<23> 2-(5-((4-benzylpiperidin-1-yl)methyl)-4H-1,2,4-triazol-3-yl)-5-(trifluoromethyl)-1H-indole;
<24> 2-(5-((4-benzylpiperidin-1-yl)methyl)-4H-1,2,4-triazol-3-yl)-4-chloro-1H-indole;
<25> 1-((5-(benzofuran-2-yl)-4H-1,2,4-triazol-3-yl)methyl)-4-benzylpiperidine;
<26> 1-((5-(benzo[b]thiophen-2-yl)-4H-1,2,4-triazol-3-yl)methyl)-4-benzylpiperidine;
<27> 1-((5-(benzo[d][1,3]dioxol-5-yl)-4H-1,2,4-triazol-3-yl)methyl)-4-benzylpiperidine;
<28> 2-(5-((4-benzylpiperidin-1-yl)methyl)-4H-1,2,4-triazol-3-yl)-1H-indol-5-carboxylic acid;
<29> 2-(5-((4-(3,4-dichlorobenzyl)piperidin-1-yl)methyl)-4H-1,2,4-triazol-3-yl)-5-fluoro-1H-indole;
<30> 2-(5-((4-(3,4-dichlorobenzyl)piperidin-1-yl)methyl)-4H-1,2,4-triazol-3-yl)-5-methyl-1H-indole;
<31> 2-(5-((4-(3,4-dichlorobenzyl)piperidin-1-yl)methyl)-4H-1,2,4-triazol-3-yl)-5,6-dimethoxy-1H-indole;
<32> 2-(5-((4-(1H-indol-3-yl)piperidin-1-yl)methyl)-4H-1,2,4-triazol-3-yl)-5-fluoro-1H-indole;
<33> 2-((4-benzylpiperidin-1-yl)methyl)-5-(1H-indol-2-yl)-1,3,4-oxadiazole;
<34> 2-((4-(3,4-dichlorobenzyl)piperidin-1-yl)methyl)-5-(1H-indol-2-yl)-1,3,4-oxadiazole;
<35> 2-(5-((4-benzylpiperidin-1-yl)methyl)-4H-1,2,4-triazol-3-yl)-1H-indol-5,6-diol;
<36> 3-(5-((4-benzylpiperidin-1-yl)methyl)-4H-1,2,4-triazol-3-yl)-5-methoxy-1H-indole;
<37> 3-(5-((4-benzylpiperidin-1-yl)methyl)-4H-1,2,4-triazol-3-yl)-7-methoxy-1H-indole;
<38> 3-(5-((4-benzylpiperidin-1-yl)methyl)-4H-1,2,4-triazol-3-yl)-6-methyl-1H-indole;
<39> 3-(5-((4-benzylpiperidin-1-yl)methyl)-4H-1,2,4-triazol-3-yl)-5-methyl-1H-indole;
<40> 3-(5-((4-benzylpiperidin-1-yl)methyl)-4H-1,2,4-triazol-3-yl)-7-chloro-1H-indole;
<41> 3-(5-((4-benzylpiperidin-1-yl)methyl)-4H-1,2,4-triazol-3-yl)-5-chloro-1H-indole;
<42> methyl 3-(5-((4-benzylpiperidin-1-yl)methyl)-4H-1,2,4-triazol-3-yl)-1H-indol-7-carboxylate;
<43> 3-(5-((4-benzylpiperidin-1-yl)methyl)-4H-1,2,4-triazol-3-yl)-1H-indol-5-amine;
<44> 3-(5-((4-(3,4-dichlorobenzyl)piperidin-1-yl)methyl)-4H-1,2,4-triazol-3-yl)-5-methoxy-1H-indole;
<45> 3-(5-((4-(3,4-dichlorobenzyl)piperidin-1-yl)methyl)-4H-1,2,4-triazol-3-yl)-7-methoxy-1H-indole;
<46> 4-(5-((4-benzylpiperidin-1-yl)methyl)-4H-1,2,4-triazol-3-yl)benzonitrile;
<47> 4-benzyl-1-((5-(4-fluorophenyl)-4H-1,2,4-triazol-3-yl)methyl)piperidine;
<48> 4-benzyl-1-((5-(3,4-dimethoxyphenyl)-4H-1,2,4-triazol-3-yl)methyl)piperidine;
<49> 4-benzyl-1-((5-(4-(trifluoromethoxy)phenyl)-4H-1,2,4-triazol-3-yl)methyl)piperidine;
<50> 4-benzyl-1-((5-(4-(trifluoromethyl)phenyl)-4H-1,2,4-triazol-3-yl)methyl)piperidine;
<51> N-(4-(5-((4-benzylpiperidin-1-yl)methyl)-4H-1,2,4-triazol-3-yl)phenyl) acetamide;
<52> 6-(5-((4-benzylpiperidin-1-yl)methyl)-4H-1,2,4-triazol-3-yl)naphthalen-2-ol;
<53> 4-benzyl-1-((5-(4-phenoxyphenyl)-4H-1,2,4-triazol-3-yl)methyl)piperidine;
<54> 4-benzyl-1-((5-(3-(trifluoromethoxy)phenyl)-4H-1,2,4-triazol-3-yl)methyl)piperidine;
<55> 4-benzyl-1-((5-(3-(trifluoromethyl)phenyl)-4H-1,2,4-triazol-3-yl)methyl)piperidine;
<56> 3-(5-((4-benzylpiperidin-1-yl)methyl)-4H-1,2,4-triazol-3-yl)-6-methoxy-1H-indole;
<57> 2-(5-((4-benzylpiperidin-1-yl)methyl)-4H-1,2,4-triazol-3-yl)-1H-indol-5-carboxamide;
<58> 4-(5-((4-benzylpiperidin-1-yl)methyl)-4H-1,2,4-triazol-3-yl)-N-(2-(dimethylamino)ethyl)benzoamide;
<59> 3-(5-((4-(3,4-dichlorobenzyl)piperidin-1-yl)methyl)-4H-1,2,4-triazol-3-yl)-6-methoxy-1H-indole;
<60> methyl 3-(5-((4-(3,4-dichlorobenzyl)piperidin-1-yl)methyl)-4H-1,2,4-triazol-3-yl)-1H-indol-7-carboxylate;
<61> 4-(3,4-dichlorobenzyl)-1-((5-(4-(trifluoromethoxy)phenyl)-4H-1,2,4-triazol-3-yl)methyl)piperidine;
<62> 4-(3,4-dichlorobenzyl)-1-((5-(4-(trifluoromethyl)phenyl)-4H-1,2,4-triazol-3-yl)methyl)piperidine;
<63> methyl 2-amino-4-(5-((4-benzylpiperidin-1-yl)methyl)-4H-1,2,4-triazol-3-yl)benzoate;
<64> 2-(5-((4-benzylpiperazin-1-yl)methyl)-4H-1,2,4-triazol-3-yl)-5-fluoro-1H-indole;
<65> 2-(5-((4-benzylpiperazin-1-yl)methyl)-4H-1,2,4-triazol-3-yl)-5-chloro-1H-indole;
<66> N-(2-(5-((4-benzylpiperidin-1-yl)methyl)-4H-1,2,4-triazol-3-yl)-1H-indol-5-yl) acetamide;
<67> 2-(5-((4-benzylpiperidin-1-yl)methyl)-4H-1,2,4-triazol-3-yl)-1H-indol-5-ol;
<68> (4-benzylpiperidin-1-yl)(5-(5-fluoro-1H-indol-2-yl)-4H-1,2,4-triazol-3-yl)methanone;
<69> 2-(5-((4-benzylpiperidin-1-yl)methyl)-4H-1,2,4-triazol-3-yl)-6-butoxy-1H-indole;
<70> 2-(5-((4-benzylpiperidin-1-yl)methyl)-4H-1,2,4-triazol-3yl)-3-methyl-1H-indole;
<71> 2-(5-((4-benzylpiperidin-1-yl)methyl)-4H-1,2,4-triazol-3-yl)-4,6-dichloro-1H-indole;
<72> 2-(5-((4-benzylpiperidin-1-yl)methyl)-4H-1,2,4-triazol-3-yl)-5,6-dichloro-1H-indole;
<73> 6-(5-((4-benzylpiperidin-1-yl)methyl)-4H-1,2,4-triazol-3-yl)-5H-[1,3]dioxolo[4,5-f]indole;
<74> 7-(5-((4-benzylpiperidin-1-yl)methyl)-4H-1,2,4-triazol-3-yl)-2,3-dihydro-6H-[1,4]dioxino[2,3-f]indole;
<75> 3-(5-((4-benzylpiperidin-1-yl)methyl)-4H-1,2,4-triazol-3-yl)-6-fluoro-1H-indole; and
<76> 6-(5-((4-benzylpiperidin-1-yl)methyl)-4H-1,2,4-triazol-3-yl)-1H-indole.

7. A pharmaceutical composition for use in preventing or treating mental disorders, comprising the compound represented by Formula 1 of claim 1, a stereoisomer thereof, or a pharmaceutically acceptable salt thereof as an active ingredient.

8. The pharmaceutical composition of claim 7, wherein the compound inhibits the reuptake of serotonin, norepinephrine, and dopamine.

9. The pharmaceutical composition of claim 7, wherein the mental disorder is one or more selected from the group consisting of bulimia nervosa, mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substanceinduced mood disorder, pseudo dementia, Ganger syndrome, obsessive-compulsive disorder, panic disorder, panic disorder without agoraphobia, panic disorder with agoraphobia, agoraphobia without history of panic disorder, panic attacks, memory deficit, memory loss, attention deficit hyperactivity disorder, obesity, anxiety, generalized anxiety disorder, eating disorder, Parkinson's disease, Parkinson's symptoms, dementia, aging dementia, senile dementia, Alzheimer's disease, Down syndrome, acquired immunodeficiency syndrome dementia complex, memory dysfunction in aging, specific phobia, social phobia, social anxiety disorder, posttraumatic stress disorder, acute stress disorder, chronic stress disorder, drug addiction, drug abuse, drug abuse tendency, cocaine abuse, nicotine abuse, tobacco abuse, alcohol addiction, alcoholism, pathological kleptomaniac, withdrawal syndrome due to withdrawal of intoxicating substances, pain, chronic pain, inflammatory pain, neuropathic pain, diabetic neuropathic pain, migraine, tension-type headache, chronic tension-type headache, depression-related pain, back pain, cancer pain, irritable bowel pain, irritable bowel syndrome, postoperative pain, postmastectomy pain syndrome (PMPS), poststroke pain, druginduced neuropathy, diabetic neuropathy, pain sustained by the sympathetic nervous system, trigeminal neuralgia, toothache, facial muscle pain, phantom limb pain, anorexia, premenstrual syndrome, premenstrual dysphoric disorder, late luteal phase syndrome, posttraumatic syndrome, chronic fatigue syndrome, persistent vegetative state, urinary incontinence, stress incontinence, urge incontinence, nocturnal incontinence, sexual dysfunction, premature ejaculation, erectile difficulty, erectile dysfunction, restless legs syndrome, periodic limb movement disorder, eating disorder, anorexia nervosa, sleep disorder, pervasive developmental disorder, autism, Asperger's disorder, Rett disorder, childhood disintegrative disorder, learning disorder, motor ability disorder, mutism, trichotillomania, narcolepsy, post-stroke depression, stroke-induced brain injury, stroke-induced nerve injury, Tourette syndrome, tinnitus, tic disorder, body dysmorphic disorder, oppositional defiant disorder, and post-stroke disorder.

10. A triple reuptake inhibitor of serotonin, norepinephrine, and dopamine for use in preventing or treating mental disorders, comprising the compound represented by Formula 1 of claim 1, a stereoisomer thereof, or a pharmaceutically acceptable salt thereof as an active ingredient.

11. A health functional food composition for use in preventing or improving mental disorders, comprising the compound represented by Formula 1 of claim 1, a stereoisomer thereof, or a pharmaceutically acceptable salt thereof as an active ingredient.
